# EUROPEAN PATENT SPECIFICATION

(11) **EP 3 615 530 B1**
(45) Date of publication and mention of the grant of the patent: **14.04.2021**
(21) Application number: 18718837.0
(22) Date of filing: 24.04.2018
(51) Int. Cl.: C07D 413/12, A01N 43/80

(54) **SUBSTITUTED SUCCINIMIDE DERIVATIVES AS PESTICIDES**
SUBSTITUIERTEN SUCCINIMID-VERBINDUNGEN ALS PESTIZID
COMPOSÉS DÉRIVÉS DE SUCCINIMIDE SUBSTITUÉ COMME PESTICIDES

(30) Priority: 26.04.2017 EP 17168101
(43) Date of publication of application: 04.03.2020
(73) Proprietor: BASF SE, 67056 Ludwigshafen am Rhein (DE)
(72) Inventor: KOERBER, Karsten, 67056 Ludwigshafen (DE); HUWYLER, Nikolas, 67056 Ludwigshafen (DE); NARINE, Arun, 67056 Ludwigshafen (DE); GOCKEL, Birgit, 67056 Ludwigshafen (DE); MCLAUGHLIN, Martin John, 67056 Ludwigshafen (DE); BRAUN, Franz-Josef, Research Triangle Park, North Carolina 27709 (US)
(74) Representative: BASF IP Association
(86) International application number: PCT/EP2018/060439
(87) International publication number: WO 2018/197466

(56) References cited:
- EP-A1- 1 731 512
- WO-A1-2013/037626
- WO-A1-2013/092943
- WO-A1-2015/128358
- WO-A1-2017/016883
- WO-A1-2017/050921
- WO-A1-2017/050922

## Description

The invention relates to succinimide compounds of formula I 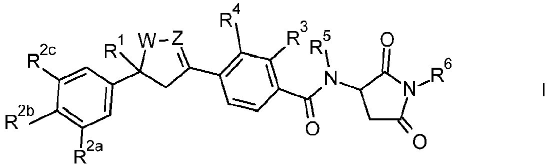 wherein
- W-Z: is -O-N=, -CH₂-N=, or -CH₂-CH=;
- R¹: halomethyl;
- R^{2a}: halogen, halomethyl, or halomethoxy;
- R^{2b},: R^{2c} are independently H, or as defined for R^{2a};
- R³: is halogen, CN, NO₂, C₁-C₂-alkyl, halomethyl, C₁-C₂-alkoxy, S(O)ₘ-C₁-C₂-alkyl, C₁-C₂-haloalkoxy, or S(O)ₘ-C₁-C₂-haloalkyl;
- R⁴: is H, or as defined for R³; or
- R³ and R⁴: form together with the C-atoms they are bound to a 5-, or 6-membered saturated, partially, or fully unsaturated carbocyclic ring;
- R⁵, R⁶: are independently H, CN, C₁-C₁₀-alkyl, C₃-C₈-cycloalkyl, C₂-C₁₀-alkenyl, C₃-C₈-cycloalkenyl, C₂-C₁₀-alkynyl, OR¹⁰, S(O)ₘR¹⁰, S(O)ₘN(R¹⁰)₂, N(R¹⁰)₂, which aliphatic groups are unsubstituted, partially or fully halogenated and/or substituted with one or more R^{a}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
R¹⁰ is independently H, C₁-C₆-alkyl, C₁-C₆-haloalkyl, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆-haloalkynyl, which groups are unsubstituted or substituted with one or more R^{a},
R^{a} is CN, N3, NO₂, SCN, SF₅, Si(C₁-C₄-alkyl)₃, OR^{a1}, OSO₂R^{a1}, S(O)ₙR^{a1}, N(R^{a2})R^{a3}, C(=O)N(R^{a2})R^{a3}, C(=S)N(R^{a2})R^{a3}, C(=O)R^{a1}, C(=O)OR^{a1}, CH=NOR^{a1}, C₃-C₈-cycloalkyl, C₃-C₈-halocycloalkyl, which cyclic moieties may be substituted with R^{a4}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
m is 0, 1, or 2;
n is 0, or 1;
R^{a1} H, C₁-C₆-alkyl, C₁-C₆-haloalkyl, C₂-C₄-alkenyl, C₂-C₄-alkynyl, CH₂-CN, C₃-C₆-cycloalkyl, C₃-C₆-halocycloalkyl, C₃-C₆-cycloalkylmethyl, C₃-C₆-halocycloalkylmethyl, phenyl and hetaryl which aromatic rings are unsubstituted or partially or fully substituted with R^{A};
R^{a2} is H, or C₁-C₆-alkyl,
R^{a3} is H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, or C₃-C₆-cycloalkyl, C₃-C₆-halocycloalkyl, C₃-C₅-cycloalkylmethyl, or C₃-C₆-halocycloalkylmethyl which rings are unsubstituted or substituted with a cyano;
R^{a4} is independently OH, CN, C₁-C₆-alkoxy, C₁-C₆-haloalkoxy, S(O)ₘ-C₁-C₆-alkyl, S(O)ₘ-C₁-C₆-haloalkyl, C(=O)N(R^{a2})R^{a3}, C₃-C₆-cycloalkyl, or C₃-C₆-halocycloalkyl which cycles are unsubstituted or substituted with one or more R^{a11}; or phenyl, partially or fully unsaturated heterocycle which rings are unsubstituted or substituted with one or more R^{A};
R^{a11} is independently OH, cyano, C₁-C₂-alkyl, or C₁-C₂-haloalkyl;
R^{A} is independently selected from halogen, CN, NO₂, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, C₁-C₄-alkoxy, C₁-C₄-haloalkoxy, S(O)ₘ-C₁-C₄-alkyl, S(O)ₘ-C₁-C₄-haloalkyl, C₁-C₄-alkylcarbonyl, C₁-C₄-haloalkylcarbonyl, C(=O)N(R^{a2})R^{a3}; or two R^{A} present on the same carbon atom of a saturated or partially saturated ring may form together =O or =S; or
two R^{A} present on the same S or SO ring member of a heterocyclic ring may together form a group =N(C₁-C₆-alkyl), =NO(C₁-C₆-alkyl), =NN(H)(C₁-C₆-alkyl) or =NN(C₁-C₆-alkyl)₂; and the N-oxides, stereoisomers and agriculturally or veterinarily acceptable salts thereof.

The invention also provides an agricultural composition comprising at least one compound of formula I, a stereoisomer thereof and/or an agriculturally acceptable salt thereof and at least one liquid and/or solid carrier, especially at least one inert liquid and/or solid agriculturally acceptable carrier.

The invention also provides a veterinary composition comprising at least one compound of formula I, a stereoisomer thereof and/or a veterinarily acceptable salt thereof and at least one liquid and/or solid carrier, especially at least one inert veterinarily liquid and/or solid acceptable carrier.

The invention also provides a method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of formula I or a salt thereof as defined herein. The invention also relates to plant propagation material, in particular seed, comprising at least one compound of formula I and/or an agriculturally acceptable salt thereof.

The invention further relates to a method for treating or protecting an animal from infestation or infection by parasites which comprises bringing the animal in contact with a parasiticidally effective amount of a compound of formula I or a veterinarily acceptable salt thereof. Bringing the animal in contact with the compound I, its salt or the veterinary composition of the invention means applying or administering it to the animal.

EP1731512, JP 2007/091708, WO 2007/123853, WO 2007/123855, WO 2008/128711, WO 2010/020522, WO 2013/037626, WO 2017/050921, and WO 2017/050922 describe structurally closely related active compounds. These compounds are mentioned to be useful for combating invertebrate pests.

Nevertheless, there remains a need for highly effective and versatile agents for combating invertebrate pests. It is therefore an object of the invention to provide compounds having a good pesticidal activity and showing a broad activity spectrum against a large number of different invertebrate pests, especially against difficult to control pests, such as insects.

It has been found that these objects can be achieved by compounds of formula I as depicted and defined below, and by their stereoisomers, salts, tautomers and N-oxides, in particular their agriculturally acceptable salts.

Compounds of formula I can be prepared by reacting an activated carboxylic acid derivative of formula II or the corresponding carboxylic acid IIa with a compound of formula III in an amidation reaction. X in formula II denotes a leaving group, preferably halogen such as e.g. Cl or Br, or C₁-C₆-alkoxy such as OCH₃ or OC₂H₅. Aminosuccinimide III is preferably used as its ammonium salt, wherein Y is an anion, preferably a halogenide such as Cl or Br.

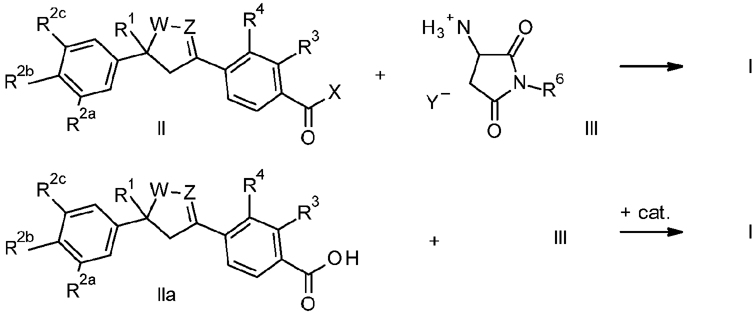

The amidation reaction is usually carried out with the acid chlorides or by prior transformation of carboxylic acids of formula IIa with oxalyl chloride [(COCl)₂] or thionylchloride (SOCl₂) to the corresponding acid chlorides, followed by reaction with the amine of formula III. Suitable reaction conditions are described in literature, e.g. in WO 2004/22536. The reaction is generally carried out in the presence of an organic base such as triethylamine (Et₃N), N,N-diisopropylethylamine (iPr₂NEt), pyridine, substituted pyridines such as collidine or lutidine, or the like. Optionally a nucleophilic catalyst such as 4-(N,N-dimethylamino)pyridine ("DMAP") can be employed in the reaction. Suitable solvents are halogenated hydrocarbons such as, dichloromethane, chloroform, and chlorobenzene, or polar aprotic solvents such as THF, and N,N-dimethylformamide (DMF), or aromatic hydrocarbons such as benzene, toluene, o-, m-, and p-xylene, or mixtures thereof. The transformation is usually carried out at temperatures from -40 °C to 100 °C, preferably from 0 °C to 30 °C. The starting materials are generally reacted with one another in equimolar amounts. In terms of yield, it may be advantageous to employ an excess of III, based on II.

Alternatively, amidation of the carboxylic acid IIa is carried out in the presence of a coupling reagent. Suitable coupling reagents (activators) are known and are, e.g. selected from carbodiimides, such as N,N-dicyclohexylcarbodiimide ("DCC") and N,N-diisopropylcarbodiimide

("DCI"), benzotriazole derivatives such as 1-[bis(dimethylamino)methylene]-1H-1,2,3-triazolo[4,5-b]pyridinium 3-oxid hexafluorophosphate ("HATU"), O-(benzotriazol-1-yl)-N,N,N',N'-tetramethyluronium hexafluorophosphate ("HBTU"), and 1-[bis(dimethylamino)methylen]-5-chlorobenzotriazolium 3-oxide hexafluorophosphate ("HCTU"), or phosphonium-derived activators, such as (Benzotriazol-1-yloxy)tris(dimethylamino)phosphonium hexafluorophosphate ("BOP"), (benzotriazol-1-yl-oxytripyrrolidinophosphonium hexafluorophosphate) ("Py-BOP"), bromotripyrrolidinophosphonium hexafluorophosphate ("Py-BrOP"). Generally, the activator is used in excess. The benzotriazole and phosphonium coupling reagents are generally used in a basic medium. Preferably, bromotripyrrolidinophosphonium hexafluorophosphate ("Py-BrOP") is used as the coupling reagent (activator). Suitable reaction conditions are described in the literature, e.g. in WO2015/128358. The reaction is generally carried out in the presence of a base such as a tertiary amine base like iPr₂NEt, Et₃N. Suitable solvents are, e.g., halogenated hydrocarbons such as dichloromethane, chloroform, and chlorobenzene. The transformation is usually carried out at temperatures from 0 °C to 100 °C, preferably from 10 °C to 40 °C. The starting materials are generally reacted with one another in equimolar amounts. In terms of yield, it may be advantageous to employ an excess of III, based on II.

Introduction of group R⁵ being different from hydrogen is preferably made to formula III compounds. Alternatively, such R⁵ can also be introduced to formula VI, or to formula I compounds wherein R⁵ is H.

Compounds of formula III can be obtained by a reaction sequence starting with cyclization of a compound of formula IV, wherein PG is a protective group, such as benzyloxycarbonyl ("Cbz"), benzyl ("Bn"), *tert*-butylcarbonyl ("Boc"), or acetyl ("Ac"). Compounds of formula IV are commercially available, or can be made by standard methods of organic chemistry known to a person skilled in the art.

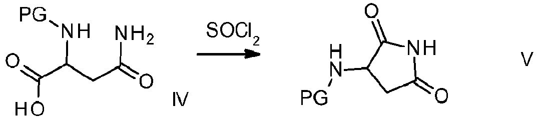

Suitable reaction conditions for the preparation of a compound of formula V by cyclization of a compound of formula IV can de found in the literature, for example in T. Polonski, J. Chem. Soc. Perkin Trans. I 1988, 629-637. The reaction is generally carried out in the presence of a carboxylic acid activator such as, e.g., thionyl chloride (SOCl₂), and performed at temperatures from -100°C to 25 °C, preferably from -78 °C to 0°C, in an inert aprotic dipolar solvent such as DMF, or the like.

Alkylation of formula V compounds can be effected by reaction of V with a compound R⁶-Z wherein Z is a nuceophilic leaving group such as halogen like Cl, Br and I, or a sulfonate such as trifluoromethanesulfoate ("OTf"), or methanesulfonate ("OMs"). Suitable reaction conditions are described in literature, e.g. in J. Maddaluno et al. Tetrahedron: Asymmetry 1992, 3, 1239-1242, in EP1553088, or in K. Kitaori et al. Tetrahedron, 1999, 55, 14381-14390

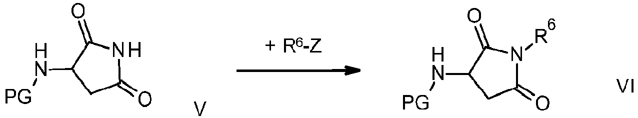

The reaction is generally carried out in the presence of a base such as, for example, inorganic bases, such as alkali metal and alkaline earth metal hydroxides, such as LiOH, NaOH, KOH, CsOH, and Ca(OH)₂, or alkali metal and alkaline earth metal carbonates, such as Li₂CO₃, K₂CO₃, and Cs₂CO₃, or alkali metal fluorides such as CsF. Suitably, the alkali metal and alkaline earth metal hydroxides and carbonates can be employed as an aqueous solution in a biphasic reaction mixture together with an organic solvent such as halogenated hydrocarbons such as dichloromethane, chloroform, and chlorobenzene. Optionally, if biphasic conditions are used, a phase transfer catalyst can be employed in the reaction such as, e.g., tetra(n-butyl)ammonium iodide (Bu₄NI). Otherwise, if the reaction is performed in the absence of water, suitable organic solvents are, e.g., halogenated hydrocarbons such as dichloromethane, chloroform, and chlorobenzene, or polar aprotic solvents such as THF, acetonitrile, or DMF. The starting materials are generally reacted with one another in equimolar amounts. In terms of yield, it may be advantageous to employ an excess of R⁶-Z, based on V.

Elimination of the protecting group can be effected under conditions generally know in the art, e.g. benzyloxycarbonyl ("Cbz") is cleaved under acidic conditions, using a mineral acid such as, for example, HBr in acetic acid [cf. T. Polonski, J. Chem. Soc. Perkin Trans. I 1988, 629-637]. Alternatively, the benzyloxycarbonyl ("Cbz") can be cleaved under hydrogenolytic conditions in the presence of a H₂ atmosphere, and a palladium catalyst such as, e.g., palladium on carbon ("Pd/C") [cf. H. Iding et al., Tetrahedron 2004, 647-653], Pd(OH)₂ on carbon ("Pearlman catalyst") [cf. J. Maddaluno et al. Tetrahedron: Asymmetry 1992, 3, 1239-1242].

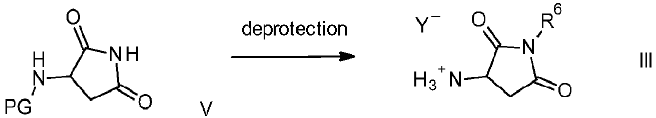

The starting materials required for preparing the compounds I are commercially available, or known from literature [cf. WO2014/029639; WO2010/72781] or can be prepared in accordance with the literature cited. Starting synthesis of compounds IV from commercially availably R-aspartic acid and following the above given synthesis yields in R-configured compounds III, and compounds I with R configuration in the succinimid group, which correspond to formula I.a:

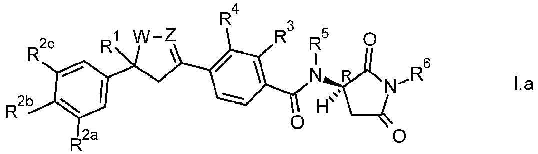

As a rule, the compounds of formula I including their stereoisomers, salts, and N-oxides, and their precursors in the synthesis process, can be prepared by the methods described above. If individual compounds cannot be prepared via the above-described routes, they can be prepared by derivatization of other compounds I or the respective precursor or by customary modifications of the synthesis routes described. For example, in individual cases, certain compounds of formula I can advantageously be prepared from other compounds of formula I by derivatization, e.g. by ester hydrolysis, amidation, esterification, ether cleavage, olefination, reduction, oxidation and the like, or by customary modifications of the synthesis routes described.

The reaction mixtures are worked up in the customary manner, e.g. by mixing with water, separating the phases, and, if appropriate, purifying the crude products by chromatography, e.g. on alumina or on silica gel. Some of the intermediates and end products may be obtained in the form of colorless or pale brown viscous oils which are freed or purified from volatile components under reduced pressure and at moderately elevated temperature. If the intermediates and end products are obtained as solids, they may be purified by recrystallization or trituration. However, if the synthesis yields mixtures of isomers, a separation is generally not necessarily required since in some cases the individual isomers can be interconverted during work-up for use or during application (e.g. under the action of light, acids or bases). Such conversions may also take place after use, e.g. in the treatment of plants in the treated plant.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl, ethyl, n-propyl, iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 10 carbon atoms, frequently from 1 to 6 carbon atoms, preferably 1 to 4 carbon atoms. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to alkyl usually comprising 1 to 10, frequently 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 10 carbon atoms, frequently from 1 to 6 carbon atoms, preferably 1 to 4 carbon atoms, wherein the hydrogen atoms of this group are partially or totally replaced with halogen atoms, in particular fluorine atoms. Preferred haloalkoxy moieties include C₁-C₄haloalkoxy, in particular C₁-C₂-fluoroalkoxy, such as fluoromethoxy, difluoromethoxy, trifluoromethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoro-ethoxy, 2,2dichloro-2-fluorethoxy, 2,2,2-trichloroethoxy, pentafluoroethoxy and the like.

The term "alkylthio "(alkylsulfanyl: S-alkyl)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom. The term "haloalkylthio" as used herein refers to an alkylthio group as mentioned above wherein the hydrogen atoms are partially or fully substituted by fluorine, chlorine, bromine and/or iodine.

The term "alkylsulfinyl" (alkylsulfoxyl: S(=O)-C₁-C₆-alkyl), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "haloalkylsulfinyl" as used herein refers to an alkylsulfinyl group as mentioned above wherein the hydrogen atoms are partially or fully substituted by fluorine, chlorine, bromine and/or iodine.

The term "alkylsulfonyl" (S(=O)₂-alkyl) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "haloalkylsulfonyl" as used herein refers to an alkylsulfonyl group as mentioned above wherein the hydrogen atoms are partially or fully substituted by fluorine, chlorine, bromine and/or iodine.

The term "alkylcarbonyl" refers to an alkyl group as defined above, which is bonded via the carbon atom of a carbonyl group (C=O) to the remainder of the molecule.

The term "haloalkylcarbonyl" refers to an alkylcarbonyl group as mentioned above, wherein the hydrogen atoms are partially or fully substituted by fluorine, chlorine, bromine and/or iodine. The term "alkoxycarbonyl" refers to an alkylcarbonyl group as defined above, which is bonded via an oxygen atom to the remainder of the molecule.

The term "haloalkoxycarbonyl" refers to an alkoxycarbonyl group as mentioned above, wherein the hydrogen atoms are partially or fully substituted by fluorine, chlorine, bromine and/or iodine.

The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2-propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the hydrogen atoms are partially or totally replaced with halogen atoms.

The term "alkynyl" as used herein denotes in each case a singly unsaturated hydrocarbon radical having usually 2 to 10, frequently 2 to 6, preferably 2 to 4 carbon atoms, e.g. ethynyl, propargyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like. The term "haloalkynyl" as used herein refers to an alkynyl group as defined above, wherein the hydrogen atoms are partially or totally replaced with halogen atoms.

The term "cycloalkyl" as used herein and in the cycloalkyl moieties of cycloalkoxy and cycloalkylthio denotes in each case a monocyclic cycloaliphatic radical having usually from 3 to 10 or from 3 to 6 carbon atoms, such as cyclopropyl, cyclobutyl, cyclopentyl, cyclohexyl, cycloheptyl , cyclooctyl, cyclononyl, and cyclodecyl, or cyclopropyl (c-C₃H₅), cyclobutyl (c-C₄H₇), cyclopentyl (c-C₅H₉), and cyclohexyl (c-C₆H₁₁).

The term "halocycloalkyl" as used herein and in the halocycloalkyl moieties of halocycloalkoxy and halocycloalkylthio denotes in each case a monocyclic cycloaliphatic radical having usually from 3 to 10 C atoms or 3 to 6 C atoms, wherein at least one, e.g. 1, 2, 3, 4 or 5 of the hydrogen atoms, are replaced by halogen, in particular by fluorine or chlorine. Examples are 1- and 2-fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl, and the like.

The term "cycloalkenyl" as used herein and in the cycloalkenyl moieties of cycloalkenyloxy and cycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms. Exemplary cycloalkenyl groups include cyclopropenyl, cycloheptenyl or cyclooctenyl.

The term "halocycloalkenyl" as used herein and in the halocycloalkenyl moieties of halocycloalkenyloxy and halocycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms, wherein at least one, e.g. 1, 2, 3, 4 or 5 of the hydrogen atoms, are replaced by halogen, in particular by fluorine or chlorine. Examples are 3,3-difluorocyclopropen-1-yl and 3,3-dichlorocyclopropen-1-yl.

The term "cycloalkenylalkyl" refers to a cycloalkenyl group as defined above which is bonded via an alkylene group, such as a C₁-C₅-alkyl group or a C₁-C₄-alkyl group, in particular a methylene group (= cycloalkenylmethyl), to the remainder of the molecule.

The term "carbocycle" or "carbocyclyl" includes in general a 3- to 12-membered, preferably a 3- to 8-membered or a 5- to 8-membered, more preferably a 5- or 6-membered mono-cyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 5-or 6-membered, in particular 6-membered monocyclic heterocyclic non-aromatic radicals. The heterocyclic non-aromatic radicals usually comprise 1, 2 or 3 heteroatoms selected from N, O and S as ring members, wherein S-atoms as ring members may be present as S, SO or SO₂. Examples of 5- or 6-membered heterocyclic radicals comprise saturated or unsaturated, non-aromatic heterocyclic rings, such as 2- and 3-azetidinyl, 2- and 3-oxetanyl, 2- and 3-thietanyl, 2-and 3-thietanyl-S-oxid (S-oxothietanyl), 2- and 3-thietanyl-S-dioxid (S-dioxothietanyl), 2- and 3-pyrrolidinyl, 2- and 3-tetrahydrofuranyl, 1,3-dioxolan-2-yl, thiolan-2-yl, S-oxothiolan-2-yl, S-dioxothiolan-2-yl, 4- and 5-oxazolidinyl, 1,3-dioxan-2-yl, 1- and 3-thiopyran-2-yl, S-oxothiopyranyl, and S-dioxothiopyranyl.

The term "hetaryl" includes monocyclic 5- or 6-membered heteroaromatic radicals comprising as ring members 1, 2, or 3 heteroatoms selected from N, O and S. Examples of 5- or 6-membered heteroaromatic radicals include pyridyl, i.e. 2-, 3-, and 4-pyridyl, pyrimidinyl, i.e. 2-, 4- and 5-pyrimidinyl, pyrazinyl, pyridazinyl, i.e. 3- and 4-pyridazinyl, thienyl, i.e. 2- and 3-thienyl, furyl, i.e. 2- and 3-furyl, pyrrolyl, i.e. 1-, 2- and 3-pyrrolyl, oxazolyl, i.e. 2-, 4- and 5-oxazolyl, isoxazolyl, i.e. 3-, 4- and 5-isoxazolyl, thiazolyl, i.e. 2-, 3- and 5-thiazolyl, isothiazolyl, i.e. 3-, 4- and 5-isothiazolyl, pyrazolyl, i.e. 1-, 3-, 4- and 5-pyrazolyl, imidazolyl, i.e. 1-, 2-, 4- and 5-imidazolyl, oxadiazolyl, e.g. 2- and 5-[1,3,4]oxadiazolyl, thiadiazolyl, e.g. 1,3,4-thiadiazol-5-yl, 1,2,4-thiadiazol-3-yl, triazolyl, e.g. 1,3,4-triazol-2-yl, and 1,2,4-triazol-3-yl.

The terms "heterocyclyolalkyl" and "hetarylalkyl" refer to heterocyclyl or hetaryl, resp., as defined above which are bound via a C₁-C₄-alkyl group, in particular a methyl group (= heterocyclylmethyl or hetarylmethyl, resp.), to the remainder of the molecule.

With respect to the variables, the particularly preferred embodiments of the intermediates correspond to those of the compounds of the formula I.

In a particular embodiment, the variables of the compounds of the formula I have the following meanings, these meanings, both on their own and in combination with one another, being particular embodiments of the compounds of formula I.

In a preferred embodiment, the compounds I are present in form of a mixture of compounds I.A and I.B, wherein compound I.A with S-configuration in the W-Z-containing ring is present in an amount of more than 50% by weight, in particular of at least 70% by weight, more particularly of at least 85% by weight, specifically of at least 90% by weight, based on the total weight of compounds I.A and I.B.

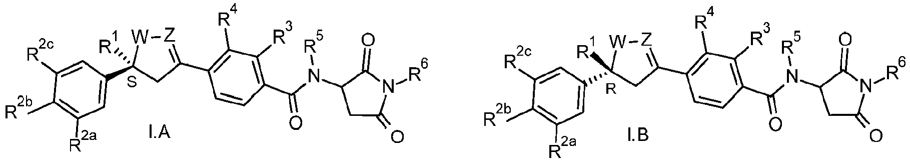

In one particularly preferred embodiment of the invention, the method comprises the step of contacting the plant, parts of it, its propagation material, the pests, their food supply, habitat or breeding grounds a pesticidally effective amount of a compound of formula I.A.

Compounds of formula I.A, and I.B, resp., can be obtained in enantiopure form by known separation methods, preferably by chiral chromatography. This is preferably applied to intermediate compounds of formula IIa.

In another preferred embodiment the succinimide ring is present in form of a mixture of compounds I.a and I.b, wherein compound I.a (R-configuration in succinimide) is present in an amount of more than 50% by weight, in particular of at least 70% by weight, more particularly of at least 85% by weight, specifically of at least 90% by weight, based on the total weight of compounds I.a and I.b.

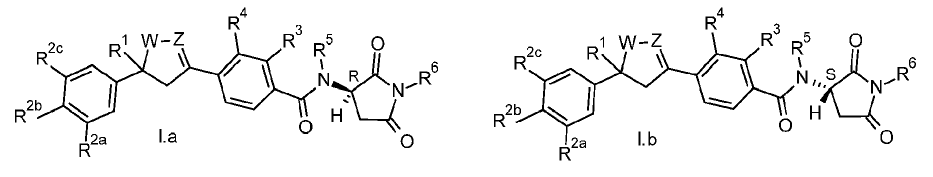

In another preferred embodiment the compounds of formula I are present in form of a mixture of stereoisomers as shown above, wherein compound I.Aa (S-configuration in W-Z-ring and R-configuration in succinimide) is present in an amount of more than 50% by weight, in particular of at least 70% by weight, more particularly of at least 85% by weight, specifically of at least 90% by weight, based on the total weight of stereoisomers of formula I.

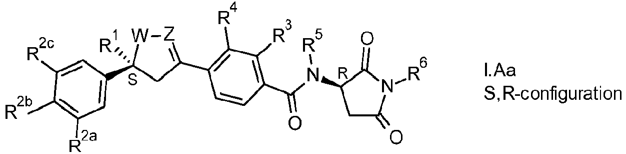

Accordingly, in a particularly preferred embodiment of the invention, the method comprises step of contacting the plant, parts of it, its propagation material, the pests, their food supply, habitat or breeding grounds a pesticidally effective amount of a compound of formula I.Aa.

Racemates of compounds of formula I consist of four stereoisomers I.Aa, I.Ab, I.Ba, and I.Bb. Accordingly isomer I.A consists of more than 50% by weight, in particular of at least 70% by weight, more particularly of at least 85% by weight, specifically of at least 90% by weight of two stereoisomers I.Aa and I.Ab.

Isomer I.a consists of more than 50% by weight, in particular of at least 70% by weight, more particularly of at least 85% by weight, specifically of at least 90% by weight of two stereoisomers I.Aa and I.Ba.

Preferably -W-Z= in formula I is -O-N=; such compounds correspond to formula 1.1.
In another embodiment W-Z in formula I is -CH₂-N=; such compounds correspond to formula 1.2. In another embodiment W-Z in formula I is -CH₂-CH=; such compounds correspond to formula I.3.

R^{2a} is preferably selected from F, Cl, Br, CF₃, and OCF₃.
R^{2b} and R^{2c} are independently preferably selected from H, F, Cl, Br, CF₃, and OCF₃.

Particularly preferred is each one of the following combinations of R^{2a}, R^{2b}, and R^{2c} wherein each line of Table A denotes a substitution pattern of the phenyl ring ("A") bearing the R^{2a}, R^{2b}, and R^{2c} moieties.

**Table A**

| No. | R^{2a} | R^{2b} | R^{2c} |
|---|---|---|---|
| A-1 | F | F | H |
| A-2 | F | H | F |
| A-3 | F | F | F |
| A-4 | F | Cl | F |
| A-5 | F | Br | F |
| A-6 | F | H | Cl |
| A-7 | F | H | Br |
| A-8 | Cl | F | H |
| A-9 | Cl | H | Cl |
| A-10 | Cl | Cl | Cl |
| A-11 | Cl | F | Cl |
| A-12 | Cl | Br | Cl |
| A-13 | Cl | H | Br |
| A-14 | Br | F | H |
| A-15 | Br | H | Br |
| A-16 | Br | F | Br |
| A-17 | Br | Cl | Br |
| A-18 | CF₃ | H | H |
| A-19 | CF₃ | H | F |
| A-20 | CF₃ | H | Cl |
| A-21 | CF₃ | H | Br |
| A-22 | CF₃ | H | CF₃ |
| A-23 | CF₃ | F | F |
| A-24 | CF₃ | F | Cl |
| A-25 | CF₃ | Cl | Cl |
| A-26 | CF₃ | F | H |
| A-27 | OCF₃ | H | F |
| A-28 | OCF₃ | H | Cl |
| A-29 | OCF₃ | F | H |
| A-30 | OCF₃ | H | CF₃ |
| A-31 | OCF₃ | H | H |

Groups A-8, A-9, and A-11 are more preferred patterns in formula I compounds. A-11 is particularly preferred.

R³ and R⁴ are preferably halogen such as Cl and F, NO₂, CN, CH₃, fluoromethyl such as CHF₂, CF₃, SCH₃, OCH₃. More preferably R⁴ is H, and R³ has one of the preferred meanings, particularly is Cl, or CH₃.

In another embodiment R³ and R⁴ together with the C-atoms they are bound to form a 5- or 6-membered saturated carbocyclic ring.

R⁵ is preferably H.

In one embodiment R⁶ is C₁-C₆-alkyl, C₁-C₄-haloalkyl, C₁-C₆-alkoxy, C₁-C₄-haloalkoxy, C₃-C₆alkenyl, S(O)ₘN(R¹⁰)₂, or N(R¹⁰)₂, which groups are unsubstituted or substituted with OH, C₁-C₄alkoxy, C(=O)OR^{a1}, C(=O)N(R^{a2})R^{a3}, CH=NOR^{a1}, or R⁶ is phenyl, benzyl, which rings are unsubstituted or substituted with halogen, C₁-C₄-alkyl, or C₁-C₄-haloalkyl, wherein R^{a1}, R^{a2}, R^{a3}, R¹⁰ are independently H, or C₁-C₄-alkyl.

Preferred embodiments relate to each of following compounds of formula I, wherein the variables are as defined in the outset and the preferred embodiments:

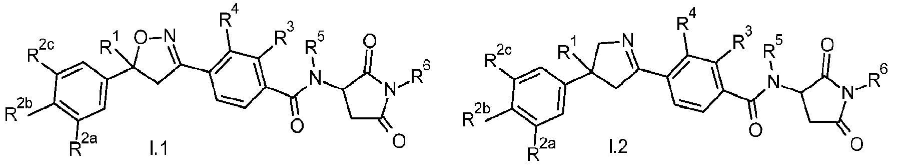

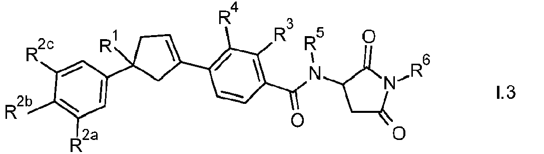

In particular with a view to their use, preference is given to the compounds of formula I compiled in the tables below, which compounds correspond to formulae 1.1, 1.2, and I.3, resp., more preferably in configuration I.a, particularly in I.Aa. Each of the groups mentioned for a substituent in the tables is furthermore per se, independently of the combination in which it is mentioned, a particularly preferred aspect of the substituent in question.
Table 1 : Compounds of formula I.1 in which R⁵ is H, R⁶ is CH₃, and the other variables for a compound correspond in each case to one row of Table B
Table 2 : Compounds of formula 1.2 in which R⁵ is H, R⁶ is CH₃, and the other variables for a compound correspond in each case to one row of Table B
Table 3 : Compounds of formula 1.3 in which R⁵ is H, R⁶ is CH₃, and the other variables for a compound correspond in each case to one row of Table B
Table 4 : Compounds of formula I.1 in which R⁵ is H, R⁶ is C₂H₅, and the other variables for a compound correspond in each case to one row of Table B
Table 5 : Compounds of formula 1.2 in which R⁵ is H, R⁶ is C₂H₅, and the other variables for a compound correspond in each case to one row of Table B
Table 6 : Compounds of formula 1.3 in which R⁵ is H, R⁶ is C₂H₅, and the other variables for a compound correspond in each case to one row of Table B
Table 7 : Compounds of formula I.1 in which R⁵ is H, R⁶ is CH₂CH₂CH₃, and the other variables for a compound correspond in each case to one row of Table B
Table 8 : Compounds of formula 1.2 in which R⁵ is H, R⁶ is CH₂CH₂CH₃, and the other variables for a compound correspond in each case to one row of Table B
Table 9 : Compounds of formula 1.3 in which R⁵ is H, R⁶ is CH₂CH₂CH₃, and the other variables for a compound correspond in each case to one row of Table B
Table 10 : Compounds of formula 1.1 in which R⁵ is H, R⁶ is CH₂(CH₃)₂, and the other variables for a compound correspond in each case to one row of Table B
Table 11 : Compounds of formula I.2 in which R⁵ is H, R⁶ is CH₂(CH₃)₂, and the other variables for a compound correspond in each case to one row of Table B
Table 12 : Compounds of formula 1.3 in which R⁵ is H, R⁶ is CH₂(CH₃)₂, and the other variables for a compound correspond in each case to one row of Table B
Table 13 : Compounds of formula 1.1 in which R⁵ is H, R⁶ is CH₂CH₂(CH₃)₂, and the other variables for a compound correspond in each case to one row of Table B
Table 14 : Compounds of formula I.2 in which R⁵ is H, R⁶ is CH₂CH₂(CH₃)₂, and the other variables for a compound correspond in each case to one row of Table B
Table 15 : Compounds of formula 1.3 in which R⁵ is H, R⁶ is CH₂CH₂(CH₃)₂, and the other variables for a compound correspond in each case to one row of Table B
Table 16 : Compounds of formula 1.1 in which R⁵ is H, R⁶ is CH₂CH=CH₂, and the other variables for a compound correspond in each case to one row of Table B
Table 17 : Compounds of formula I.2 in which R⁵ is H, R⁶ is CH₂CH=CH₂, and the other variables for a compound correspond in each case to one row of Table B
Table 18 : Compounds of formula 1.3 in which R⁵ is H, R⁶ is CH₂CH=CH₂, and the other variables for a compound correspond in each case to one row of Table B
Table 19 : Compounds of formula 1.1 in which R⁵ is H, R⁶ is CH₂CH₂F, and the other variables for a compound correspond in each case to one row of Table B
Table 20 : Compounds of formula I.2 in which R⁵ is H, R⁶ is CH₂CH₂F, and the other variables for a compound correspond in each case to one row of Table B
Table 21 : Compounds of formula 1.3 in which R⁵ is H, R⁶ is CH₂CH₂F, and the other variables for a compound correspond in each case to one row of Table B
Table 22 : Compounds of formula 1.1 in which R⁵ is H, R⁶ is CH₂CHF₂, and the other variables for a compound correspond in each case to one row of Table B
Table 23 : Compounds of formula I.2 in which R⁵ is H, R⁶ is CH₂CHF₂, and the other variables for a compound correspond in each case to one row of Table B
Table 24 : Compounds of formula 1.3 in which R⁵ is H, R⁶ is CH₂CHF₂, and the other variables for a compound correspond in each case to one row of Table B
Table 25 : Compounds of formula 1.1 in which R⁵ is H, R⁶ is CH₂CF₃, and the other variables for a compound correspond in each case to one row of Table B
Table 26 : Compounds of formula I.2 in which R⁵ is H, R⁶ is CH₂CF₃, and the other variables for a compound correspond in each case to one row of Table B
Table 27 : Compounds of formula 1.3 in which R⁵ is H, R⁶ is CH₂CF₃, and the other variables for a compound correspond in each case to one row of Table B
Table 28 : Compounds of formula 1.1 in which R⁵ is H, R⁶ is CH₂CH₂CF₃, and the other variables for a compound correspond in each case to one row of Table B
Table 29 : Compounds of formula I.2 in which R⁵ is H, R⁶ is CH₂CH₂CF₃, and the other variables for a compound correspond in each case to one row of Table B
Table 30 : Compounds of formula 1.3 in which R⁵ is H, R⁶ is CH₂CH₂CF₃, and the other variables for a compound correspond in each case to one row of Table B
Table 31 : Compounds of formula 1.1 in which R⁵ is H, R⁶ is CH₂CH₂OH, and the other variables for a compound correspond in each case to one row of Table B
Table 32 : Compounds of formula I.2 in which R⁵ is H, R⁶ is CH₂CH₂OH, and the other variables for a compound correspond in each case to one row of Table B
Table 33 : Compounds of formula 1.3 in which R⁵ is H, R⁶ is CH₂CH₂OH, and the other variables for a compound correspond in each case to one row of Table B
Table 34 : Compounds of formula 1.1 in which R⁵ is H, R⁶ is CH₂CH₂OCH₃, and the other variables for a compound correspond in each case to one row of Table B
Table 35 : Compounds of formula I.2 in which R⁵ is H, R⁶ is CH₂CH₂OCH₃, and the other variables for a compound correspond in each case to one row of Table B
Table 36 : Compounds of formula 1.3 in which R⁵ is H, R⁶ is CH₂CH₂OCH₃, and the other variables for a compound correspond in each case to one row of Table B
Table 37 : Compounds of formula 1.1 in which R⁵ is H, R⁶ is c-C₃H₅, and the other variables for a compound correspond in each case to one row of Table B
Table 38 : Compounds of formula 1.2 in which R⁵ is H, R⁶ is c-C₃H₅, and the other variables for a compound correspond in each case to one row of Table B
Table 39 : Compounds of formula 1.3 in which R⁵ is H, R⁶ is c-C₃H₅, and the other variables for a compound correspond in each case to one row of Table B
Table 40 : Compounds of formula 1.1 in which R⁵ is H, R⁶ is CH₂C₆H₅, and the other variables for a compound correspond in each case to one row of Table B
Table 41 : Compounds of formula 1.2 in which R⁵ is H, R⁶ is CH₂C₆H₅, and the other variables for a compound correspond in each case to one row of Table B
Table 42 : Compounds of formula 1.3 in which R⁵ is H, R⁶ is CH₂C₆H₅, and the other variables for a compound correspond in each case to one row of Table B
Table 43 : Compounds of formula 1.1 in which R⁵ is H, R⁶ is OCH₃, and the other variables for a compound correspond in each case to one row of Table B
Table 44 : Compounds of formula 1.2 in which R⁵ is H, R⁶ is OCH₃, and the other variables for a compound correspond in each case to one row of Table B
Table 45 : Compounds of formula 1.3 in which R⁵ is H, R⁶ is OCH₃, and the other variables for a compound correspond in each case to one row of Table B
Table 46 : Compounds of formula 1.1 in which R⁵ is H, R⁶ is OCH₂CH₃, and the other variables for a compound correspond in each case to one row of Table B
Table 47 : Compounds of formula 1.2 in which R⁵ is H, R⁶ is OCH₂CH₃, and the other variables for a compound correspond in each case to one row of Table B
Table 48 : Compounds of formula 1.3 in which R⁵ is H, R⁶ is OCH₂CH₃, and the other variables for a compound correspond in each case to one row of Table B
Table 49 : Compounds of formula 1.1 in which R⁵ is H, R⁶ is OCH₂CF₃, and the other variables for a compound correspond in each case to one row of Table B
Table 50 : Compounds of formula 1.2 in which R⁵ is H, R⁶ is OCH₂CF₃, and the other variables for a compound correspond in each case to one row of Table B
Table 51 : Compounds of formula 1.3 in which R⁵ is H, R⁶ is OCH₂CF₃, and the other variables for a compound correspond in each case to one row of Table B
Table 52 : Compounds of formula 1.1 in which R⁵ is H, R⁶ is SO₂N(CH₃)₂, and the other variables for a compound correspond in each case to one row of Table B
Table 53 : Compounds of formula 1.2 in which R⁵ is H, R⁶ is SO₂N(CH₃)₂, and the other variables for a compound correspond in each case to one row of Table B
Table 54 : Compounds of formula 1.3 in which R⁵ is H, R⁶ is SO₂N(CH₃)₂, and the other variables for a compound correspond in each case to one row of Table B
Table 55 : Compounds of formula 1.1 in which R⁵ is H, R⁶ is N(CH₃)₂, and the other variables for a compound correspond in each case to one row of Table B
Table 56 : Compounds of formula 1.2 in which R⁵ is H, R⁶ is N(CH₃)₂, and the other variables for a compound correspond in each case to one row of Table B
Table 57 : Compounds of formula 1.3 in which R⁵ is H, R⁶ is N(CH₃)₂, and the other variables for a compound correspond in each case to one row of Table B
Table 58 : Compounds of formula 1.1 in which R⁵ is H, R⁶ is CH₂C(=O)OCH₃, and the other variables for a compound correspond in each case to one row of Table B
Table 59 : Compounds of formula 1.2 in which R⁵ is H, R⁶ is CH₂C(=O)OCH₃, and the other variables for a compound correspond in each case to one row of Table B
Table 60 : Compounds of formula 1.3 in which R⁵ is H, R⁶ is CH₂C(=O)OCH₃, and the other variables for a compound correspond in each case to one row of Table B
Table 61 : Compounds of formula 1.1 in which R⁵ is H, R⁶ is CH₂C(=O)OC₂H₅, and the other variables for a compound correspond in each case to one row of Table B
Table 62 : Compounds of formula 1.2 in which R⁵ is H, R⁶ is CH₂C(=O)OC₂H₅, and the other variables for a compound correspond in each case to one row of Table B
Table 63 : Compounds of formula 1.3 in which R⁵ is H, R⁶ is CH₂C(=O)OC₂H₅, and the other variables for a compound correspond in each case to one row of Table B

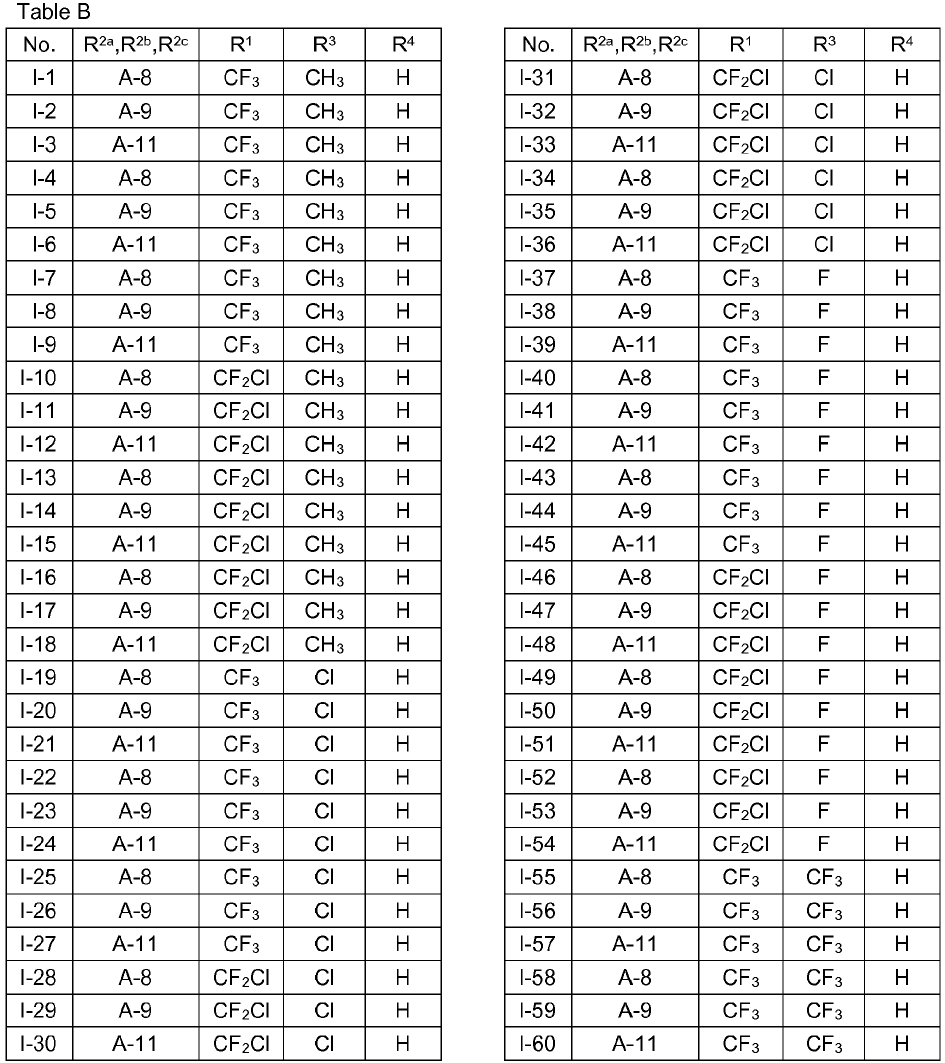

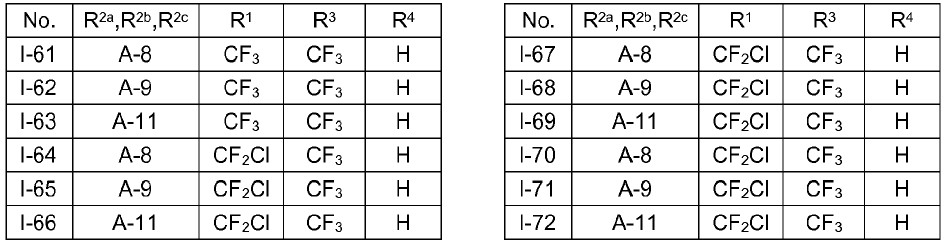

A preferred embodiment relates to compounds in configuration I.a, particularly I.Aa, which correspond to formulae I.1, I.2, or I.3, particularly to formula I.1. In such compounds ring "A" is substituted by patterns A-8, A-9, or A-11, R¹ is CF₂Cl or CF₃, R³ is Cl or CH₃, R⁴ and R⁵ are H, and R⁶ is CH₃, C₂H₅, CH₂CH₂CH₃, CH₂(CH₃)₂, CH₂CH₂(CH₃)₂, CH₂CH=CH₂, CH₂CH₂F, CH₂CHF₂, CH₂CF₃, CH₂CH₂CF₃, c-C₃H₅, CH₂CH₂OH, CH₂CH₂OCH₃, CH₂C₆H₅, CH₂C(=O)OCH₃, CH₂C(=O)OC₂H₅, OCH₃, OC₂H₅, OCH₂CF₃, SO₂N(CH₃)₂, or N(CH₃)₂.

More preferred embodiments relate to compounds in configuration I.a, particularly I.Aa, which correspond to formulae I.1, 1.2, or 1.3, particularly to formula 1.1. In such compounds ring "A" is substituted by patterns A-8, A-9, or A-11, R¹ is CF₃, R³ is Cl or CH₃, R⁴ and R⁵ are H, and R⁶ is C₁-C₄-alkyl, C₁-C₃-haloalkyl, C₃-C₄-cycloalkyl, C₁-C₃-alkoxy, C₁-C₃-haloalkoxy, C₁-C₄-alkylamino, and di-C₁-C₄-alkylamino. Particularly preferred compounds are selected from following compounds of formula 1.1, preferably in at least 85% by weight of the given isomer:

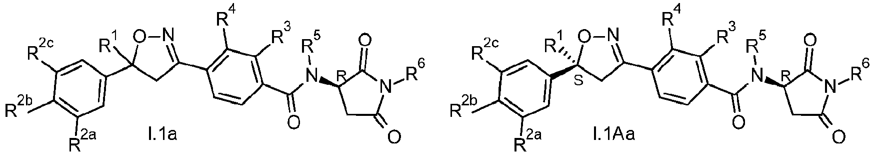

| No. | R¹ | R^{2a} | R^{2b} | R^{2c} | R³ | R⁴ | R⁵ | R⁶ | Isomer |
|---|---|---|---|---|---|---|---|---|---|
| I-1-13 | CF₃ | Cl | F | Cl | Cl | H | H | CH₃ | I.1Aa |
| I-1-19 | CF₃ | Cl | H | Cl | Cl | H | H | CH₃ | I.1a |
| I-1-20 | CF₃ | Cl | F | Cl | Cl | H | H | N(CH₃)₂ | I.1Aa |
| I-1-21 | CF₃ | Cl | F | Cl | Cl | H | H | CH₂CH₃ | I.1Aa |
| I-1-24 | CF₃ | Cl | F | Cl | Cl | H | H | CH₂CHF₂ | I.1Aa |
| I-1-25 | CF₃ | Cl | F | Cl | Cl | H | H | c-C₃H₅ | I.1Aa |
| I-1-28 | CF₃ | Cl | H | Cl | CH₃ | H | H | CH₃ | I.1a |
| I-1-32 | CF₃ | Cl | F | Cl | Cl | H | H | CH₃ | I.1A |
| I-1-40 | CF₃ | Cl | F | Cl | Cl | H | H | OCH₃ | I.1Aa |
| I-1-47 | CF₃ | Cl | H | Cl | CH₃ | H | H | CH(CH₃)₂ | I.1a |
| I-1-48 | CF₃ | Cl | H | Cl | Cl | H | H | CH(CH₃)₂ | I.1a |
| I-1-51 | CF₃ | Cl | H | Cl | CH₃ | H | H | CH₂CH₃ | I.1a |
| I-1-52 | CF₃ | Cl | H | Cl | Cl | H | H | CH₂CH₃ | I.1a |
| I-1-76 | CF₃ | Cl | F | Cl | Cl | H | H | OCH₂CF₃ | I.1Aa |
| I-1-80 | CF₃ | Cl | F | Cl | Cl | H | H | CH₂CH(CH₃)₂ | I.1Aa |
| I-1-82 | CF₃ | Cl | F | Cl | Cl | H | H | OCH₂CH₃ | I.1Aa |

As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

The invention also relates to a mixture of at least one compound of the invention with at least one mixing partner as defined herein after. Preferred are binary mixtures of one compound of the invention as component I with one mixing partner as defined herein after as component II. Preferred weight ratios for such binary mixtures are from 5000:1 to 1:5000, preferably from 1000:1 to 1:1000, more preferably from 100:1 to 1:100, particularly preferably from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers, and the like. Preferred mixing partners are insecticides, nematicides, and fungicides.

The following list M of pesticides, grouped and numbered according the Mode of Action Classification of the Insecticide Resistance Action Committee (IRAC), together with which the compounds of the invention can be used and with which potential synergistic effects might be produced, is intended to illustrate the possible combinations, but not to impose any limitation:
M.1 Acetylcholine esterase (AChE) inhibitors: M.1A carbamates, e.g. aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb and triazamate; or M.1B organophosphates, e.g. acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, and vamidothion;
M.2. GABA-gated chloride channel antagonists: M.2A cyclodiene organochlorine compounds, e.g. endosulfan or chlordane; or M.2B fiproles (phenylpyrazoles), e.g. ethiprole, fipronil, flufiprole, pyrafluprole, and pyriprole;
M.3 Sodium channel modulators from the class of M.3A pyrethroids, e.g. acrinathrin, allethrin, d-cis-trans allethrin, d-trans allethrin, bifenthrin, kappa-bifenthrin, bioallethrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappatefluthrin, tetramethylfluthrin, tetramethrin, tralomethrin, and transfluthrin; or M.3B sodium channel modulators such as DDT or methoxychlor;
M.4 Nicotinic acetylcholine receptor agonists (nAChR): M.4A neonicotinoids, e.g. acetamiprid, clothianidin, cycloxaprid, dinotefuran, imidacloprid, nitenpyram, thiacloprid and thiamethoxam; or the compounds M.4A.1 4,5-Dihydro-N-nitro-1-(2-oxiranylmethyl)-1H-imidazol-2-amine,
M.4A.2: (2E-)-1-[(6-Chloropyridin-3-yl)methyl]-N'-nitro-2-pentylidenehydrazinecarboximidamide; or M4.A.3: 1-[(6-Chloropyridin-3-yl)methyl]-7-methyl-8-nitro-5-propoxy-1,2,3,5,6,7-hexahydroimidazo[1,2-a]pyridine; or M.4B nicotine; M.4C sulfoxaflor; M.4D flupyradifurone; M.4E triflumezopyrim;
M.5 Nicotinic acetylcholine receptor allosteric activators: spinosyns, e.g. spinosad or spinetoram;
M.6 Chloride channel activators from the class of avermectins and milbemycins, e.g. abamectin, emamectin benzoate, ivermectin, lepimectin, or milbemectin;
M.7 Juvenile hormone mimics, such as M.7A juvenile hormone analogues hydroprene, kinoprene, and methoprene; or M.7B fenoxycarb, or M.7C pyriproxyfen;
M.8 miscellaneous non-specific (multi-site) inhibitors, e.g. M.8A alkyl halides as methyl bromide and other alkyl halides, M.8B chloropicrin, M.8C sulfuryl fluoride, M.8D borax, or M.8E tartar emetic;
M.9 Chordotonal organ TRPV channel modulators, e.g. M.9B pymetrozine; pyrifluquinazon;
M.10 Mite growth inhibitors, e.g. M.10A clofentezine, hexythiazox, and diflovidazin, or M.10B etoxazole;
M.11 Microbial disruptors of insect midgut membranes, e.g. *bacillus thuringiensis* or *bacillus sphaericus,* and the insecticdal proteins they produce such as *bacillus thuringiensis subsp. is-raelensis, bacillus sphaericus, bacillus thuringiensis subsp. aizawai, bacillus thuringiensis subsp. kurstaki* and *bacillus thuringiensis subsp. tenebrionis,* or the Bt crop proteins: Cry1Ab, Cry1Ac, Cry1Fa, Cry2Ab, mCry3A, Cry3Ab, Cry3Bb, and Cry34/35Ab1;
M.12 Inhibitors of mitochondrial ATP synthase, e.g. M.12A diafenthiuron, or M.12B organotin miticides such as azocyclotin, cyhexatin, orfenbutatin oxide, M.12C propargite, or M.12D tetradifon;
M.13 Uncouplers of oxidative phosphorylation via disruption of the proton gradient, e.g. chlorfenapyr, DNOC, or sulfluramid;
M.14 Nicotinic acetylcholine receptor (nAChR) channel blockers, e.g. nereistoxin analogues bensultap, cartap hydrochloride, thiocyclam, or thiosultap sodium;
M.15 Inhibitors of the chitin biosynthesis type 0, such as benzoylureas e.g. bistrifluron, chlorfluazuron, diflubenzuron, flucycloxuron, flufenoxuron, hexaflumuron, lufenuron, novaluron, noviflumuron, teflubenzuron, or triflumuron;
M.16 Inhibitors of the chitin biosynthesis type 1, e.g. buprofezin;
M.17 Moulting disruptors, Dipteran, e.g. cyromazine;
M.18 Ecdyson receptor agonists such as diacylhydrazines, e.g. methoxyfenozide, tebufenozide, halofenozide, fufenozide, or chromafenozide;
M.19 Octopamin receptor agonists, e.g. amitraz;
M.20 Mitochondrial complex III electron transport inhibitors, e.g. M.20A hydramethylnon, M.20B acequinocyl, M.20C fluacrypyrim; or M.20D bifenazate;
M.21 Mitochondrial complex I electron transport inhibitors, e.g. M.21A METI acaricides and insecticides such as fenazaquin, fenpyroximate, pyrimidifen, pyridaben, tebufenpyrad or tolfenpyrad, or M.21B rotenone;
M.22 Voltage-dependent sodium channel blockers, e.g. M.22A indoxacarb, M.22B metaflumizone, or M.22B.1: 2-[2-(4-Cyanophenyl)-1-[3-(trifluoromethyl)phenyl]ethylidene]-N-[4-(difluoromethoxy)phenyl]-hydrazinecarboxamide or M.22B.2: N-(3-Chloro-2-methylphenyl)-2-[(4-chlorophenyl)[4-[methyl(methylsulfonyl)amino]phenyl]methylene]-hydrazinecarboxamide;
M.23 Inhibitors of the of acetyl CoA carboxylase, such as Tetronic and Tetramic acid derivatives, e.g. spirodiclofen, spiromesifen, or spirotetramat; M.23.1 spiropidion;
M.24 Mitochondrial complex IV electron transport inhibitors, e.g. M.24A phosphine such as aluminium phosphide, calcium phosphide, phosphine or zinc phosphide, or M.24B cyanide;
M.25 Mitochondrial complex II electron transport inhibitors, such as beta-ketonitrile derivatives, e.g. cyenopyrafen or cyflumetofen;
M.28 Ryanodine receptor-modulators from the class of diamides, e.g. flubendiamide, chlorantraniliprole, cyantraniliprole, tetraniliprole, M.28.1: (R)-3-Chlor-N1-{2-methyl-4-[1,2,2,2 -tetrafluoro-1-(trifluoromethyl)ethyl]phenyl}-N2-(1-methyl-2-methylsulfonylethyl)phthalamid, M.28.2: (S)-3-Chloro-N1-{2-methyl-4-[1,2,2,2-tetrafluoro-1-(trifluoromethyl)ethyl]phenyl}-N2-(1-methyl-2-methylsulfonylethyl)phthalamid, M.28.3: cyclaniliprole, or M.28.4: methyl-2-[3,5-dibromo-2-({[3-bromo-1-(3-chlorpyridin-2-yl)-1H-pyrazol-5-yl]carbonyl}amino)benzoyl]-1,2-dimethylhydrazine-carboxylate; or M.28.5a) N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; M.28.5b) N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; M.28.5c) N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; M.28.5d) N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; M.28.5h) N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide; M.28.5i) N-[2-(5-Amino-1,3,4-thiadiazol-2-yl)-4-chloro-6-methylphenyl]-3-bromo-1-(3-chloro-2-pyridinyl)-1H-pyrazole-5-carboxamide; M.28.5j) 3-Chloro-1-(3-chloro-2-pyridinyl)-N-[2,4-dichloro-6-[[(1-cyano-1-methylethyl)amino]carbonyl]phenyl]-1H-pyrazole-5-carboxamide; M.28.5k) 3-Bromo-N-[2,4-dichloro-6-(methylcarbamoyl)phenyl]-1-(3,5-dichloro-2-pyridyl)-1H-pyrazole-5-carboxamide; M.28.5I) N-[4-Chloro-2-[[(1,1-dimethylethyl)amino]carbonyl]-6-methylphenyl]-1-(3-chloro-2-pyridinyl)-3-(fluoromethoxy)-1H-pyrazole-5-carboxamide; or
M.28.6: cyhalodiamide; or
M.29: Chordotonal organ Modulators - undefined target site, e.g. flonicamid;
M.UN. insecticidal active compounds of unknown or uncertain mode of action, e.g. afidopyropen, afoxolaner, azadirachtin, amidoflumet, benzoximate, broflanilide, bromopropylate, chinomethionat, cryolite, dicloromezotiaz, dicofol, flufenerim, flometoquin, fluensulfone, fluhexafon, fluopyram, fluralaner, metaldehyde, metoxadiazone, piperonyl butoxide, pyflubumide, pyridalyl, tioxazafen, M.UN.3: 11-(4-chloro-2,6-dimethylphenyl)-12-hydroxy-1,4-dioxa-9-azadispiro[4.2.4.2]-tetradec-11-en-10-one,
M.UN.4: 3-(4'-fluoro-2,4-dimethylbiphenyl-3-yl)-4-hydroxy-8-oxa-1-azaspiro[4.5]dec-3-en-2-one,
M.UN.5: 1-[2-fluoro-4-methyl-5-[(2,2,2-trifluoroethyl)sulfinyl]phenyl]-3-(trifluoromethyl)-1H-1,2,4-triazole-5-amine, or actives on basis of *bacillus firmus* (Votivo, I-1582);
M.UN.6: flupyrimin;
M.UN.8: fluazaindolizine; M.UN.9.a): 4-[5-(3,5-dichlorophenyl)-5-(trifluoromethyl)-4H-isoxazol-3-yl]-2-methyl-N-(1-oxothietan-3-yl)benzamide; M.UN.9.b): fluxametamide; M.UN.10: 5-[3-[2,6-dichloro-4-(3,3-dichloroallyloxy)phenoxy]propoxy]-1H-pyrazole;
M.UN.11.i) 4-cyano-N-[2-cyano-5-[[2,6-dibromo-4-[1,2,2,3,3,3-hexafluoro-1-(trifluoromethyl)-propyl]phenyl]carbamoyl]phenyl]-2-methyl-benzamide; M.UN.11.j) 4-cyano-3-[(4-cyano-2-methyl-benzoyl)amino]-N-[2,6-dichloro-4-[1,2,2,3,3,3-hexafluoro-1-(trifluoromethyl)propyl]phenyl]-2-fluoro-benzamide; M.UN.11.k) N-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; M.UN.11.1) N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; M.UN.11.m) N-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; M.UN.11.n) 4-cyano-N-[2-cyano-5-[[2,6-dichloro-4-[1,2,2,3,3,3-hexafluoro-1-(trifluoromethyl)propyl]phenyl]carbamoyl]phenyl]-2-methyl-benzamide; M.UN.11.o) 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide; M.UN.11.p) N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; or
M.UN.12.a) 2-(1,3-Dioxan-2-yl)-6-[2-(3-pyridinyl)-5-thiazolyl]-pyridine; M.UN.12.b) 2-[6-[2-(5-Fluoro-3-pyridinyl)-5-thiazolyl]-2-pyridinyl]-pyrimidine; M.UN.12.c) 2-[6-[2-(3-Pyridinyl)-5-thiazolyl]-2-pyridinyl]-pyrimidine; M.UN.12.d) N-Methylsulfonyl-6-[2-(3-pyridyl)thiazol-5-yl]pyridine-2-carboxamide; M.UN.12.e) N-Methylsulfonyl-6-[2-(3-pyridyl)thiazol-5-yl]pyridine-2-carboxamide;
M.UN.14a) 1-[(6-Chloro-3-pyridinyl)methyl]-1,2,3,5,6,7-hexahydro-5-methoxy-7-methyl-8-nitro-imidazo[1,2-a]pyridine; or M.UN.14b) 1-[(6-Chloropyridin-3-yl)methyl]-7-methyl-8-nitro-1,2,3,5,6,7-hexahydroimidazo[1,2-a]pyridin-5-ol;
M.UN.16a) 1-isopropyl-N,5-dimethyl-N-pyridazin-4-yl-pyrazole-4-carboxamide; or M.UN.16b) 1-(1,2-dimethylpropyl)-N-ethyl-5-methyl-N-pyridazin-4-yl-pyrazole-4-carboxamide; M.UN.16c) N,5-dimethyl-N-pyridazin-4-yl-1-(2,2,2-trifluoro-1-methyl-ethyl)pyrazole-4-carboxamide; M.UN.16d) 1-[1-(1-cyanocyclopropyl)ethyl]-N-ethyl-5-methyl-N-pyridazin-4-yl-pyrazole-4-carboxamide; M.UN.16e) N-ethyl-1-(2-fluoro-1-methyl-propyl)-5-methyl-N-pyridazin-4-yl-pyrazole-4-carboxamide; M.UN.16f) 1-(1,2-dimethylpropyl)-N,5-dimethyl-N-pyridazin-4-yl-pyrazole-4-carboxamide; M.UN.16g) 1-[1-(1-cyanocyclopropyl)ethyl]-N,5-dimethyl-N-pyridazin-4-yl-pyrazole-4-carboxamide; M.UN.16h) N-methyl-1-(2-fluoro-1-methyl-propyl]-5-methyl-N-pyridazin-4-yl-pyrazole-4-carboxamide; M.UN.16i) 1-(4,4-difluorocyclohexyl)-N-ethyl-5-methyl-N-pyridazin-4-yl-pyrazole-4-carboxamide; or M.UN.16j) 1-(4,4-difluorocyclohexyl)-N,5-dimethyl-N-pyridazin-4-yl-pyrazole-4-carboxamide,
M.UN.17a) N-(1-methylethyl)-2-(3-pyridinyl)-2H-indazole-4-carboxamide; M.UN.17b) N-cyclopropyl-2-(3-pyridinyl)-2H-indazole-4-carboxamide; M.UN.17c) N-cyclohexyl-2-(3-pyridinyl)-2H-indazole-4-carboxamide; M.UN.17d) 2-(3-pyridinyl)-N-(2,2,2-trifluoroethyl)-2H-indazole-4-carboxamide; M.UN.17e) 2-(3-pyridinyl)-N-[(tetrahydro-2-furanyl)methyl]-2H-indazole-5-carboxamide; M.UN.17f) methyl 2-[[2-(3-pyridinyl)-2H-indazol-5-yl]carbonyl]hydrazinecarboxylate; M.UN.17g) N-[(2,2-difluorocyclopropyl)methyl]-2-(3-pyridinyl)-2H-indazole-5-carboxamide; M.UN.17h) N-(2,2-difluoropropyl)-2-(3-pyridinyl)-2H-indazole-5-carboxamide; M.UN.17i) 2-(3-pyridinyl)-N-(2-pyrimidinylmethyl)-2H-indazole-5-carboxamide; M.UN.17j) N-[(5-methyl-2-pyrazinyl)methyl]-2-(3-pyridinyl)-2H-indazole-5-carboxamide,
M.UN.18. tyclopyrazoflor; M.UN.19 sarolaner; M.UN.20 lotilaner;
M.UN.21 N-[4-Chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; M.UN.22a 2-(3-ethylsulfonyl-2-pyridyl)-3-methyl-6-(trifluoromethyl)imidazo[4,5-b]pyridine, or M.UN.22b 2-[3-ethylsulfonyl-5-(trifluoromethyl)-2-pyridyl]-3-methyl-6-(trifluoromethyl)imidazo[4,5-b]pyridine; M.UN.23a) 4-[5-(3,5-dichlorophenyl)-5-(trifluoromethyl)-4H-isoxazol-3-yl]-N-[(4R)-2-ethyl-3-oxo-isoxazolidin-4-yl]-2-methyl-benzamide, or M.UN.23b) 4-[5-(3,5-dichloro-4-fluoro-phenyl)-5-(trifluoromethyl)-4H-isoxazol-3-yl]-N-[(4R)-2-ethyl-3-oxo-isoxazolidin-4-yl]-2-methyl-benzamide; M.UN.24a) N-[4-chloro-3-(cyclopropylcarbamoyl)phenyl]-2-methyl-5-(1,1,2,2,2-pentafluoroethyl)-4-(trifluoromethyl)pyrazole-3-carboxamide or M.UN.24b) N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; M.UN.25 acynonapyr; M.UN.26 benzpyrimoxan; M.UN.27 Tigolaner; M.UN.28 Oxazosulfyl; M.UN.29a) [(2S,3R,4R,5S,6S)-3,5-dimethoxy-6-methyl-4-propoxy-tetrahydropyran-2-yl] N-[4-[1-[4-(trifluoromethoxy)phenyl]-1,2,4-triazol-3-yl]phenyl]carbamate; M.UN.29b) [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate; M.UN.29c) [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; M.UN.29d) [(2S,3R,4R,5S,6S)-3,4,5-trimethoxy-6-methyl-tetrahydropyran-2-yl] N-[4-[1-[4-(1,1,2,2,2-pentafluoroethoxy)phenyl]-1,2,4-triazol-3-yl]phenyl]carbamate; M.UN.29.e) (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one or M.UN.29f) (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one.

The commercially available compounds of the group M listed above may be found in The Pesticide Manual, 16th Edition, C. MacBean, British Crop Protection Council (2013) among other publications. The online Pesticide Manual is updated regularly and is accessible through http://bcpcdata.com/pesticide-manual.html.

Another online data base for pesticides providing the ISO common names is http://www.alanwood.net/pesticides.

The commercially available compounds of the group M listed above may be found in The Pesticide Manual, 17th Edition, C. MacBean, British Crop Protection Council (2015) among other publications. The online Pesticide Manual is updated regularly and is accessible through http://bcpcdata.com/pesticide-manual.html.

Another online data base for pesticides providing the ISO common names is http://www.alanwood.net/pesticides.

M.4 cycloxaprid is known from WO2010/069266 and WO2011/069456. M.4A.1 is known from CN 103814937; CN105367557, CN 105481839. M.4A.2, guadipyr, is known from WO 2013/003977, and M.4A.3 (paichongding) is known from WO 2007/101369. M.22B.1 is described in CN10171577 and M.22B.2 in CN102126994. Spiropidion M.23.1 is known from WO 2014/191271. M.28.1 and M.28.2 are known from WO2007/101540. M.28.3 is described in WO2005/077934. M.28.4 is described in WO2007/043677. M.28.5a) to M.28.5d) and M.28.5h) are described in WO 2007/006670, WO2013/024009 and WO 2013/024010, M.28.5i) is described in WO2011/085575, M.28.5j) in WO2008/134969, M.28.5k) in US2011/046186 and M.28.51) in WO2012/034403. M.28.6 can be found in WO2012/034472. M.UN.3 is known from WO2006/089633 and M.UN.4 from WO2008/067911. M.UN.5 is described in WO2006/043635, and biological control agents on the basis of *bacillus firmus* are described in WO2009/124707. Flupyrimin is described in WO2012/029672. M.UN.8 is known from WO2013/055584. M.UN.9.a) is described in WO2013/050317. M.UN.9.b) is described in WO2014/126208. M.UN.10 is known from WO2010/060379. Broflanilide and M.UN.11.b) to M.UN.11.h) are described in WO2010/018714, and M.UN.11i) to M.UN.11.p) in WO 2010/127926. M.UN.12.a) to M.UN.12.c) are known from WO2010/006713, M.UN.12.d) and M.UN.12.e) are known from WO2012/000896. M.UN.14a) and M.UN.14b) are known from WO2007/101369. M.UN.16.a) to M.UN.16h) are described in WO2010/034737, WO2012/084670, and WO2012/143317, resp., and M.UN.16i) and M.UN.16j) are described in WO20151055497. M.UN.17a) to M.UN.17.j) are described in WO2015/038503. M.UN.18 Tycloprazoflor is described in US2014/0213448. M.UN.19 is described in WO2014/036056. M.UN.20 is known from WO2014/090918. M.UN.21 is known from EP2910126. M.UN.22a and M.UN.22b are known from WO2015/059039 and WO2015/190316. M.UN.23a and M.UN.23b are known from WO2013/050302. M.UN.24a) and M.UN.24b) are known from WO2012/126766. Acynonapyr M.UN.25 is known from WO 2011/105506. Benzpyrimoxan M.UN.26 is known from WO2016/104516. M.UN.27 is known from WO2016/174049. M.UN.28 Oxazosulfyl is known from WO2017/104592. M.UN.29a) to M.UN.29f) are known from WO2009/102736 or WO2013116053.

The following list of fungicides, in conjunction with which the compounds of the invention can be used, is intended to illustrate the possible combinations but does not limit them:

### A) Respiration inhibitors

- Inhibitors of complex III at Qₒ site (e. g. strobilurins): azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxy.strobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenyl-pyrazol-3-yl)oxylmethyl]phenyl]-N-methoxy-carbamate (A.1.22), 1-[3-chloro-2-[[1-(4-chlorophenyl)-1H-pyrazol-3-yl]oxymethyl]phenyl]-4-methyl-tetrazol-5-one (A.1.23), 1-[3-bromo-2-[[1-(4-chlorophenyl)pyrazol-3-yl]oxymethyl]phenyl]-4-methyl-tetrazol-5-one (A.1.24), 1-[2-[[1-(4-chlorophenyl)pyrazol-3-yl]oxymethyl]-3-methyl-phenyl]-4-methyl-tetrazol-5-one (A.1.25), 1-[2-[[1-(4-chlorophenyl)pyrazol-3-yl]oxymethyl]-3-fluoro-phenyl]-4-methyl-tetrazol-5-one (A.1.26), 1-[2-[[1-(2,4-dichlorophenyl)pyrazol-3-yl]oxymethyl]-3-fluoro-phenyl]-4-methyl-tetrazol-5-one (A.1.27), 1-[2-[[4-(4-chlorophenyl)thiazol-2-yl]oxymethyl]-3-methyl-phenyl]-4-methyl-tetrazol-5-one (A.1.28), 1-[3-chloro-2-[[4-(p-tolyl)thiazol-2-yl]oxymethyl]phenyl]-4-methyl-tetrazol-5-one (A.1.29), 1-[3-cyclopropyl-2-[[2-methyl-4-(1-methylpyrazol-3-yl)phenoxy]methyl]phenyl]-4-methyl-tetrazol-5-one (A.1.30), 1-[3-(difluoromethoxy)-2-[[2-methyl-4-(1-methylpyrazol-3-yl)phenoxy]methyl]phenyl]-4-methyl-tetrazol-5-one (A.1.31), 1-methyl-4-[3-methyl-2-[[2-methyl-4-(1-methylpyrazol-3-yl)phenoxy]methyl]phenyl]tetrazol-5-one (A.1.32), 1-methyl-4-[3-methyl-2-[[1-[3-(trifluoromethyl)phenyl]-ethylideneamino]oxymethyl]phenyl]tetrazol-5-one (A.1.33), (*Z*,2*E*-)-5-[1-(2,4-dichlorophenyl)pyrazol-3-yl]-oxy-2-methoxyimino-*N*,3-dimethyl-pent-3-enamide (A.1.34), (*Z*,2*E*-)-5-[1-(4-chlorophenyl)pyrazol-3-yl]oxy-2-methoxyimino-*N*,3-dimethyl-pent-3-enamide (A.1.35), (*Z*,2*E*-)-5-[1-(4-chloro-2-fluoro-phenyl)pyrazol-3-yl]oxy-2-methoxyimino-*N*,3-dimethyl-pent-3-enamide (A.1.36),
- inhibitors of complex III at Qᵢ site: cyazofamid (A.2.1), amisulbrom (A.2.2), [(3S,6S,7R,8R)-8-benzyl-3-[(3-acetoxy-4-methoxy-pyridine-2-carbonyl)amino]-6-methyl-4,9-dioxo-1,5-dioxonan-7-yl]2-methylpropanoate (A.2.3), [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl]2-methylpropanoate (A.2.4), [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate (A.2.5), [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl]2-methylpropanoate (A.2.6); (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate (A.2.7), (3S,6S,7R,8R)-8-benzyl-3-[3-[(isobutyryloxy)methoxy]-4-methoxypicolinamido]-6-methyl-4,9-dioxo-1,5-dioxonan-7-yl isobutyrate (A.2.8);
- inhibitors of complex II (e. g. carboxamides): benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.14), penthiopyrad (A.3.15), sedaxane (A.3.16), tecloftalam (A.3.17), thifluzamide (A.3.18), N-(4'-trifluoromethylthiobiphenyl-2-yl)-3-difluoromethyl-1-methyl-1H-pyrazole-4-carboxamide (A.3.19), N-(2-(1,3,3-trimethyl-butyl)-phenyl)-1,3-dimethyl-5-fluoro-1H-pyrazole-4-carboxamide (A.3.20), 3-(difluoromethyl)-1-methyl-N-(1,1,3-trimethylindan-4-yl)pyrazole-4-carboxamide (A.3.21), 3-(trifluoromethyl)-1-methyl-N-(1,1,3-trimethylindan-4-yl)pyrazole-4-carboxamide (A.3.22), 1,3-dimethyl-N-(1,1,3-trimethylindan-4-yl)pyrazole-4-carboxamide (A.3.23), 3-(trifiuoromethyl)-1,5-dimethyl-N-(1,1,3-trimethylindan-4-yl)pyrazole-4-carboxamide (A.3.24), 1,3,5-trimethyl-N-(1,1,3-trimethylindan-4-yl)pyrazole-4-carboxamide (A.3.25), N-(7-fluoro-1,1,3-trimethyl-indan-4-yl)-1,3-dimethyl-pyrazole-4-carboxamide (A.3.26), N-[2-(2,4-dichlorophenyl)-2-methoxy-1-methyl-ethyl]-3-(difluoromethyl)-1-methyl-pyrazole-4-carboxamide (A.3.27);
- other respiration inhibitors (e. g. complex I, uncouplers): diflumetorim (A.4.1), (5,8-difluoroquinazolin-4-yl)-{2-[2-fluoro-4-(4-trifluoromethylpyridin-2-yloxy)-phenyl]-ethyl}-amine (A.4.2); nitrophenyl derivates: binapacryl (A.4.3), dinobuton (A.4.4), dinocap (A.4.5), fluazinam (A.4.6); ferimzone (A.4.7); organometal compounds: fentin salts, such as fentin-acetate (A.4.8), fentin chloride (A.4.9) or fentin hydroxide (A.4.10); ametoctradin (A.4.11); and silthiofam (A.4.12);

### B) Sterol biosynthesis inhibitors (SBI fungicides)

- C14 demethylase inhibitors (DMI fungicides): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[*rel-*(2*S*;3*R*)-3-(2-chlorophenyl)-2-(2,4-difluorophenyl)-oxiranylmethyl]-5-thiocyanato-1H-[1,2,4]triazolo (B.1.31), 2-[*rel-*(2*S*;3*R*)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chlorophenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), **2-[4-(4-chloro**phenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1-(1,2,4-triazol-1-yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), 2-[2-chloro-4-(4-chlorophenoxy)phenyl]-1-(1,2,4-triazol-1-yl)pent-3-yn-2-ol (B.1.51); imidazoles: imazalil (B.1.42), pefurazoate (B.1.43), prochloraz (B.1.44), triflumizol (B.1.45); pyrimidines, pyridines and piperazines: fenarimol (B.1.46), nuarimol (B.1.47), pyrifenox (B.1.48), triforine (B.1.49), [3-(4-chloro-2-fluoro-phenyl)-5-(2,4-difluorophenyl)isoxazol-4-yl]-(3-pyridyl)methanol (B.1.50);
- Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
- Inhibitors of 3-keto reductase: fenhexamid (B.3.1);

### C) Nucleic acid synthesis inhibitors

- phenylamides or acyl amino acid fungicides: benalaxyl (C.1.1), benalaxyl-M (C.1.2), kiralaxyl (C.1.3), metalaxyl (C.1.4), metalaxyl-M (mefenoxam, C.1.5), ofurace (C.1.6), oxadixyl (C.1.7);
- others: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7);

### D) Inhibitors of cell division and cytoskeleton

- tubulin inhibitors, such as benzimidazoles, thiophanates: benomyl (D1.1), carbendazim (D1.2), fuberidazole (D1.3), thiabendazole (D1.4), thiophanate-methyl (D1.5); triazolopyrimidines: 5-chloro-7-(4-methylpiperidin-1-yl)-6-(2,4,6-trifluorophenyl)-[1,2,4]triazolo[1,5-a]pyrimidine (D1.6);
- other cell division inhibitors: diethofencarb (D2.1), ethaboxam (D2.2), pencycuron (D2.3), fluopicolide (D2.4), zoxamide (D2.5), metrafenone (D2.6), pyriofenone (D2.7);

### E) Inhibitors of amino acid and protein synthesis

- methionine synthesis inhibitors (anilino-pyrimidines): cyprodinil (E.1.1), mepanipyrim (E.1.2), pyrimethanil (E.1.3);
- protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6), polyoxine (E.2.7), validamycin A (E.2.8);

### F) Signal transduction inhibitors

- MAP / histidine kinase inhibitors: fluoroimid (F.1.1), iprodione (F.1.2), procymidone (F.1.3), vinclozolin (F.1.4), fenpiclonil (F.1.5), fludioxonil (F.1.6);
- G protein inhibitors: quinoxyfen (F.2.1);

### G) Lipid and membrane synthesis inhibitors

- Phospholipid biosynthesis inhibitors: edifenphos (G.1.1), iprobenfos (G.1.2), pyrazophos (G.1.3), isoprothiolane (G.1.4);
- lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
- phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7) and N-(1-(1-(4-cyano-phenyl)ethanesulfonyl)-but-2-yl) carbamic acid-(4-fluorophenyl) ester (G.3.8);
- compounds affecting cell membrane permeability and fatty acides: propamocarb (G.4.1);
- fatty acid amide hydrolase inhibitors: oxathiapiprolin (G.5.1), 2-{3-[2-(1-{[3,5-bis(difluoromethyl-1 H-pyrazol-1-yl]acetyl}piperidin-4-yl)-1 ,3-thiazol-4-yl]-4,5-dihydro-1,2-oxazol-5-yl}phenyl methanesulfonate (G.5.2), 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5-yl}-3-chlorophenyl methanesulfonate (G.5.3);

### H) Inhibitors with Multi Site Action

- inorganic active substances: Bordeaux mixture (H.1.1), copper acetate (H.1.2), copper hydroxide (H.1.3), copper oxychloride (H.1.4), basic copper sulfate (H.1.5), sulfur (H.1.6);
- thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
- organochlorine compounds (e. g. phthalimides, sulfamides, chloronitriles): anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11), N-(4-chloro-2-nitro-phenyl)-N-ethyl-4-methyl-benzenesulfonamide (H.3.12);
- guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1 H,5H-[1,4]dithi-ino[2,3-c:5,6-c']dipyrrole-1,3,5,7(2H,6H)-tetraone (H.4.10);

### I) Cell wall synthesis inhibitors

- inhibitors of glucan synthesis: validamycin (I.1.1), polyoxin B (I.1.2);
- melanin synthesis inhibitors: pyroquilon (I.2.1), tricyclazole (I.2.2), carpropamid (I.2.3), di-cyclomet (I.2.4), fenoxanil (1.2.5);

### J) Plant defence inducers

- acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9);

### K) Unknown mode of action

- bronopol (K.1.1), chinomethionat (K.1.2), cyflufenamid (K.1.3), cymoxanil (K.1.4), dazomet (K.1.5), debacarb (K.1.6), diclomezine (K.1.7), difenzoquat (K.1.8), difenzoquat-methylsulfate (K.1.9), diphenylamin (K.1.10), fenpyrazamine (K.1.11), flumetover (K.1.12), flusulfamide (K.1.13), flutianil (K.1.14), methasulfocarb (K.1.15), nitrapyrin (K.1.16), nitrothal-isopropyl (K.1.18), oxathiapiprolin (K.1.19), tolprocarb (K.1.20), oxin-copper (K.1.21), proquinazid (K.1.22), tebufloquin (K.1.23), tecloftalam (K.1.24), triazoxide (K.1.25), 2-butoxy-6-iodo-3-propylchromen-4-one (K.1.26), 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone (K.1.27), 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone (K.1.28), 2-[3,5-bis(difluoromethyl)-1 H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone (K.1.29), N-(cyclopropylmethoxyimino-(6-difluoromethoxy-2,3-difluoro-phenyl)-methyl)-2-phenyl acetamide (K.1.30), N'-(4-(4-chloro-3-trifluoromethyl-phenoxy)-2,5-dimethyl-phenyl)-N-ethyl-N-methyl formamidine (K.1.31), N'-(4-(4-fluoro-3-trifluoromethyl-phenoxy)-2,5-dimethyl-phenyl)-N-ethyl-N-methyl formamidine (K.1.32), N'-(2-methyl-5-trifluoromethyl-4-(3-trimethylsilanyl-propoxy)-phenyl)-N-ethyl-N-methyl formamidine (K.1.33), N'-(5-difluoromethyl-2-methyl-4-(3-trimethylsilanyl-propoxy)-phenyl)-N-ethyl-N-methyl formamidine (K.1.34), methoxy-acetic acid 6-tert-butyl-8-fluoro-2,3-dimethyl-quinolin-4-yl ester (K.1.35), 3-[5-(4-methylphenyl)-2,3-dimethyl-isoxazolidin-3-yl]-pyridine (K.1.36), 3-[5-(4-chlorophenyl)-2,3-dimethyl-isoxazolidin-3-yl]-pyridine (pyrisoxazole) (K.1.37), N-(6-methoxy-pyridin-3-yl) cyclopropanecarboxylic acid amide (K.1.38), 5-chloro-1-(4,6-dimethoxy-pyrimidin-2-yl)-2-methyl-1 H-benzoimidazole (K.1.39), 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), 2-[2-[(7,8-difluoro-2-methyl-3-quinolyl)oxy]-6-fluoro-phenyl]propan-2-ol (K.1.43), 2-[2-fluoro-6-[(8-fluoro-2-methyl-3-quinolyl)oxy]phen-yl]propan-2-ol (K.1.44), 3-(5-fluoro-3,3,4,4-tetramethyl-3,4-dihydroisoquinolin-1-yl)quinoline (K.1.45), 3-(4,4-difluoro-3,3-dimethyl-3,4-dihydroisoquinolin-1-yl)quinoline (K.1.46), 3-(4,4,5-trifluoro-3,3-dimethyl-3,4-dihydroisoquinolin-1-yl)quinoline (K.1.47), 9-fluoro-2,2-dimethyl-5-(3-quinolyl)-3H-1,4-benzoxazepine (K.1.48).

The fungicides described by common names, their preparation and their activity e.g. against harmful fungi is known (cf.: http://www.alanwood.net/pesticides/); these substances are commercially available.

The fungicides described by IUPAC nomenclature, their preparation and their pesticidal activity is also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657, WO2012/168188, WO 2007/006670, WO 2011/77514; WO 13/047749, WO 10/069882, WO 13/047441, WO 03/16303, WO 09/90181, WO 13/007767, WO 13/010862, WO 13/127704, WO 13/024009, WO 13/024010 and WO 13/047441, WO 13/162072, WO 13/092224, WO 11/135833).

Suitable mixing partners for the compounds of the invention also include biopesticides.

Biopesticides have been defined as a form of pesticides based on micro-organisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (such as growth or developmental regulation, attractents, repellents or defence activators (e.g. induced resistance) and are relatively non-toxic to mammals.

Biopesticides for use against crop diseases have already established themselves on a variety of crops. For example, biopesticides already play an important role in controlling downy mildew diseases. Their benefits include: a 0-Day Pre-Harvest Interval, the ability to use under moderate to severe disease pressure, and the ability to use in mixture or in a rotational program with other registered pesticides.

A major growth area for biopesticides is in the area of seed treatments and soil amendments. Biopesticidal seed treatments are e.g. used to control soil borne fungal pathogens that cause seed rots, damping-off, root rot and seedling blights. They can also be used to control internal seed borne fungal pathogens as well as fungal pathogens that are on the surface of the seed. Many biopesticidal products also show capacities to stimulate plant host defenses and other physiological processes that can make treated crops more resistant to a variety of biotic and abiotic stresses or can regulate plant growth. Many biopesticidal products also show capacities to stimulate plant health, plant growth and/or yield enhancing activity.

The following list of biopesticides, in conjunction with which the compounds of the invention can be used, is intended to illustrate the possible combinations but does not limit them:

### L) Biopesticides

L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. megaterium, B. mojavensis, B. mycoides, B. pumilus, B. simplex, B. solisalsi, B. subtilis, B. subtilis* var. *amyloliquefaciens, Candida oleophila, C. saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea* f*. catenulate* (also named *Gliocladium catenulatum*)*, Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
L2) Biochemical pesticides with fungicidal, bactericidal, viricidal and/or plant defense activator activity: harpin protein, *Reynoutria sachalinensis* extract;
L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae, Metarhizium anisopliae* var. *anisopliae, M. anisopliae* var. *acridum, Nomuraea rileyi, Paecilomyces fumosoroseus, P. lilacinus, Paenibacillus popilliae, Pasteuria* spp., *P. nishizawae, P. penetrans, P. ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microflavus;*
L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadecadien-1-ol acetate, (E,Z)-3,13-octadecadien-1-ol, R-1-octen-3-ol, pentatermanone, (E,Z,Z)-3,8,11-tetradecatrienyl acetate, (Z,E)-9,12-tetradecadien-1-yl acetate, Z-7-tetradecen-2-one, Z-9-tetradecen-1-yl acetate, Z-11-tetradecenal, Z-11-tetradecen-1-ol, extract of *Chenopodium ambrosiodes,* Neem oil, Quillay extract;
L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B. elkanii, B. japonicum, B. liaoningense, B. lupini, Delftia acidovorans, Glomus intraradices, Mesorhizobium* spp., *Rhizobium leguminosarum* bv. *phaseoli, R. I*. bv. *trifolii, R. I*. bv. *viciae, R. tropici, Sinorhizobium meliloti.*

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices such as ATCC or DSM refer to the acronym of the respective culture collection, for details see e. g. here: http://www. wfcc.info/ccinfo/collection/by_acronym/), are referred to in literature, registered and/or are commercially available: mixtures of *Aureobasidium pullulans* DSM 14940 and DSM 14941 isolated in 1989 in Konstanz, Germany (e. g. blastospores in BlossomProtect® from bio-ferm GmbH, Austria), *Azospirillum brasilense* Sp245 originally isolated in wheat reagion of South Brazil (Passo Fundo) at least prior to 1980 (BR 11005; e. g. GELFIX® Gramíneas from BASF Agricultural Specialties Ltd., Brazil), *A. brasilense* strains Ab-V5 and Ab-V6 (e. g. in AzoMax from Novozymes BioAg Produtos papra Agricultura Ltda., Quattro Barras, Brazil or Simbiose-Maíz®from Simbiose-Agro, Brazil; Plant Soil 331, 413-425, 2010), *Bacillus amyloliquefaciens* strain AP-188 (NRRL B-50615 and B-50331; US 8,445,255); *B. amyloliquefaciens* spp. *plantarum* D747 isolated from air in Kikugawa-shi, Japan (US 20130236522; FERM BP-8234; e. g. Double Nickel™ 55 WDG from Certis LLC, USA), *B. amyloliquefaciens* spp*. plantarum* FZB24 isolated from soil in Brandenburg, Germany (also called SB3615; DSM 96-2; J. Plant Dis. Prot. 105, 181-197, 1998; e. g. Taegro®from Novozyme Biologicals, Inc., USA), *B. amyloliquefaciens* ssp. *plantarum* FZB42 isolated from soil in Brandenburg, Germany (DSM 23117; J. Plant Dis. Prot. 105, 181-197, 1998; e. g. RhizoVital® 42 from AbiTEP GmbH, Germany), *B. amyloliquefaciens ssp. plantarum* MBI600 isolated from faba bean in Sutton Bonington, Nottinghamshire, U.K. at least before 1988 (also called 1430; NRRL B-50595;
US 2012/0149571; e. g. Integral® from BASF Corp., USA), *B. amyloliquefaciens* spp. *plantarum* QST-713 isolated from peach orchard in 1995 in California, U.S.A. (NRRL B-21661; e. g. Serenade® MAX from Bayer Crop Science LP, USA), *B. amyloliquefaciens* spp. *plantarum* TJ1000 isolated in 1992 in South Dakoda, U.S.A. (also called 1BE; ATCC BAA-390; CA 2471555; e.g. QuickRoots™ from TJ Technologies, Watertown, SD, USA), *B. firmus* CNCM I-1582, a variant of parental strain EIP-N1 (CNCM I-1556) isolated from soil of central plain area of Israel (WO 2009/126473, US 6,406,690; e. g. Votivo® from Bayer CropScience LP, USA), *B. pumilus* GHA 180 isolated from apple tree rhizosphere in Mexico (IDAC 260707-01; e. g. PRO-MIX® BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754; WO 2014/029697; e. g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e. g. Sonata® or Ballad® Plus from Bayer Crop Science LP, USA), *B. simplex* ABU 288 (NRRL B-50304; US 8,445,255), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. thuringiensis* ssp. *aizawai* ABTS-1857 isolated from soil taken from a lawn in Ephraim, Wisconsin, U.S.A., in 1987 (also called ABG-6346; ATCC SD-1372; e. g. XenTari® from BioFa AG, Münsingen, Germany), *B. t.* ssp. *kurstaki* ABTS-351 identical to HD-1 isolated in 1967 from diseased Pink Bollworm black larvae in Brownsville, Texas, U.S.A. (ATCC SD-1275; e. g. Dipel® DF from Valent BioSciences, IL, USA), *B. t.* ssp. *kurstaki* SB4 isolated from *E. saccharina* larval cadavers (NRRL B-50753; e. g. Beta Pro® from BASF Agricultural Specialities (Pty) Ltd., South Africa), *B. t.* ssp. *tenebrionis* NB-176-1, a mutant of strain NB-125, a wild type strain isolated in 1982 from a dead pupa of the beetle Tenebrio molitor (DSM 5480; EP 585 215 B1; e. g. Novodor® from Valent BioSciences, Switzerland), *Beauveria bassiana* GHA (ATCC 74250; e. g. BotaniGard® 22WGP from Laverlam Int. Corp., USA), *B. bassiana* JW-1 (ATCC 74040; e. g. Naturalis® from CBC (Europe) S.r.l., Italy), *B. bassiana* PPRI 5339 isolated from the larva of the tortoise beetle *Conchyloctenia punctata* (NRRL 50757; e. g. BroadBand® from BASF Agricultural Specialities (Pty) Ltd., South Africa), *Bradyrhizobium elkanii strains* SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e. g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e. g. in Rhizoflo®, Histick®, Hicoat® Super from BASF Agricultural Specialties Ltd., Canada), *B. japonicum* E-109 variant of strain USDA 138 (INTA E109, SEMIA 5085; Eur. J. Soil Biol. 45, 28-35, 2009; Biol. Fertil. Soils 47, 81-89, 2011); *B.japonicum* strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e. g. GELFIX 5 or ADHERE 60 from BASF Agricultural Specialties Ltd., Brazil), *B.japonicum* SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e. g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape (WO 1996/021358; DSM 9660; e. g. Contans® WG, Intercept® WG from Bayer CropScience AG, Germany), harpin (alpha-beta) protein (Science 257, 85-88, 1992; e. g. Messenger™ or HARP-N-Tek from Plant Health Care plc, U.K.), *Helicoverpa armigera* nucleopolyhedrovirus (HearNPV) (J. Invertebrate Pathol. 107, 112-126, 2011; e. g. Helicovex® from Adermatt Biocontrol, Switzerland; Diplomata® from Koppert, Brazil; Vivus® Max from AgBiTech Pty Ltd., Queensland, Australia), *Helicoverpa zea* single capsid nucleopolyhedrovirus (HzSNPV) (e. g. Gemstar® from Certis LLC, USA), *Helicoverpa zea* nucleopolyhedrovirus ABA-NPV-U (e. g. Heligen® from AgBiTech Pty Ltd., Queensland, Australia), *Heterorhabditis bacteriophora* (e. g. Nemasys® G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e. g. PFR-97™ or PreFeRal® from Certis LLC, USA), *Metarhizium anisopliae* var. *anisopliae* F52 also called 275 or V275 isolated from codling moth in Austria (DSM 3884, ATCC 90448; e.g. Met52® Novozymes Biologicals BioAg Group, Canada), *Metschnikowia fructicola* 277 isolated from grapes in the central part of Israel (US 6,994,849; NRRL Y-30752; e. g. formerly Shemer® from Agrogreen, Israel), *Paecilomyces ilacinus* 251 isolated from infected nematode eggs in the Philippines (AGAL 89/030550; WO1991/02051; Crop Protection 27, 352-361, 2008; e.g. Bio-Act®from Bayer CropScience AG, Germany and MeloCon® from Certis, USA), *Paenibacillus alvei* NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), *Pasteuria nishizawae* Pn1 isolated from a soybean field in the mid-2000s in Illinois, U.S.A. (ATCC SD-5833; Federal Register 76(22), 5808, February 2, 2011; e.g. Clariva™ PN from Syngenta Crop Protection, LLC, USA), *Penicillium bilaiae*(also called *P. bilaii*) strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e. g. Jump Start®, Provide® from Novozymes Biologicals BioAg Group, Canada), *Reynoutria sachalinensis* extract (EP 0307510 B1; e.g. Regalia® SC from Marrone Biolnnovations, Davis, CA, USA or Milsana® from BioFa AG, Germany), *Steinernema carpocapsae* (e. g. Millenium® from BASF Agricultural Specialities Limited, UK), *S. feltiae* (*e.* g. Nemashield® from BioWorks, Inc., USA; Nemasys® from BASF Agricultural Specialities Limited, UK), *Streptomyces microflavus* NRRL B-50550 (WO 2014/124369; Bayer CropScience, Germany), *Trichoderma asperelloides* JM41R isolated in South Africa (NRRL 50759; also referred to as *T. fertile;* e. g. Trichoplus® from BASF Agricultural Specialities (Pty) Ltd., South Africa), *T. harzianum* T-22 also called KRL-AG2 (ATCC 20847; BioControl 57, 687-696, 2012; e. g. Plantshield® from BioWorks Inc., USA or SabrEx™ from Advanced Biological Marketing Inc., Van Wert, OH, USA).

According to the invention, the solid material (dry matter) of the biopesticides (with the exception of oils such as Neem oil) are considered as active components (e.g. to be obtained after drying or evaporation of the extraction or suspension medium in case of liquid formulations of the microbial pesticides).

In accordance with the invention, the weight ratios and percentages used herein for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calclulate the total weight of the respective active component with the following equation that 1 x 10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as *Steinernema feltiae.*

When mixtures comprising microbial pesticides are employed in crop protection, the application rates preferably range from about 1 x 106 to 5 x 1015 (or more) CFU/ha, preferably from about 1 x 108 to about 1 x 1013 CFU/ha, and even more preferably from about 1 x 109 to about 1 x 1012 CFU/ha. In the case of (entomopathogenic) nematodes as microbial pesticides (e. g. Steinernema feltiae), the application rates preferably range inform about 1 x 105 to 1 x 1012 (or more), more preferably from 1 x 108 to 1 x 1011, even more preferably from 5 x 108 to 1 x 1010 individuals (e. g. in the form of eggs, juvenile or any other live stages, preferably in an infetive juvenile stage) per ha.

When mixtures comprising microbial pesticides are employed in seed treatment, the application rates with respect to plant propagation material preferably range from about 1 x 106 to 1 x 1012 (or more) CFU/seed. Preferably, the concentration is about 1 x 106 to about 1 x 109 CFU/seed. In the case of the microbial pesticides II, the application rates with respect to plant propagation material also preferably range from about 1 x 107 to 1 x 1014 (or more) CFU per 100 kg of seed, preferably from 1 x 109 to about 1 x 1012 CFU per 100 kg of seed.

The invention also relates to agrochemical compositions comprising an auxiliary and at least one compound of the invention or a mixture thereof.

An agrochemical composition comprises a pesticidally effective amount of a compound of the invention or a mixture thereof. The term "pesticidally effective amount" is defined below.

The compounds of the invention or the mixtures thereof can be converted into customary types of agro-chemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclo-hexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharide powders, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl-sulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl¬naphthalenes, sulfosuccinates or sulfosuccinamates. Examples of sulfates are sulfates of fatty acids and oils, of ethoxylated alkylphenols, of alcohols, of ethox-ylated alcohols, or of fatty acid esters. Examples of phosphates are phosphate esters. Exam-ples of carboxylates are alkyl carboxylates, and carboxylated alcohol or alkylphenol eth-oxylates. Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.

### ii) Dispersible concentrates (DC)

5-25 wt% of a compound I according to the invention and 1-10 wt% dispersant (e. g. polyvinylpyrrolidone) are dissolved in up to 100 wt% organic solvent (e.g. cyclohexanone). Dilution with water gives a dispersion.

### iii) Emulsifiable concentrates (EC)

15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.

### iv) Emulsions (EW, EO, ES)

5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.

### v) Suspensions (SC, OD, FS)

In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active substance. For FS type composition up to 40 wt% binder (e.g. polyvinylalcohol) is added.

### vi) Water-dispersible granules and water-soluble granules (WG, SG)

50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

20-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.

### viii) Gel (GW, GF)

In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.

### ix) Microemulsion (ME)

5-20 wt% of a compound I according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water up to 100 %. This mixture is stirred for 1 h to produce spontaneously a thermodynamically stable microemulsion.

### x) Microcapsules (CS)

An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radi-cal initiator results in the formation of poly(meth)acrylate microcapsules. Alternatively, an oil phase comprising 5-50 wt% of a compound I according to the invention, 0-40 wt% water insolu-ble organic solvent (e.g. aromatic hydrocarbon), and an isocyanate monomer (e.g. diphenylme-thene-4,4'-diisocyanatae) are dispersed into an aqueous solution of a protective colloid (e.g. polyvinyl alcohol). The addition of a polyamine (e.g. hexamethylenediamine) results in the for-mation of a polyurea microcapsule. The monomers amount to 1-10 wt%. The wt% relate to the total CS composition.

### xi) Dustable powders (DP, DS)

1-10 wt% of a compound I according to the invention are ground finely and mixed intimately with up to 100 wt% solid carrier, e.g. finely divided kaolin.

### xii) Granules (GR, FG)

0.5-30 wt% of a compound I according to the invention is ground finely and associated with up to 100 wt% solid carrier (e.g. silicate). Granulation is achieved by extrusion, spray-drying or the fluidized bed.

### xiii) Ultra-low volume liquids (UL)

1-50 wt% of a compound I according to the invention are dissolved in up to 100 wt% organic solvent, e.g. aromatic hydrocarbon.

The compositions types i) to xi) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and most preferably between 0.5 and 75%, by weight of active sub-stance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions com¬prising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

In a further embodiment, either individual components of the composition according to the invention or partially premixed components, e. g. components comprising compounds of the invention and/or mixing partners as defined above, may be mixed by the user in a spray tank and further auxiliaries and additives may be added, if appropriate.

In a further embodiment, either individual components of the composition according to the invention or partially premixed components, e. g. components comprising compounds of the invention and/or mixing partners as defined above, can be applied jointly (e.g. after tank mix) or consecutively.

The compounds of the invention are suitable for use in protecting crops, plants, plant propagation materials, such as seeds, or soil or water, in which the plants are growing, from attack or infestation by animal pests. Therefore, the invention also relates to a plant protection method, which comprises contacting crops, plants, plant propagation materials, such as seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, such as seeds, or soil, or the area, material or environment in which the animal pests are growing or may grow, with a pesticidally effective amount of a compound of the invention.

The compounds of the invention are effective through both contact and ingestion. Furthermore, the compounds of the invention can be applied to any and all developmental stages, such as egg, larva, pupa, and adult.

The compounds of the invention can be applied as such or in form of compositions comprising them as defined above. Furthermore, the compounds of the invention can be applied together with a mixing partner as defined above or in form of compositions comprising said mixtures as defined above. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, such as seeds, soil, or the area, material or environment by the pests.

Suitable application methods include inter alia soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known to a skilled person and publicly available from databases of pheromones and semiochemicals, such as http://www.pherobase.com .

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet or fodder beet; fruits, such as pomes, stone fruits or soft fruits, e.g. apples, pears, plums, peaches, nectarines, almonds, cherries, papayas, strawberries, raspberries, blackberries or gooseberries; leguminous plants, such as beans, lentils, peas, alfalfa or soybeans; oil plants, such as rapeseed (oilseed rape), turnip rape, mustard, olives, sunflowers, coconut, cocoa beans, castor oil plants, oil palms, ground nuts or soybeans; cucurbits, such as squashes, pumpkins, cucumber or melons; fiber plants, such as cotton, flax, hemp or jute; citrus fruit, such as oranges, lemons, grapefruits or mandarins; vegetables, such as eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, such as avocados, cinnamon or camphor; energy and raw material plants, such as corn, soybean, rapeseed, sugar cane or oil palm; tobacco; nuts, e.g. walnuts; pistachios; coffee; tea; bananas; vines (table grapes and grape juice grape vines); hop; sweet leaf (also called Stevia); natural rubber plants or ornamental and forestry plants, such as flowers (e.g. carnation, petunias, geranium/pelargoniums, pansies and impatiens), shrubs, broad-leaved trees (e.g. poplar) or evergreens, e.g. conifers; eucalyptus; turf; lawn; grass such as grass for animal feed or ornamental uses. Preferred plants include potatoes sugar beets, tobacco, wheat, rye, barley, oats, rice, corn, cotton, soybeans, rapeseed, legumes, sunflowers, coffee or sugar cane; fruits; vines; ornamentals; or vegetables, such as cucumbers, tomatoes, beans or squashes.

The term "plant" is to be understood as including wild type plants and plants, which have been modified by either conventional breeding, or mutagenesis or genetic engineering, or by a combination thereof.

Plants, which have been modified by mutagenesis or genetic engineering, and are of particular commercial importance, include alfalfa, rapeseed (e.g. oilseed rape), bean, carnation, chicory, cotton, eggplant, eucalyptus, flax, lentil, maize, melon, papaya, petunia, plum, poplar, potato, rice, soybean, squash, sugar beet, sugarcane, sunflower, sweet pepper, tobacco, tomato, and cereals (e.g. wheat), in particular maize, soybean, cotton, wheat, and rice. In plants, which have been modified by mutagenesis or genetic engineering, one or more genes have been mutagenized or integrated into the genetic material of the plant. The one or more mutagenized or integrated genes are preferably selected from pat, epsps, cry1Ab, bar, cry1Fa2, cry1Ac, cry34Ab1, cry35AB1, cry3A, cryF, cry1F, mcry3a, cry2Ab2, cry3Bb1, cry1A.105, dfr, barnase, vip3Aa20, barstar, als, bxn, bp40, asn1, and ppo5. The mutagenesis or integration of the one or more genes is performed in order to improve certain properties of the plant. Such properties, also known as traits, include abiotic stress tolerance, altered growth/yield, disease resistance, herbicide tolerance, insect resistance, modified product quality, and pollination control. Of these properties, herbicide tolerance, e.g. imidazolinone tolerance, glyphosate tolerance, or glufosinate tolerance, is of particular importance. Several plants have been rendered tolerant to herbicides by mutagenesis, e.g. Clearfield® oilseed rape being tolerant to imidazolinones, e.g. imazamox. Alternatively, genetic engineering methods have been used to render plants, such as soybean, cotton, corn, beets and oil seed rape, tolerant to herbicides, such as glyphosate and glufosinate, some of which are commercially available under the trade names RoundupReady® (glyphosate) and LibertyLink® (glufosinate). Furthermore, insect resistance is of importance, in particular lepidopteran insect resistance and coleopteran insect resistance. Insect resistance is typically achieved by modifying plants by integrating cry and/or vip genes, which were isolated from *Bacillus thuringiensis* (Bt), and code for the respective Bt toxins. Genetically modified plants with insect resistance are commercially available under trade names including WideStrike®, Bollgard®, Agrisure®, Herculex®, YieldGard®, Genuity®, and Intacta®. Plants may be modified by mutagenesis or genetic engineering either in terms of one property (singular traits) or in terms of a combination of properties (stacked traits). Stacked traits, e.g. the combination of herbicide tolerance and insect resistance, are of increasing importance. In general, all relevant modified plants in connection with singular or stacked traits as well as detailed information as to the mutagenized or integrated genes and the respective events are available from websites of the organizations "International Service for the Acquisition of Agri-biotech Applications (ISAAA)" (http://www.isaaa.org/gmapprovaldatabase) and "Center for Environmental Risk Assessment (CERA)" (http://cera-gmc.org/GMCropDatabase).

It has surprisingly been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant such as seeds and vegetative plant material such as cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises all suitable seed treatment techniques known in the art, such as seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

In addition, the active compound may also be used for the treatment of seeds from plants, which have been modified by mutagenisis or genetic engineering, and which e.g. tolerate the action of herbicides or fungicides or insecticides. Such modified plants have been described in detail above.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40 % by weight.

In a preferred embodiment a FS formulation is used for seed treatment. Typically, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20 % by weight (1 to 200 g/l) of at least one surfactant, e.g. 0.05 to 5 % by weight of a wetter and from 0.5 to 15 % by weight of a dispersing agent, up to 20 % by weight, e.g. from 5 to 20 % of an anti-freeze agent, from 0 to 15 % by weight, e.g. 1 to 15 % by weight of a pigment and/or a dye, from 0 to 40 % by weight, e.g. 1 to 40 % by weight of a binder (sticker /adhesion agent), optionally up to 5 % by weight, e.g. from 0.1 to 5 % by weight of a thickener, optionally from 0.1 to 2 % of an anti-foam agent, and optionally a preservative such as a biocide, antioxidant or the like, e.g. in an amount from 0.01 to 1 % by weight and a filler/vehicle up to 100 % by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops such as lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

The terms "plant" and "plant propagation material" are defined above.

"Plant health" is defined as a condition of the plant and/or its products which is determined by several aspects alone or in combination with each other such as yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (for example, harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, such as ants, termites, wasps, flies, ticks, mosquitos, crickets, or cockroaches.

The bait can be a liquid, a solid or a semisolid preparation (e.g. a gel). The bait employed in the composition is a product, which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature (e.g. http://www.pherobase.com), and are known to those skilled in the art.

For use in bait compositions, the typical content of active ingredient is from 0.001 weight % to 15 weight %, desirably from 0.001 weight % to 5% weight % of active compound.

Formulations of the compounds of the invention as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents, furthermore auxiliaries such as emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its respective compositions also comprise treating surfaces of huts and houses, air spraying and impregnation of curtains, tents, clothing items, bed nets, tsetse-fly trap or the like. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials such as trees, board fences, sleepers, frames, artistic artifacts, etc. and buildings, but also construction materials, furniture, leathers, fibers, vinyl articles, electric wires and cables etc. from ants and/or termites, and for controlling ants and termites from doing harm to crops or human being (e.g. when the pests invade into houses and public facilities).

Customary application rates in the protection of materials are, for example, from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and/or insecticide.

The compounds of the the invention are especially suitable for efficiently combating animal pests such as arthropods, gastropods and nematodes including but not limited to:
insects from the order of Lepidoptera, e.g. *Achroia grisella, Acleris* spp. such as *A. fimbriana, A. gloverana, A. variana;Acrolepiopsis assectella, Acronicta major, Adoxophyes* spp. such as *A. cyrtosema, A. orana; Aedia leucomelas, Agrotis* spp. such as *A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia)* spp. such as *A. gemmatalis;Apamea* spp., *Aproaerema modicella, Archips* spp. such as *A. argyrospila, A. fuscocupreanus, A. rosana, A. xyloseanus;Argyresthia conjugella, Argyroploce* spp., *Argyrotaeniaspp.* such as *A. velutinana; Athetis mindara, Austroasca viridigrisea, Autographa gamma, Autographa nigrisigna, Barathra brassicae, Bedellia* spp., *Bonagota salubricola, Borbo cinnara, Bucculatrix thurberiella, Bupalus piniarius, Busseola* spp., *Cacoecia* spp. such as *C. murinana, C. podana; Cactoblastis cactorum, Cadra cautella, Calingo braziliensis, Caloptilis theivora, Capua reticulana, Carposina* spp. such as *C. niponensis, C. sasakii; Cephus* spp., *Chaetocnema aridula, Cheimatobia brumata, Chilo* spp. such as *C. Indicus, C. suppressalis, C. partellus; Choreutis pariana, Choristoneura* spp. such as *C. conflictana, C. fumiferana, C. longicellana, C. murinana, C. occidentalis, C. rosaceana; Chrysodeixis (=Pseudoplusia)* spp. *such as C. eriosoma, C. inciudens; Cirphis unipuncta, C*/*ysia ambiguella, Cnaphalocerus* spp., *Cnaphalocrocis medinalis, Cnephasia* spp., *Cochylis hospes, Coleophora* spp., *Colias eurytheme, Conopomorpha* spp., *Conotrachelus* spp., *Copitarsia* spp., *Corcyra cephalonica, Crambus caliginosellus, Crambus teterrellus, Crocidosema (=Epinotia) aporema, Cydalima (=Diaphania) perspectalis, Cydia (=Carpocapsa)* spp. such as *C. pomonella, C. latiferreana,_{'} Dalaca noctuides, Datana integerrima, Dasychirapinicola, Dendrolimus* spp. such as *D. pini, D. spectabilis, D. sibiricus; Desmia funeralis, Diaphania* spp. such as *D. nitidalis, D. hyalinata; Diatraea grandiosella, Diatraea saccharalis, Diphthera festiva, Earias* spp. such as *E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia* spp. such as *E*. *cautella, E. elutella, E. kuehniella; Epinotia aporema, Epiphyas postvittana, Erannis tiliaria, Erionota thrax, Etiella* spp., *Eulia* spp., *Eupoecilia ambiguella, Euproctis chrysorrhoea, Euxoa* spp., *Evetria bouliana, Faronta albilinea, Feltia* spp. such as *F. subterranean; Galleria mellonella, Gracillaria* spp., *Grapholita* spp. such as *G. funebrana, G. molesta, G. inopinata; Halysidota* spp., *Harrisina americana, Hedylepta* spp., *Helicoverpa* spp. such as *H. armigera (=Heliothis armigera), H. zea (=Heliothis zea); Heliothis* spp. such as *H. assulta, H. subflexa, H. virescens; Hellula* spp. such as *H*. *undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeutapadella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera* spp. such as *L. coffeella, L. scitella; Leuminivora lycinivorelia, Lithocolletis blancardella, Lithophane antennata, Llattia octo (=Amyna axis), Lobesia botrana, Lophocampa*spp., *Loxagrotis albicosta, Loxostege* spp. such as *L. sticticalis, L. cereralis; Lymantria* spp. such as *L. dispar, L. monacha; Lyonetia clerkella, Lyonetia prunifoliella, Malacosoma* spp. such as *M. americanum, M. californicum, M. constricttum, M. neustria; Mamestra* spp. such as *M. brassicae, M. configurata; Mamstra brassicae, Manduca* spp. such as *M. quinquemaculata, M. sexta; Marasmia spp, Marmara* spp., *Maruca testulalis, Megalopyge lanata, Melanchra picta, Melanitis leda, Mods* spp. such as *M. lapites, M. repanda; Mods latipes, Monochroa fragariae, Mythimna separata, Nemapogon e*/*oacella, Neoleucinodes elegantalis, Nepytia* spp., *Nymphula* spp., *Oiketicus* spp., *Omiodes indicata, Omphisa anastomosalis, Operophtera brumata, Orgyia pseudotsugata, Oria* spp., *Orthaga thyrisalis, Ostrinia* spp. such as *O. nubilalis; Oulema oryzae, Paleacrita vernata, Panolis flammea, Parnara* spp., *Papaipema nebris, Papilio cresphontes, Paramyelois transitella, Paranthrene regalis, Paysandisia archon, Pectinophora* spp. such as *P. gossypiella; Peridroma saucia, Perileucoptera* spp., such as *P. coffeella; Phalera bucephala, Phryganidia californica, Phthorimaea* spp. such as *P. operculella; Phyllocnistis citrella, Phyllonorycter spp.* such as *P. blancardella, P. crataegella, P. issikii, P. ringoniella; Pieris* spp. such as *P. brassicae, P. rapae, P. napi; Pilocrocis tripunctata, Plathypena scabra, Platynota* spp. such as *P. flavedana, P. idaeusalis, P. stultana; Platyptilia carduidactyla, Plebejus argus, Plodia interpunctella, Plusia spp, Plutella maculipennis, Plutella xylostella, Pontia protodica, Prays* spp., *Prodenia* spp., *Proxenus lepigone, Pseudaletia* spp. such as *P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius* spp., *Schreckensteinia festaliella, Scirpophaga* spp. such as *S. incertulas, S. innotata; Scotia segetum, Sesamia* spp. such as *S. inferens, Seudyra subflava, Sitotroga cerealella, Sparganothis pilleriana, Spilonota lechriaspis, S. ocellana, Spodoptera (=Lamphygma)* spp. such as *S. cosmoides, S. eridania, S. exigua, S. frugiperda, S. latisfascia, S. littoralis, S. litura, S. omithogalli; Stigmella* spp., *Stomopteryx subsecivella, Strymon bazochii, Sylepta derogata, Synanthedon* spp. such as S. exitiosa, *Tecia solanivora, Telehin licus, Thaumatopoea pityocampa, Thaumatotibia (=Cryptophlebia) leucotreta, Thaumetopoea pityocampa, Thecla* spp., *Theresimima ampelophaga, Thyrinteina* spp, *Tildenia inconspicuella, Tinea* spp. such as *T. cloacella, T. pellionella; Tineola bisselliella, Tortrix spp.* such as *T. viridana; Trichophaga tapetzella, Trichoplusia* spp. such as *T. ni; Tuta (=Scrobipalpula) absoluta, Udea* spp. such as *U. rubigalis, U. rubigalis; Virachola* spp., *Yponomeuta padella, and Zeiraphera canadensis;* insects from the order of Coleoptera, e.g. *Acalymma vittatum, Acanthoscehdes obtectus, Adoretus* spp., *Agelastica alni, Agrilus* spp. such as *A. anxius, A. planipennis, A. sinuatus;Agriotes spp.* such as *A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora* spp. such as *A. glabripennis; Anthonomus* spp. such as *A. eugenii, A. grandis, A. pomorum;Anthrenus* spp., *Aphthona euphoridae, Apion* spp., *Apogonia* spp., *Athous haemorrhoidalis, Atomaria* spp. such as *A. linearis;Attagenus* spp., *Aulacophora femoralis, Blastophagus piniperda, Blitophaga undata, Bruchidius obtectus, Bruchus* spp. such as *B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus* spp. such as *C. assimilis, C. napi; Chaetocnema tibialis, Cleonus mendicus, Conoderus* spp. such as *C*. *vespertinus; Conotrachelus nenuphar, Cosmopolites* spp., *Costelytra zealandica, Crioceris asparagi, Cryptolestes ferrugineus, Cryptorhynchus lapathi, Ctenicera* spp. such as *C. destructor; Curculio* spp., *Cylindrocopturus* spp., *Cyclocephala* spp., *Dactylispa balyi, Dectes texanus, Dermestes* spp., *Diabrotica* spp. such as *D. undecimpunctata, D. speciosa, D. longicornis, D. semipunctata, D. virgifera; Diaprepes abbreviates, Dichocrocis* spp., *Dicladispa armigera, Diloboderus abderus, Diocalandra frumenti (Diocalandra stigmaticollis), Enaphalodes rufulus, Epilachna* spp. such as *E. varivestis, E. vigintioctomaculata; Epitrix spp.* such as *E. hirtipennis, E. simi*/*aris; Eutheola humilis, Eutinobothrus brasiliensis, Faustinus cubae, Gibbium psylloides, Gnathocerus cornutus, Hellula undalis, Heteronychus arator, Hylamorpha elegans Hylobius abietis, Hylotrupes bajulus, Hypera* spp. such as *H. brunneipennis, H. postica; Hypomeces squamosus, Hypothenemus* spp., *Ips typographus, Lachnosterna consanguinea, Lasioderma serricorne, Latheticus oryzae, Lathridius* spp., *Lema spp.* such as *L bilineata, L. melanopus; Leptinotarsa* spp. such as *L. decemlineata; Leptispa pygmaea, Limonius californicus, Lissorhoptrus oryzophilus, Lixus* spp., *Luperodes* spp., *Lyctus* spp. such as *L. bruneus; Liogenys fuscus, Macrodactylus* spp. such as *M. subspinosus; Maladera matrida, Megaplatypus mutates, Megasce*/*is* spp., *Melanotus communis, Meligethes* spp. such as *M. aeneus; Melolontha* spp. such as *M. hippocastani, M. melolontha; Metamasius hemipterus, Microtheca* spp., *Migdolus* spp. such as *M. fryanus, Monochamus* spp. such as *M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon* spp. such as *P. brassicae, P. cochleariae; Phoracantha recurva, Phyllobius pyri, Phyllopertha horticola, Phyllophaga* spp. such as *P. helleri; Phyllotreta* spp. such as *P. chrysocephala, P. nemorum, P. striolata, P. vittula; Phyllopertha horticola, Popillia japonica, Premnotrypes* spp., *Psacothea hilaris, Psylliodes chrysocephala, Prostephanus truncates, Psylliodes* spp., *Ptinus* spp., *Pulga saltona, Rhizopertha dominica, Rhynchophorus* spp. such as *R. billineatus, R. ferrugineus, R. palmarum, R. phoenicis, R. vulneratus; Saperda candida, Scolytus schevyrewi, Scyphophorus acupunctatus, Sitona lineatus, Sitophilus* spp. such as *S. granaria, S. oryzae, S. zeamais; Sphenophorus* spp. such as *S. levis; Stegobium paniceum, Sternechus* spp. such as *S. subsignatus; Strophomorphus ctenotus, Symphyletes* spp., *Tanymecus* spp., *Tenebrio molitor, Tenebrioides mauretanicus, Tribolium* spp. such as *T. castaneum; Trogoderma* spp., *Tychius* spp., *Xylotrechus* spp. such as *X. pyrrhoderus; and, Zabrus* spp. *such as Z. tenebrioides;*
insects from the order of Diptera e.g. *Aedes* spp. such as *A. aegypti, A. albopictus, A. vexans; Anastrepha ludens, Anopheles* spp. such as *A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia* spp. such as *C. bezziana, C. hominivorax, C. macellaria; Chrysops atlanticus, Chrysops discalis, Chrysops silacea, Cochliomyia* spp. such as *C. hominivorax; Contarinia* spp. such as *C. sorghicola; Cordylobia anthropophaga, Culex spp.* such as *C. nigripalpus, C. pipiens, C. quinquefasciatus, C. tarsalis, C. tritaeniorhynchus; Culicoides furens, Culiseta inornata, Culiseta melanura, Cuterebra* spp., *Dacus cucurbitae, Dacus oleae, Dasineura brassicae, Dasineura oxycoccana, Delia* spp. such as *D. antique, D. coarctata, D. platura, D. radicum; Dermatobia hominis, Drosophila* spp. such as *D. suzukii, Fannia* spp. such as *F. canicularis; Gastraphilus spp.* such as *G. intestinalis; Geomyza tipunctata, Glossina* spp. such as *G. fuscipes, G. morsitans, G. palpalis, G. tachinoides; Haematobia irritans, Haplodiplosis equestris, Hippelates* spp., Hylemyia spp. such as *H. platura; Hypoderma* spp. such as *H. lineata; Hyppobosca* spp., *Hydrellia philippina, Leptoconops torrens, Liriomyza* spp. such as *L. sativae, L. trifolii; Lucilia* spp. such as *L. caprina, L. cuprina, L. sericata; Lycoria pectoralis, Mansonia titillanus, Mayetiola* spp. such as *M. destructor; Musca* spp. such as *M. autumnalis, M. domestica; Muscina stabulans, Oestrus* spp. such as *O. ovis; Opomyza fiorum, Oscinella* spp. such as *O. frit; Orseolia oryzae, Pegomya hysocyami, Phlebotomus argentipes, Phorbia* spp. such as *P. antiqua, P. brassicae, P. coarctata; Phytomyza gymnostoma, Prosimulium mixtum, Psila rosae, Psorophora columbiae, Psorophora discolor, Rhagoletis* spp. such as *R. cerasi, R. cingulate, R. indifferens, R. mendax, R. pomonella; Rivellia quadrifasciata, Sarcophaga* spp. such as *S. haemorrhoidalis; Simulium vittatum, Sitodiplosis mosellana, Stomoxys* spp. such as *S. calcitrans; Tabanus* spp. such as *T. atratus, T. bovinus, T. lineola, T. similis; Tannia spp., Thecodiplosis japonensis, Tipula oleracea, Tipula paludosa,* and *Wohlfahrtia* spp;
insects from the order of Thysanoptera for example, *Baliothrips biformis, Dichromothrips corbetti, Dichromothrips* ssp., *Echinothrips americanus, Enneothrips flavens, Frankliniella* spp. such as *F. fusca, F. occidentalis, F. tritici; Heliothrips* spp., *Hercinothrips femoralis, Kakothrips* spp., *Microcephalothrips abdominalis, Neohydatothrips samayunkur, Pezothrips kellyanus, Rhipiphorothrips cruentatus, Scirtothrips* spp. such as *S. citri, S. dorsalis, S. perseae; Stenchaetothrips spp, Taeniothrips cardamoni, Taeniothrips inconsequens, Thrips* spp. such as *T.imagines, T.hawaiiensis, T. oryzae, T. palmi, T. parvispinus, T. tabaci;*
insects from the order of Hemiptera for example, *Acizzia jamatonica, Acrosternum* spp. such as *A. hilare;* Acyrthosipon spp. such as *A. onobrychis, A. pisum; Adelges laricis, Adelges tsugae, Adelphocoris* spp., such as *A. rapidus, A. superbus; Aeneolamia* spp., *Agonoscena* spp., *Aulacorthum solani, Aleurocanthus woglumi, Aleurodes* spp., *Aleurodicus disperses, Aleurolobus barodensis, Aleurothrixus* spp., *Amrasca* spp., *Anasa tristis, Antestiopsis* spp., *Anuraphis cardui, Aonidiella* spp., *Aphanostigma piri, Aphidula nasturtii, Aphis* spp. such as *A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella* spp., *Aspidiotus* spp., *Atanus* spp., *Aulacaspis yasumatsui, Aulacorthum solani, Bactericera cockerelli (Paratrioza cockerelli), Bemisia* spp. such as *B. argentifolii, B. tabaci (Aleurodes tabaci); Blissus* spp. such as *B. leucopterus; Brachycaudus* spp. such as *B. cardui, B. helichrysi, B. persicae, B. prunicola; Brachycolus* spp., *Brachycorynella asparagi, Brevicoryne brassicae, Cacopsylla* spp. such as *C. fulguralis, C. pyricola (Psylla piri); Calligypona marginata, Calocoris* spp., *Campylomma livida, Capitophorus horni, Carneocephala fulgida, Cavelerius* spp., *Ceraplastes* spp., *Ceratovacuna lanigera, Ceroplastes ceriferus, Cerosipha gossypii, Chaetosiphon fragaefolii, Chionaspis tegalensis, Chlorita onukii, Chromaphisjuglandicola, Chrysomphalus ficus, Cicadulina mbila, Cimex* spp. such as *C. hemipterus, C. lectularius; Coccomytilus halli, Coccus* spp. such as *C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus* spp., *Dasynus piperis, Dialeurodes* spp. such as *D. citrifolii; Dalbulus maidis, Diaphorina* spp. such as *D. citri; Diaspis* spp. such as *D. bromeliae; Dichelops furcatus, Diconocoris hewetti, Doralis* spp., *Dreyfusia nordmannianae, Dreyfusia piceae, Drosicha* spp., *Dysaphis* spp. such as *D. plantaginea, D. pyri, D. radicola; Dysaulacorthum pseudosolani, Dysdercus* spp. such as *D. cingulatus, D. intermedius; Dysmicoccus* spp., *Edessa* spp., *Geocoris* spp., *Empoasca* spp. such as *E. fabae, E. solana; Epidiaspis leperii, Eriosoma* spp. such as *E. lanigerum, E. pyricola; Erythroneura* spp., *Eurygaster* spp*.* such as *E. integriceps; Euscelis bilobatus, Euschistus* spp. such as *E. heros, E. impictiventris, E. servus; Fiorinia theae, Geococcus coffeae, Glycaspis brimblecombei, Halyomorpha* spp. such as *H. halys; Heliopeltis* spp., *Homalodisca vitripennis (=H. coagulata), Horcias nobilellus, Hyalopterus pruni, Hyperomyzus lactucae, Icerya* spp. such as /. *purchase; Idiocerus* spp., *Idioscopus* spp., *Laodelphax striatellus, Lecanium* spp., *Lecanoideus floccissimus, Lepidosaphes* spp. such as *L. ulmi; Leptocorisa* spp., *Leptoglossus phyllopus, Lipaphis erysimi, Lygus* spp. such as *L. hesperus, L. lineolaris, L. pratensis; Maconellicoccus hirsutus, Marchalina hellenica, Macropes excavatus, Macrosiphum* spp. such as *M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella* spp., *Metopolophium dirhodum, Monellia costalis, Monelliopsis pecanis, Myzocallis coryli, Murgantia* spp., *Myzus* spp. such as *M. ascalonicus, M. cerasi, M. nicotianae, M. persicae, M. varians; Nasonovia ribis-nigri, Neotoxoptera formosana, Neomegalotomus spp, Nephotettixspp.* such as *N. malayanus, N. nigropictus, N. parvus, N. virescens; Nezara* spp. such as *N. viridula; Nilaparvata lugens, Nysius huttoni, Oebalus* spp. such as *O. pugnax; Oncometopia* spp., *Orthezia praelonga, Oxycaraenus hyalinipennis, Parabemisia myricae, Parlatoria* spp., *Parthenolecanium* spp. such as *P. corni, P. persicae; Pemphigus* spp. such as *P. bursarius, P. populivenae; Peregrinus maidis, Perkinsiella saccharicida, Phenacoccus spp.* such as *P. aceris, P. gossypii; Phloeomyzus passerinii, Phorodon humuli, Phylloxera* spp. such as *P. devastatrix, Piesma quadrata, Piezodorus* spp. such as *P. guildinii; Pinnaspis aspidistrae, Planococcus* spp. such as *P. citri, P. ficus; Prosapia bicincta, Protopulvinaria pyriformis, Psallus seriatus, Pseudacysta persea, Pseudaulacaspis pentagona, Pseudococcus* spp. such as *P. comstocki; Psylla* spp. such as *P. mali; Pteromalus* spp., *Pulvinaria amygdali, Pyrilla* spp., *Quadraspidiotus* spp., such as *Q. perniciosus; Quesada gigas, Rastrococcus* spp., *Reduvius senilis, Rhizoecus americanus, Rhodnius* spp., *Rhopalomyzus ascalonicus, Rhopalosiphum* spp. such as *R. pseudobrassicas, R. insertum, R. maidis, R. padi; Sagatodes* spp., *Sah*/*bergella singularis, Saissetia* spp., *Sappaphis mala, Sappaphis mali, Scaptocoris* spp., *Scaphoides titanus, Schizaphis graminum, Schizoneura lanuginosa, Scotinophora* spp., *Selenaspidus articulatus, Sitobion avenae, Sogata* spp., *Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta* spp. such as *T. accerra, T. perditor; Tibraca* spp., *Tomaspis* spp., *Toxoptera* spp. such as *T. aurantii; Trialeurodes* spp. such as *T. abutilonea, T. ricini, T. vaporariorum; Triatoma* spp., *Trioza* spp., *Typhlocyba* spp., *Unaspis* spp. such as *U. citri, U. yanonensis; and Viteus vitifolii,*
Insects from the order Hymenoptera e.g. *Acanthomyops interjectus, Athalia rosae, Atta* spp. *such as A. capiguara, A. cephalotes, A. cephalotes, A. laevigata, A. robusta, A. sexdens, A. texana, Bombus* spp., *Brachymyrmex* spp*., Camponotus* spp. such as *C. floridanus, C. pennsylvanicus, C. modoc; Cardiocondyla nuda, Chalibion sp, Crematogasterspp., Dasymutilla occidentalis, Diprion* spp., *Dolichovespula maculata, Dorymyrmex* spp*., Dryocosmus kuriphilus, Formica spp., Hoplocampa* spp*.* such as *H. minuta, H. testudinea; Iridomyrmex humilis,* Lasius spp. such as *L. niger, Linepithema humile, Liometopum* spp., *Leptocybe invasa, Monomorium* spp. such as *M. pharaonis, Monomorium, Nylandria fulva, Pachycondyla chinensis, Paratrechina longicornis, Paravespula* spp., such as *P. germanica, P. pennsylvanica, P. vulgaris; Pheidole* spp. such as *P. megacephala; Pogonomyrmex* spp. such as *P. barbatus, P. californicus, Polistes rubiginosa, Prenolepis impairs, Pseudomyrmex gracilis, Schelipron* spp., *Sirex cyaneus, Solenopsis* spp*.* such as *S. geminata, S.invicta, S. molesta, S. richteri, S. xyloni, Sphecius speciosus, Sphex* spp*., Tapinoma* spp. such as *T. melanocephalum, T. sessile; Tetramorium* spp. such as *T. caespitum, T. bicarinatum, Vespa* spp. such as *V. crabro; Vespula* spp. such as *V. squamosal; Wasmannia auropunctata, Xylocopa* sp;
Insects from the order Orthoptera e.g. *Acheta domesticus, Calliptamus italicus, Chortoicetes terminifera, Ceuthophilus* spp., *Diastrammena asynamora, Dociostaurus maroccanus, Gryllota*/*pa* spp. such as *G. africana, G. gryllotalpa; Gryllus* spp., *Hieroglyphus daganensis, Kraussaria angulifera, Locusta* spp. such as *L. migratoria, L. pardalina; Melanoplus* spp. such as *M. bivittatus, M. femurrubrum, M. mexicanus, M. sanguinipes, M. spretus; Nomadacris septemfasciata, Oedaleus senegalensis, Scapteriscus* spp., *Schistocerca* spp. such as *S. americana, S. gregaria, Stemopelmatus* spp., *Tachycines asynamorus,* and *Zonozerus variegatus;*
Pests from the Class Arachnida e.g. Acari,e.g. of the families Argasidae, Ixodidae and Sarcoptidae, such as *Amblyomma* spp. (e.g. *A. americanum, A. variegatum, A. maculatum),* Argas spp. such as *A. persicu), Boophilus* spp. such as *B. annulatus, B. decoloratus, B. microplus, Dermacentor* spp*.* such as *D.silvarum, D. andersoni, D. variabilis, Hyalomma* spp. such as *H. truncatum, Ixodes* spp. such as *I. ricinus, I*. *rubicundus, I. scapularis, I*. *holocyclus, I. pacificus, Rhipicephalus sanguineus, Ornithodorus* spp. such as *O. moubata, O. hermsi, O. turicata, Ornithonyssus bacoti, Otobius megnini, Dermanyssus gallinae, Psoroptes* spp. such as *P. ovis, Rhipicephalus* spp. such as *R. sanguineus, R. appendiculatus, Rhipicephalus evertsi, Rhizoglyphus* spp*., Sarcoptes* spp*.* such as *S. Scabier,* and Family Eriophyidae including *Aceria* spp. such as *A. sheldoni, A. anthocoptes, Acallitus* spp., *Aculops* spp. such as *A. iycopersici, A. pelekassi; Aculus* spp. such as *A. schlechtendali; Colomerus vitis, Epitrimerus pyri, Phyllocoptruta oleivora; Eriophytes ribis* and *Eriophyes* spp. such as *Eriophyes sheldoni,* Family Tarsonemidae including *Hemitarsonemus* spp., *Phytonemus pallidus and Polyphagotarsonemus latus, Stenotarsonemus* spp. *Steneotarsonemus spinki,* Family Tenuipalpidae including Brevipalpus spp. such as *B. phoenicis,* Family Tetranychidae including *Eotetranychus* spp., *Eutetranychus* spp.*, Oligonychus* spp., *Petrobia latens, Tetranychus* spp. such as *T. cinnabarinus, T. evansi, T. kanzawai, T, pacificus, T. phaseulus, T. telarius* and *T. urticae; Bryobia praetiosa; Panonychus* spp. such as *P. ulmi, P. citri; Metatetranychus* spp. and *Oligonychus* spp. such as *O. pratensis, O. perseae, Vasates lycopersici, Raoiella indica, Family* Carpoglyphidae including *Carpoglyphus* spp.; *Penthaleidae* spp*.* such as *Halotydeus destructor,* Family Demodicidae with species such as *Demodexspp.;* Family Trombicidea including *Trombicula* spp.*;* Family Macronyssidae including *Ornothonyssus* spp.; Family Pyemotidae including *Pyemotes tritici, Tyrophagus putrescentiae;* Family Acaridae including *Acarus siro;* Family Araneida including *Latrodectus mactans, Tegenaria agrestis, Chiracanthium sp, Lycosa sp Achaearanea tepidariorum* and *Loxosceles reclusa;*
Pests from the Phylum Nematoda, for example, plant parasitic nematodes such as root-knot nematodes, *Meloidogyne* spp. such as *M. hapla, M. incognita, M. javanica;* cyst-forming nematodes, *Globodera* spp*.* such as *G. rostochiensis; Heterodera*spp. such as *H. avenae, H. glycines, H. schachtii, H. trifolii; Seed* gall nematodes, *Anguina* spp.; Stem and foliar nematodes, *Aphelenchoides* spp. such as *A. besseyi;* Sting nematodes, *Belonolaimus* spp. such as *B. longicaudatus;* Pine nematodes, *Bursaphelenchus* spp. such as *B. lignicolus, B. xylophilus;* Ring nematodes, *Criconema* spp., *Criconemella* spp. such as *C. xenoplax* and *C. ornata;* and, *Criconemoides* spp. such as *Criconemoides informis; Mesocriconema* spp.; Stem and bulb nematodes, *Ditylenchus* spp. such as *D. destructor, D. dipsaci;* Awl nematodes, *Dolichodorus* spp.*;* Spiral nematodes, *Heliocotylenchus multicinctus;* Sheath and sheathoid nematodes, *Hemicycliophora* spp. and *Hemicriconemoides* spp.; *Hirshmanniella* spp.; Lance nematodes, *Hoploaimus* spp.; False rootknot nematodes, *Nacobbus* spp.; Needle nematodes, *Longidorus* spp. such as *L. e*/*ongatus;* Lesion nematodes, *Pratylenchus* spp. such as *P. brachyurus, P. neglectus, P. penetrans, P. curvitatus, P. goodeyi;* Burrowing nematodes, *Radopholus* spp. such as *R. similis; Rhadopholus* spp.*; Rhodopholus* spp.*;* Reniform nematodes, *Rotylenchus* spp. such as *R. robustus, R. reniformis; Scutellonema* spp.*;* Stubby-root nematode, *Trichodorus* spp. such as *T. obtusus, T. primitivus; Paratrichodorus* spp. such as *P. minor;* Stunt nematodes, *Tylenchorhynchus* spp*.* such as *T. claytoni, T. dubius;* Citrus nematodes, *Tylenchulus* spp. such as *T. semipenetrans;* Dagger nematodes, *Xiphinema* spp.*;* and other plant parasitic nematode species;
Insects from the order Isoptera e.g. *Calotermes flavicollis, Coptotermes* spp. such as *C. formosanus, C. gestroi, C. acinaciformis; Cornitermes cumulans, Cryptotermes* spp. such as *C. brevis, C. cavifrons; Globitermes sulfureus, Heterotermes* spp. such as *H. aureus, H. longiceps, H. tenuis; Leucotermes fiavipes, Odontotermes spp., Incisitermes* spp. such as *I. minor, I*. *Snyder, Marginitermes hubbardi, Mastotermes* spp. such as *M. darwiniensis Neocapritermes* spp. such as *N. opacus, N. parvus; Neotermes* spp., *Procornitermes* spp., *Zootermopsis* spp. *such as Z. angusticollis, Z. nevadensis, Reticulitermes* spp. such as *R. hesperus, R. tibialis, R. speratus, R. flavipes, R. grassei, R. lucifugus, R. santonensis, R. virginicus; Termes natalensis,*
Insects from the order Blattaria e.g. *Blatta* spp. such as *B. orientalis, B. lateralis; Blattella* spp. such as *B. asahinae, B. germanica; Leucophaea maderae, Panchlora nivea, Periplaneta* spp. such as *P. americana, P. australasiae, P. brunnea, P. fuligginosa, P. japonica; Supella longipalpa, Parcoblatta pennsylvanica, Eurycotis floridana, Pycnoscelus surinamensis.*
Insects from the order Siphonoptera e.g. *Cediopsylla simples, Ceratophyllus* spp., *Ctenocephalides* spp. such as *C. felis, C. canis, Xenopsylla cheopis, Pulex irritans, Trichodectes canis, Tunga penetrans,* and *Nosopsyllus fasciatus,*
Insects from the order Thysanura e.g. *Lepisma saccharina, Ctenolepisma urbana,* and *Thermobia domestica,*
Pests from the class Chilopoda e.g. *Geophilus* spp., Scutigera spp. such as *Scutigera coleoptrata,*
Pests from the class Diplopoda e.g. *Blaniulus guttulatus, Julus* spp., *Narceus* spp.,
Pests from the class Symphyla e.g. *Scutigerella immaculata,*
Insects from the order Dermaptera, e.g. *Forficula auricularia,*
Insects from the order Collembola, e.g. *Onychiurus* spp., such as *Onychiurus armatus,*
Pests from the order Isopoda for example, *Armadillidium vulgare, Oniscus asellus, Porcellio scaber,*
Insects from the order Phthiraptera, e.g. *Damalinia* spp*.,* Pediculus spp. such as *Pediculus humanus capitis, Pediculus humanus corporis, Pediculus humanus humanus; Pthirus pubis,* Haematopinus spp. such as *Haematopinus eurysternus, Haematopinus suis;* Linognathus spp. such as *Linognathus vituli; Bovicola bovis, Menopon gallinae, Menacanthus stramineus* and *Solenopotes capillatus, Trichodectes* spp.,
Examples of further pest species which may be controlled by compounds of fomula I include: from the Phylum Mollusca, class Bivalvia, for example, *Dreissena* spp.; class Gastropoda, for example, *Arion* spp., *Biomphalaria* spp., *Bulinus* spp., *Deroceras* spp., *Galba* spp., *Lymnaea* spp., *Oncomelania* spp., *Pomacea canaliclata, Succinea* spp.*;* from the class of the helminths, for example, *Ancylostoma duodenale, Ancylostoma ceylanicum, Acylostoma brazi*/*iensis, Ancylostoma* spp., *Ascaris lubricoides, Ascaris spp., Brugia malayi, Brugia timori, Bunostomum* spp., *Chabertia* spp., *Clonorchis* spp., *Cooperia* spp., *Dicrocoelium* spp., *Dictyocaulus filaria, Diphyllobothrium latum, Dracunculus medinensis, Echinococcus granulosus, Echinococcus multilocularis, Enterobius vermicularis, Faciola* spp*.,* Haemonchus spp. such as *Haemonchus contortus; Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuelleborni, Strongyloides stercora lis, Stronyloides* spp.,
*Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.*

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or controlling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics such as Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at it's locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
fleas (Siphonaptera), e.g. *Ctenocephalides felis, Ctenocephalides canis, Xenopsylla cheopis, Pulexirritans, Tunga penetrans,* and *Nosopsyllus fasciatus;* cockroaches (Blattaria - Blattodea), e.g. *Blattella germanica, Blattella asahinae, Periplaneta americana, Periplaneta japonica, Periplaneta brunnea, Periplaneta fuligginosa, Periplaneta australasiae,* and *Blatta orientalis;* flies*,* mosquitoes (Diptera), e.g. *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola,* and *Tabanus similis;* lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus* and *Solenopotes capillatus;* ticks and parasitic mites (Parasitiformes): ticks (Ixodida), e.g. *Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata* and parasitic mites (Mesostigmata), e.g. *Ornithonyssus bacoti*and *Dermanyssus gallinae;* Actinedida (Prostigmata) und Acaridida (Astigmata), e.g. *Acarapis spp., Cheyletiella spp., Ornithocheyletia spp., Myobia spp., Psorergates spp., Demodex spp., Trombicula spp., Listrophorus spp., Acarus spp., Tyrophagus spp., Calog*/*yphus spp., Hypodectes spp., Pterolichus spp., Psoroptes spp., Chorioptes spp., Otodectes spp., Sarcoptes spp., Notoedres spp., Knemidocoptes spp., Cytodites spp.,* and *Laminosioptes spp;* Bugs (Heteropterida): *Cimex lectularius, Cimex hemipterus, Reduvius senilis, Triatoma spp., Rhodnius ssp., Panstrongylus ssp.,* and *Arilus critatus;* Anoplurida, e.g. *Haematopinus spp., Linognathus spp., Pediculus spp., Phtirus spp.,* and *Solenopotes spp.;* Mallophagida (suborders Arnblycerina and Ischnocerina), e.g. *Trimenopon spp., Menopon spp., Trinoton spp., Bovicola spp., Werneckiella spp., Lepikentron spp., Trichodectes spp.,* and *Felicola spp.;* Roundworms Nematoda: Wipeworms and Trichinosis (Trichosyringida), e.g. Trichinellidae *(Trichinella spp.),* (Trichuridae) *Trichuris spp., Capillaria spp.;* Rhabditida, e.g. *Rhabditis spp., Strongyloides spp., Helicephalobus spp.;* Strongylida, e.g. *Strongylus spp., Ancylostoma spp., Necator americanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus,* and *Dioctophyma renale;* Intestinal roundworms (Ascaridida), e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi;* Camallanida, e.g. *Dracunculus medinensis* (guinea worm); Spirurida, e.g. *Thelazia spp., Wuchereria spp., Brugia spp., Onchocerca spp., Dirofilari spp.a, Dipetalonema spp., Setaria spp., Elaeophora spp., Spirocerca lupi,* and *Habronema spp.;* Thorny headed worms (Acanthocephala), e.g. *Acanthocephalus spp., Macracanthorhynchus hirudinaceus* and *Oncicola spp.;* Planarians (Plathelminthes): Flukes (Trematoda), e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp.;* Cercomeromorpha, in particular Cestoda (Tape-worms), e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp..*

As used herein, the term "animal" includes warm-blooded animals (including humans) and fish. Preferred are mammals, such as cattle, sheep, swine, camels, deer, horses, pigs, poultry, rabbits, goats, dogs and cats, water buffalo, donkeys, fallow deer and reindeer, and also in furbearing animals such as mink, chinchilla and raccoon, birds such as hens, geese, turkeys and ducks and fish such as fresh- and salt-water fish such as trout, carp and eels. Particularly preferred are domestic animals, such as dogs or cats.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/ compositions used in the invention. A parasiticidally effective amount of the compositions will also vary according to the prevailing conditions such as desired parasiticidal effect and duration, target species, mode of application, and the like.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals in their drinking water. For oral administration, the dosage form chosen should provide the animal with 0.01 mg/kg to 100 mg/kg of animal body weight per day of the formula I compound, preferably with 0.5 mg/kg to 100 mg/kg of animal body weight per day.

Alternatively, the formula I compounds may be administered to animals parenterally, for example, by intraruminal, intramuscular, intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition the formula I compound may be transdermally administered to animals. For parenteral administration, the dosage form chosen should provide the animal with 0.01 mg/kg to 100 mg/kg of animal body weight per day of the formula I compound.

The formula I compounds may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the formula I compound. In addition, the formula I compounds may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries such as acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures. If appropriate, other auxiliaries such as colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art. Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80 per cent by weight, preferably from 0.1 to 65 per cent by weight, more preferably from 1 to 50 per cent by weight, most preferably from 5 to 40 per cent by weight.

Preparations which are diluted before use contain the compounds acting against ectoparasites in concentrations of 0.5 to 90 per cent by weight, preferably of 1 to 50 per cent by weight.

Furthermore, the preparations comprise the compounds of formula I against endoparasites in concentrations of 10 ppm to 2 per cent by weight, preferably of 0.05 to 0.9 per cent by weight, very particularly preferably of 0.005 to 0.25 per cent by weight.

Topical application may be conducted with compound-containing shaped articles such as collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### Examples

### A. Preparation examples

With appropriate modification of the starting materials, the procedures given in the synthesis description were used to obtain further compounds I. The compounds obtained in this manner are listed in the table that follows, together with physical data.

The products shown below were characterized by melting point determination, by NMR spectroscopy or by the masses ([m/z]) or retention time (RT; [min.]) determined by HPLC-MS or HPLC spectrometry. Enantionmers were separated by SFC.

HPLC-MS = high performance liquid chromatography-coupled mass spectrometry;
SFC = Supercritical fluid chromatography;
HPLC method A: HPLC method: Phenomenex Kinetex 1.7 µm XB-C18 100A; 50 x 2.1 mm; mobile phase: A: water + 0.1 % trifluoroacetic acid (TFA); B: acetonitrile; gradient: 5-100% B in 1.50 minutes; 100% B 0.25 min; flow: 0.8-1.0ml/min in 1.51 minutes at 60°C. MS: ESI positive, m/z 100-1400.

HPLC method B: HPLC Phenomenex Kinetex 1,7µm XB-C18 100A, 50 x 2,1mm", Mobile Phase: A: water + 0,1% TFA; B: Acetonitrile; Temperature: 60°C; Gradient: 5% B to 100% B in 1,50min; 100% B 0,25min; Flow: 0,8ml/min to 1,0ml/min in 1,51 min; MS method: ESI positive; Mass range (m/z): 100-700".

HPLC method C: Daicel Chiralpak AD-RH 5 µm 150 x 4.6 mm, mobile phase A: water + 0.1% v/v H₃PO₄, B: acetonitrile/2-propanol (1:1) Temperature 50°C, Gradient: 50%B 0 min, 50%B 10 min, 70% B 25 min, 100% B 30 min100%B 35 min, 50%B 35.5 min, total runtime 40 min; flow 1.2 mL/min. UV-detector lambda = 216 nm; BW 4 nm; pressure 130 bar.

SFC method D: Thar analytical SFC; Daicel ChiralCel OJ-3, 3µm,100*4.6 mm; mobile phase: A for CO₂ and B for methanol (0.05% isopropylamine); gradient: B% from 10% to 40% in 2.5 min, 40% B 2.0min; 40% B to 10% B in 0.2min; 10% B 0.5min; flow rate: 4.0 mL/min; back pressure: 100 bar; column temperature: 35°C, wavelength: 220 nm.

The synthesis of 2-chloro-4-[5-(3,5-dichloro-4-fluoro-phenyl)-5-(trifluoromethyl)-4H-isoxazol-3-yl]benzoic acid was performed in analogy to WO 2017/050922 and WO 2013/026695.

### Example 1: Synthesis of 2-Chloro-4-[5-(3,5-dichloro-4-fluoro-phenyl)-5-(trifluoromethyl)-4H-isoxazol-3-yl]-N-(1-methyl-2,5-dioxo-pyrrolidin-3-yl)benzamide [I-1-2]

To a solution of 364 mg 2-chloro-4-[5-(3,5-dichloro-4-fluoro-phenyl)-5-(trifluoromethyl)-4H-isoxazol-3-yl]benzoic acid, 200 mg 3-amino-1-methyl-pyrrolidine-2,5-dione hydrobromide (commercial), 446 mg Bromotripyrrolidinophosphonium hexafluorophosphate (PyBroP) in 15 mL dichloromethane was added 432 mg diisopropyl ehtylamine at 20-25°C and stirred for about 14 h. The mixture was concentrated at reduced pressure and purified via flash chromatography on silica gel to obtain the title compound (265 mg, 59%).

¹H NMR: (400 MHz, CDCl₃): δ 7.80 (d, 1H), 7.72 (s, 1H), 7.65-7.56 (m, 3H), 7.16 (d, 1H), 4.60 (m, 1H), 4.13 (d, 1H), 3.70 (d, 1H), 3.24 (dd, 1H), 3.09 (s, 3H), 2.91 (dd, 1H) ppm.

### Example 2: Synthesis of 4-[5-(3,5-Dichlorophenyl)-5-(trifluoromethyl)-4H-isoxazol-3-yl]-N-[(3R)-1-methoxy-2,5-dioxo-pyrrolidin-3-yl]-2-methyl-benzamide [I-1-55]

### Step 1: Synthesis of Benzyl N-[(3R)-1-methoxy-2,5-dioxo-pyrrolidin-3-yl]carbamate

To a solution of commercially available benzyl N-[(3*R*)-2,5-dioxotetrahydrofuran-3-yl]carbamate (20.0 g, 80.3 mmol) in CH₂Cl₂ (400 mL) at 20-25°C was sequentially added MeONH₂×HCl (7.10 g, 85.0 mmol), followed by N-methylmorpholine (8.93 g, 88.2 mmol), and the resulting mixture was stirred at that temperature for 6 h. After that time, 1,1'-carbonyldiimidazole (14.3 g, 88.2 mmol) was added portionwise to the above mixture and stirring was continued over night. The resulting reaction mixture was transferred to a separatory funnel and the organic phase washed with HCl solution (10% aqueous, 2×100 mL), NaCl solution (sat. aqueous, 1×100 mL), dried over Na₂SO₄, filtered and concentrated under reduced pressure. Purification by column chromatograohy (EtOAc/cyclohexane 0:100 to 60:40, gradient), followed by recrystallization from MTBE afforded the title compound as a white solid (12.1 g, 54%).
LC-MS: Mass calculated for C₁₃H₁₅N₂O₅⁺ [(M+H)⁺)] 279.1, found 278.9; RT= 0.844 min.

### Step 2: Synthesis of (3R)-3-Amino-1-methoxy-pyrrolidine-2,5-dione hydrochloride

To a solution of benzyl N-[(3R)-1-methoxy-2,5-dioxo-pyrrolidin-3-yl]carbamate (3.00 g, 10.8 mmol) in THF (120 mL) at 20-25°C under N₂ atmosphere was added 1.0 M aq. HCI solution (15.0 mL, 15.0 mmol), followed by Pd(OH)₂, 20 wt% on carbon (454 mg, 0.647 mmol). The flask was purged with H₂ using a gas burette and the resulting suspension was vigorously stirred under H₂ atmosphere for 2 h. Then the flask was purged with N₂ and the resulting reaction mixture filtered through a short plug of Celite, eluting with MeOH. The filtrate was concentrated under reduced pressure, the residue dried *via* azeotropical distillation with toluene (3×), to afford the title compound (2.08 g) in crude form which was used in the next step without further purification.
¹H NMR (400 MHz, DMSO-d6): 9.02 (br s, 3H), 4.33 (dd, J = 8.9, 4.5 Hz, 1H), 3.86 (s, 3H), 3.03 (dd, J = 17.5, 9.0 Hz, 1H), 2.79 (dd, J = 17.5, 4.5 Hz, 1H).

### Step 3: Synthesis of 4-[5-(3,5-Dichlorophenyl)-5-(trifluoromethyl)-4H-isoxazol-3-yl]-N-[(3R)-1-methoxy-2,5-dioxo-pyrrolidin-3-yl]-2-methyl-benzamide

To a solution of 4-[5-(3,5-dichlorophenyl)-5-(trifluoromethyl)-4H-isoxazol-3-yl]-2-methyl-benzoic acid (965 mg, 2.31 mmol), (3R)-3-amino-1-methoxy-pyrrolidine-2,5-dione hydrochloride (500 mg, 2.77 mmol), and PyBroP (1.29 g, 2,77 mmol) in CH₂Cl₂ (20 mL) at 20-25°C was added iPr₂NEt (1.25 g, 9.69 mmol) and the resulting reaction mixture was stirred at that temperature over night. Concentration of the reaction mixture under reduced pressure and purification by column chromatography (EtOAc/cyclohexane 0:100 to 100:0, gradient) afforded the title compound (939 mg, 75%) as a white solid and an enantiomeric ratio of SR/RR/SS/RS = 46:46:4:4 (HPLC-Method C).
LC-MS: Mass calculated for C₂₃H₁₉CbF₃N₃O₅⁺ [(M+H)⁺)] 544.1, found 544.0; RT= 1.289 min

### Example 3: 2-Chloro-N-[(3R)-1-cyclopropyl-2,5-dioxo-pyrrolidin-3-yl]-4-[(5S)-5-(3,5-dichloro-4-fluoro-phenyl)-5-(trifluoromethyl)-4H-isoxazol-3-yl]benzamide [I-1-25]

### Step 1: Synthesis of Benzyl N-[(3R)-1-cyclopropyl-2,5-dioxo-pyrrolidin-3-yl]carbamate

To a solution of commercially available benzyl N-[(3*R*)-2,5-dioxotetrahydrofuran-3-yl]carbamate (1.15 g, 4.61 mmol) in CH₂Cl₂ (50 mL) at 20-25°C was added cyclopropylamine (279 mg, 5.57 mmol), followed by N-methylmorpholine (513 mg, 5.07 mmol), and the resulting mixture was stirred at 20-25°C over night. After that time, 1,1'-carbonyldiimidazole (823 mg, 5.08 mmol) was added portionwise and the mixture was heated at reflux for 3 hours. The resulting reaction mixture was allowed to cool to 20-25°C, transferred to a separatory funnel and the organic phase was washed with HCI solution (10% aqueous, 2 × 10 mL), NaCl solution (sat. aqueous, 1 × 10 mL), dried over Na₂SO₄, filtered and concentrated under reduced pressure. Purification by column chromatograohy (EtOAc/cyclohexane 0:100 to 60:40, gradient) afforded the title compound as a white solid (757 mg, 57%).
LC-MS: Mass calculated for C₁₅H₁₇N₂O₄⁺ [(M+H)⁺)] 289.1, found 288.9; RT= 0.890 min.

### Step 2: Synthesis of (3R)-3-Amino-1-cyclopropyl-pyrrolidine-2,5-dione hydrobromide

In a 100 mL round bottom flask, benzyl N-[(3R)-1-cyclopropyl-2,5-dioxo-pyrrolidin-3-yl]carbamate (757 mg, 2.62 mmol), at 20-25°C was dissolved in HBr solution, 33% in AcOH (20.0 mL) and the resulting mixture was stirred at that temperature for 1.5 h. After that time, the reaction mixture was poured on H₂O (40 mL) and the aqueous phase was washed with CH₂Cl₂ (3 × 20 mL). The combined organic washes were extracted with H₂O (1 × 10 mL) and the combined aqueous phases concentrated under reduced pressure. The residue was dried via azeotropical distillation with toluene (5×), to afford the title compound (568 mg) in crude form which was used in the next step withouth further purification.

¹H NMR (400 MHz, DMSO-d6): 8.50 (s, 3H), 4.25 (dd, J = 9.2, 5.2 Hz, 1H), 2.95 (dd, J = 17.7, 9.2 Hz, 1H), 2.60 (dd, J = 17.8, 5.3 Hz, 1H), 2.56-2.51 (m, 1H), 0.92-0.72 (m, 4H).

### Step 3: Synthesis of 2-Chloro-N-[(3R)-1-cyclopropyl-2,5-dioxo-pyrrolidin-3-yl]-4-[(5S)-5-(3,5-dichloro-4-fluoro-phenyl)-5-(trifluoromethyl)-4H-isoxazol-3-yl]benzamide

To a solution of 2-chloro-4-[(5S)-5-(3,5-dichloro-4-fluoro-phenyl)-5-(trifluoromethyl)-4H-isoxazol-3-yl]benzoic acid (468 mg, 1.02 mmol; known from WO 2017/050921), (3R)-3-amino-1-cyclopropyl-pyrrolidine-2,5-dione hydrobromide (289 mg, 1.23 mmol), and PyBroP (573 mg, 1.23 mmol) in CH₂Cl₂ (20 mL) at 20-25°C was added iPr₂NEt (556 mg, 4.30 mmol) and the resulting reaction mixture was stirred at that temperature over night. Concentration of the reaction mixture under reduced pressure and purification by column chromatography (EtOAc/cyclohexane 0:100 to 80:20, gradient) afforded the title compound (607 mg, 100%) as a white solid and in an enantiomeric ratio of SR/RR/SS/RS = 97:0:3:0 (HPLC-Method C).

LC-MS: Mass calculated for C₂₄H₁₇Cl₃F₄N₃O₄⁺ [(M+H)⁺)] 592.0, found 591.8; RT= 1.341 min

### Example 4: 2-Chloro-4-[5-(3,5-dichlorophenyl)-5-(trifluoromethyl)-4H-isoxazol-3-yl]-N-[(3R)-1-(2-methoxyethyl)-2,5-dioxo-pyrrolidin-3-yl]benzamide [I-1-106]

### Step 1: Synthesis of (3R)-3-Amino-1-(2-methoxyethyl)pyrrolidine-2,5-dione hydrochloride

To a solution of benzyl N-[(3R)-1-(2-methoxyethyl)-2,5-dioxo-pyrrolidin-3-yl]carbamate (3.80 g, 12.4 mmol), prepared analogously to Example 3, dissolved in MeOH (50 mL) at 20-25°C under N₂ atmosphere was added Pd, 10 wt% on carbon (2.64 g, 2.48 mmol). The flask was purged with H₂ from a balloon source and the resulting reaction mixture was stirred under H₂ atmosphere over night. Then the reaction mixture was purged with N₂, filtered through a plug of Celite and the filtrate was concentrated under reduced pressure. The residue was taken up in 2.0 M aq. HCI solution (50 mL), washed with CH₂Cl₂ (3 × 5 mL), and the aqueous phase concentrated under reduced pressure. The residue was dried via azeotropical distillation with toluene (3×), to afford the title compound (1.98 g) in crude form which was used in the next step withouth further purification.

LC-MS: Mass calculated for C₇H₁₃N₂O₃⁺ [(M+H)⁺)] 173.1, found 172.9; RT= 0.274 min.

### Step 2: Synthesis of 2-Chloro-4-[5-(3,5-dichlorophenyl)-5-(trifluoromethyl)-4H-isoxazol-3-yl]-N-[(3R)-1-(2-methoxyethyl)-2,5-dioxo-pyrrolidin-3-yl]benzamide

2-Chloro-4-[5-(3,5-dichlorophenyl)-5-(trifluoromethyl)-4H-isoxazol-3-yl]benzoic acid (520 mg, 1.19 mmol) and (3R)-3-Amino-1-(2-methoxyethyl)pyrrolidine-2,5-dione hydrochloride (300 mg, 1.44 mmol) were reacted in an amide coupling reaction, analogously to Example 3, to afford the title compound (162 mg, 22%) as a white solid and an enantiomeric ratio of SR/RR/SS/RS = 44:44:6:6 (HPLC-Method C).

LC-MS: Mass calculated for C₂₄H₂₀Cl₃F₃N₃O₅⁺ [(M+H)⁺)] 592.0, found 592.0; RT= 1.347 min

### Example 5: 2-Chloro-4-[5-(3,5-dichlorophenyl)-5-(trifluoromethyl)-4H-isoxazol-3-yl]-N-[(3R)-1-(2-hydroxyethyl)-2,5-dioxo-pyrrolidin-3-yl]benzamide [I-1-104]

### Step 1: Synthesis of (3R)-3-Amino-1-(2-hydroxyethyl)pyrrolidine-2,5-dione hydrobromide

Benzyl N-[(3R)-1-(2-methoxyethyl)-2,5-dioxo-pyrrolidin-3-yl]carbamate (3.70 g, 12.1 mmol), prepared analogously to Example 3, at 20-25°C was dissolved in HBr solution, 33% in AcOH (40.0 mL) and the resulting mixture was stirred at that temperature for 1.5 h. Then the reaction mixture was poured on H₂O (200 mL), the aqueous phase washed with CH₂Cl₂ (3 × 15 mL) and concentrated under reduced pressure. The residue was dried via azeotropical distillation with toluene (3×), to afford the title product (3.00 g) in crude form which was used in the next step withouth further purification.

¹H NMR (400 MHz, MeOH-d4): 4.41 (dd, J = 9.3, 5.6 Hz, 1H), 3.74 - 3.65 (m, 4H), 3.19 (dd, J = 17.9, 9.3 Hz, 1H), 2.74 (dd, J = 17.9, 5.6 Hz, 1H).

### Step 2: Synthesis of 2-Chloro-4-[5-(3,5-dichlorophenyl)-5-(trifluoromethyl)-4H-isoxazol-3-yl]-N-[(3R)-1-(2-hydroxyethyl)-2,5-dioxo-pyrrolidin-3-yl]benzamide

2-Chloro-4-[5-(3,5-dichlorophenyl)-5-(trifluoromethyl)-4H-isoxazol-3-yl]benzoic acid (550 mg, 1.26 mmol) and (3R)-3-Amino-1-(2-hydroxyethyl)pyrrolidine-2,5-dione hydrobromide (300 mg, 1.26 mmol) were reacted in an amide coupling reaction, analogously to Example 3, to afford the title compound (576 mg, 75%) in an enantiomeric ratio of SR/RR/SS/RS = 44:44:6:6 (HPLC-Method C).

LC-MS: Mass calculated for C₂₃H₁₈Cl₃F₃N₃O₅⁺ [(M+H)⁺)] 578.0, found 577.8; RT= 1.249 min

### Example 6: 2-Chloro-4-[5-(3,5-dichloro-4-fluoro-phenyl)-5-(trifluoromethyl)-4H-isoxazol-3-yl]-N-[(3R)-1-hydroxy-2,5-dioxo-pyrrolidin-3-yl]benzamide [I-1-33]

To a solution of N-[(3R)-1-benzyloxy-2,5-dioxo-pyrrolidin-3-yl]-2-chloro-4-[5-(3,5-dichloro-4-fluoro-phenyl)-5-(trifluoromethyl)-4H-isoxazol-3-yl]benzamide (284 mg, 0.431 mmol, SR/RR/SS/RS = 45:45:5:5 as determined with HPLC-Method C), prepared analogously to Example 3, in EtOAc (10 mL) at 20-25°C under N₂ atmosphere was added Pd, 10 wt% on carbon (60.0 mg, 0.0564 mmol). The flask was purged with H₂ from a balloon source and the resulting reaction mixture was stirred under H₂ atmosphere over 2 days. Then the reaction mixture was purged with N₂, filtered through a plug of Celite and the filtrate was concentrated under reduced pressure. Purification by column chromatography (EtOAc/cyclohexane 0:100 to 80:20, gradient) afforded the title compound (205 mg, 75%).

LC-MS: Mass calculated for C₂₁H₁₄Cl₃F₄N₃O₅⁺ [(M+H)+)] 568.0, found 567.7; RT= 1.250 min

### Example 7: 4-[5-(3,5-Dichlorophenyl)-5-(trifluoromethyl)-4H-isoxazol-3-yl]-2-methyl-N-[(3R)-1-(methylamino)-2,5-dioxo-pyrrolidin-3-yl]benzamide [I-1-45]

### Step 1: Synthesis of Benzyl N-[(3R)-3-(benzyloxycarbonylamino)-2,5-dioxo-pyrrolidin-1-yl]-N-methyl-carbamate

To a solution of commercially available benzyl N-[(3R)-2,5-dioxotetrahydrofuran-3-yl]carbamate (3.60 g, 14.4 mmol) in CH₂Cl₂ (100 mL) at 20-25°C was sequentially added benzyl 1-methylhydrazinecarboxylate (2.76 g, 15.3 mmol), followed by N-methylmorpholine (1.61 g, 15.9 mmol), and the resulting mixture was stirred at that temperature over night. Then 1,1'-carbonyldiimidazole (2.58 g, 15.9 mmol) was added portionwise to the mixture and the mixture was refluxed for 3 hours. Additional 1,1'-carbonyldiimidazole (2.58 g, 15.9 mmol) was added and refluxing was continued for another 3 hours. The reaction mixture was allowed to cool to 20-25°C, transferred to a separatory funnel and the organic phase was washed with 10% aq. HCI solution (2×20 mL), NaCl solution (sat. aqueous, 1×20 mL), dried over Na₂SO₄, filtered and concentrated under reduced pressure. Purification by column chromatograohy (EtOAc/cyclohexane 0:100 to 60:40, gradient), afforded the title compound (1.76 g, 30%).

LC-MS: Mass calculated for C₂₁H₂₂N₃O₆⁺ [(M+H)⁺)] 412.2, found 412.0; RT= 1.109 min

### Step 2: From the above benzyl N-[(3R)-3-(benzyloxycarbonylamino)-2,5-dioxo-pyrrolidin-1-yl]-N-methyl-carbamate, the desired 4-[5-(3,5-dichlorophenyl)-5-(trifluoromethyl)-4H-isoxazol-3-yl]-2-methyl-N-[(3R)-1-(methylamino)-2,5-dioxo-pyrrolidin-3-yl]benzamide was obtained analogously to Example 2.

### Example 8: Separation of enantiomers of 2-chloro-4-[5-(3,5-dichloro-4-fluoro-phenyl)-5-(trifluoromethyl)-4H-isoxazol-3-yl]benzoic acid

A solution of 24 g racemic 2-chloro-4-[5-(3,5-dichloro-4-fluoro-phenyl)-5-(trifluoromethyl)-4H-isoxazol-3-yl]benzoic acid in methanol at 100 mg/mL was filtered through a membrane with pore size 0.45 µm. The filtrate was injected into a Thar 80 preparative SFC instrument at 2.5 mL per injection. Separation was performed by a Daicel ChiralCel OJ column, 10 µm, 300x30 mm. Mobile phase A was CO₂ and B was methanol, the gradient was 35% B at a flow rate of 75 g/min. The system was run with a back pressure of 100 bar at 40°C column temperature. Cycle time was 8 minutes. Detection was measured at 220 nm. After separation, the fractions were dried off via rotary evaporator at a bath temperature of 35°C to yield the faster eluting S-enantiomer (9.51 g), and the slower eluting R-enantiomer (9.72 g), repsectively.
Analytical SFC (Method D): S-enantiomer: RT = 0.53 min, >99% e.e.; R-enantiomer: RT = 2.32 min, >99% e.e.

**Table C.1 - Compounds of formula I.1**

| | | | | | | | | | | |
|---|---|---|---|---|---|---|---|---|---|---|
| 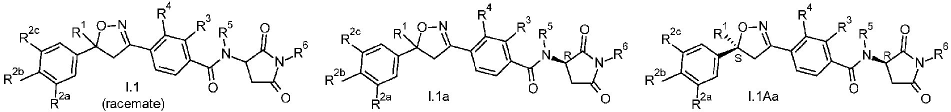 | | | | | | | | | | |
| 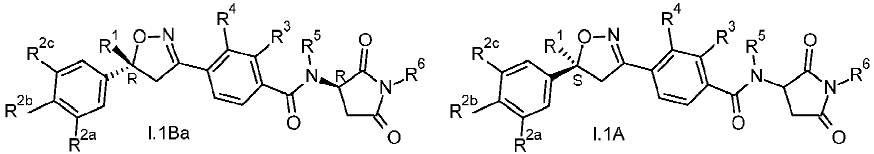 | | | | | | | | | | |
| For all compounds R¹ = CF₃, R⁵ = H | | | | | | | | | | |

| No. | R^{2a} | R^{2b} | R^{2c} | R⁴ | R³ | R⁶ | Isomer^{x)} | HPLC R^{t} [min] | M+H [m/z] | HPLC Method |
|---|---|---|---|---|---|---|---|---|---|---|
| I-1-1 | Cl | F | Cl | H | Cl | CH₂C₆H₅ | I.1 | 1.387 | 644 | A |
| I-1-2 | Cl | F | Cl | H | Cl | CH₃ | I.1 | 1.300 | 566 | A |
| I-1-3 | Cl | F | Cl | H | Cl | CH₂CH₃ | I.1 | 1.303 | 580 | A |
| I-1-4 | Cl | F | Cl | H | Cl | CH₂CH=CH₂ | I.1 | 1.328 | 594.8 | A |
| I-1-5 | Cl | F | Cl | H | Cl | CH₂C(=O)OCH₃ | I.1 | 1.297 | 623.9 | A |
| I-1-6 | Cl | F | Cl | H | Cl | CH₂CF₃ | I.1 | 1.344 | 636 | A |
| I-1-7 | Cl | F | Cl | H | Cl | CH₂CH₂CF₃ | I.1 | 1.351 | 649.9 | A |
| I-1-8 | Cl | F | Cl | H | Cl | CH₃ | I.1a | 1.305 | 565.9 | A |
| I-1-9 | Cl | F | Cl | H | Cl | CH₃ | I.1a | 1.305 | 568 | A |
| I-1-10 | Cl | F | Cl | -CH₂CH₂CH₂- | | CH₃ | I.1 | 1.363 | 572 | B |
| I-1-11 | H | F | Cl | -CH₂CH₂CH₂- | | CH₃ | I.1 | 1.282 | 538.1 | B |
| I-1-12 | Cl | H | Cl | -CH₂CH₂CH₂- | | CH₃ | I.1 | 1.361 | 553.9 | B |
| I-1-13 | Cl | F | Cl | H | Cl | CH₃ | I.1Aa | 1.311 | 567.9 | A |
| I-1-14 | Cl | F | Cl | H | Cl | CH₂CH₂F | I.1a | 1.331 | 619.9 | A |
| I-1-15 | Cl | F | Cl | H | Cl | CH₂CHF₂ | I.1a | 1.355 | 615.9 | A |
| I-1-16 | Cl | F | Cl | H | Cl | c-C₃H₅ | I.1a | 1.340 | 593.8 | A |
| I-1-17 | Cl | F | Cl | H | Cl | CH₃ | I.1Ba | 1.311 | 565.9 | A |
| I-1-18 | H | F | Cl | H | Cl | CH₃ | I.1a | 1.212 | 532 | A |
| I-1-19 | Cl | H | Cl | H | Cl | CH₃ | I.1a | 1.281 | 548 | A |
| I-1-20 | Cl | F | Cl | H | Cl | N(CH₃)₂ | I.1Aa | 1.299 | 595 | A |
| I-1-21 | Cl | F | Cl | H | Cl | CH₂CH₃ | I.1Aa | 1.347 | 579.8 | A |
| I-1-22 | Cl | H | Cl | H | Cl | N(CH₃)₂ | I.1a | 1.290 | 578.8 | A |
| I-1-23 | Cl | F | Cl | H | Cl | CH₂CH₂F | I.1Aa | 1.332 | 597.9 | A |
| I-1-24 | Cl | F | Cl | H | Cl | CH₂CHF₂ | I.1Aa | 1.357 | 615.7 | A |
| I-1-25 | Cl | F | Cl | H | Cl | c-C₃H₅ | I.1Aa | 1.341 | 591.8 | A |
| I-1-26 | Cl | F | Cl | H | Cl | OCH₂C₆H₅ | I.1a | 1.433 | 659.9 | A |
| I-1-27 | Cl | F | Cl | H | CH₃ | CH₃ | I.1a | 1.301 | 545.9 | A |
| I-1-28 | Cl | H | Cl | H | CH₃ | CH₃ | I.1a | 1.288 | 527.9 | A |
| I-1-29 | Cl | H | Cl | H | CH₃ | CH₂CHF₂ | I.1a | 1.315 | 578.1 | B |
| I-1-30 | Cl | H | Cl | H | Cl | CH₂CHF₂ | I.1a | 1.322 | 599.8 | B |
| I-1-31 | Cl | F | Cl | H | CH₃ | CH₂CHF₂ | I.1a | 1.326 | 596 | B |
| I-1-32 | Cl | F | Cl | H | Cl | CH₃ | I.1A | 1.304 | 567.8 | A |
| I-1-33 | Cl | F | Cl | H | Cl | OH | I.1a | 1.250 | 569.7 | A |
| I-1-34 | Cl | F | Cl | H | CH₃ | N(CH₃)₂ | I.1 | 1.304 | 574.9 | B |
| I-1-35 | Cl | F | Cl | H | CH₃ | N(CH₃)₂ | I.1a | 1.293 | 575 | B |
| I-1-36 | Cl | H | Cl | H | Cl | CH₂CH₂CH₃ | I.1a | 1.363 | 577.9 | A |
| I-1-37 | Cl | F | Cl | H | Cl | CH₂CH₂CH₃ | I.1Aa | 1.372 | 596 | A |
| I-1-38 | H | F | Cl | H | Cl | CH₂CH₂CH₃ | I.1a | 1.291 | 560.1 | A |
| I-1-39 | Cl | F | Cl | H | Cl | SO₂N(CH₃)₂ | I.1a | 1.417 | 661 | A |
| I-1-40 | Cl | F | Cl | H | Cl | OCH₃ | I.1Aa | 1.314 | 581.8 | A |
| I-1-41 | Cl | H | Cl | H | CH₃ | CH₂CH₂CH₃ | I.1a | 1.368 | 555.9 | A |
| I-1-42 | Cl | H | Cl | H | Cl | OCH₃ | I.1a | 1.297 | 565.9 | A |
| I-1-43 | H | F | Cl | H | Cl | OCH₃ | I.1a | 1.225 | 548 | A |
| I-1-44 | Cl | F | Cl | H | CH₃ | OCH₃ | I.1a | 1.309 | 561.9 | A |
| I-1-45 | Cl | H | Cl | H | CH₃ | NHCH₃ | I.1a | 1.251 | 542.9 | A |
| I-1-46 | Cl | F | Cl | H | CH₃ | CH(CH₃)₂ | I.1a | 1.375 | 574 | B |
| I-1-47 | Cl | H | Cl | H | CH₃ | CH(CH₃)₂ | I.1a | 1.365 | 555.9 | B |
| I-1-48 | Cl | H | Cl | H | Cl | CH(CH₃)₂ | I.1a | 1.372 | 577.9 | B |
| I-1-49 | H | F | Cl | H | Cl | CH(CH₃)₂ | I.1a | 1.302 | 559.9 | B |
| I-1-50 | Cl | F | Cl | H | CH₃ | CH₂CH₃ | I.1a | 1.309 | 560 | B |
| I-1-51 | Cl | H | Cl | H | CH₃ | CH₂CH₃ | I.1a | 1.307 | 542 | B |
| I-1-52 | Cl | H | Cl | H | Cl | CH₂CH₃ | I.1a | 1.317 | 562 | B |
| I-1-53 | H | F | Cl | H | Cl | CH₂CH₃ | I.1a | 1.247 | 546 | B |
| I-1-54 | Cl | F | Cl | H | Cl | CH(CH₃)₂ | I.1a | 1.381 | 595.8 | B |
| I-1-55 | Cl | H | Cl | H | CH₃ | OCH₃ | I.1a | 1.285 | 544.1 | A |
| I-1-56 | Cl | F | Cl | H | CH₃ | OCH₂CF₃ | I.1a | 1.399 | 530.1 | B |
| I-1-57 | Cl | F | Cl | H | CH₃ | CH₂CF₃ | I.1a | 1.366 | 614 | B |
| I-1-58 | H | F | Cl | H | Cl | CH₂CF₃ | I.1a | 1.297 | 600 | B |
| I-1-59 | Cl | F | Cl | H | Cl | CH₂CF₃ | I.1a | 1.372 | 635.9 | B |
| I-1-60 | Cl | H | Cl | H | CH₃ | CH₂CF₃ | I.1a | 1.356 | 596.1 | B |
| I-1-61 | Cl | H | Cl | H | Cl | CH₂CF₃ | I.1a | 1.362 | 617.9 | B |
| I-1-62 | Cl | H | Cl | H | CH₃ | NHCH₃ | I.1 | 1.239 | 543 | A |
| I-1-63 | Cl | H | Cl | H | Cl | NHCH₃ | I.1 | 1.251 | 563 | A |
| I-1-64 | H | F | Cl | H | Cl | NHCH₃ | I.1 | 1.180 | 547 | A |
| I-1-65 | Cl | F | Cl | H | CH₃ | CH₂CH₂CH₃ | I.1a | 1.371 | 573.9 | A |
| I-1-66 | Cl | F | Cl | H | CH₃ | CH₂CH(CH₃)₂ | I.1a | 1.397 | 588.1 | A |
| I-1-67 | Cl | H | Cl | H | CH₃ | CH₂CH(CH₃)₂ | I.1a | 1.388 | 570.1 | A |
| I-1-68 | Cl | F | Cl | H | Cl | NHCH₃ | I.1a | 1.264 | 582.9 | A |
| I-1-69 | Cl | F | Cl | H | Cl | OCH₃ | I.1 | 1.319 | 583.8 | A |
| I-1-70 | Cl | H | Cl | H | Cl | CH₂CH(CH₃)₂ | I.1a | 1.393 | 592 | A |
| I-1-71 | Cl | F | Cl | H | CH₃ | NHCH₃ | I.1a | 1.250 | 561 | A |
| I-1-72 | Cl | F | Cl | H | Cl | OCH₂CF₃ | I.1a | 1.433 | 549.9 | B |
| I-1-73 | Cl | H | Cl | H | CH₃ | OCH₂CF₃ | I.1a | 1.389 | 612 | B |
| I-1-74 | H | F | Cl | H | Cl | OCH₂CF₃ | I.1a | 1.341 | 616 | B |
| I-1-75 | Cl | H | Cl | H | Cl | OCH₂CF₃ | I.1a | 1.418 | 633.9 | B |
| I-1-76 | Cl | F | Cl | H | Cl | OCH₂CF₃ | I.1Aa | 1.373 | 651.9 | B |
| I-1-77 | H | F | Cl | H | Cl | N(CH₃)₂ | I.1a | 1.244 | 560.9 | B |
| I-1-78 | Cl | H | Cl | H | CH₃ | N(CH₃)₂ | I.1a | 1.306 | 556.9 | B |
| I-1-79 | Cl | F | Cl | H | Cl | N(CH₃)₂ | I.1a | 1.325 | 596.8 | B |
| I-1-80 | Cl | F | Cl | H | Cl | CH₂CH(CH₃)₂ | I.1Aa | 1.390 | 608 | A |
| I-1-81 | H | F | Cl | H | Cl | CH₂CH(CH₃)₂ | I.1a | 1.357 | 573.9 | A |
| I-1-82 | Cl | F | Cl | H | Cl | OCH₂CH₃ | I.1Aa | 1.361 | 597.9 | B |
| I-1-83 | Cl | F | Cl | H | Cl | CH₂CH₂OH | I.1a | 1.262 | 597.8 | B |
| I-1-84 | H | F | Cl | H | Cl | CH₂CHF₂ | I.1a | 1.259 | 582 | B |
| I-1-85 | H | F | Cl | H | Cl | CH₂CH₂OH | I.1a | 1.178 | 561.8 | B |
| I-1-86 | Cl | H | Cl | H | Cl | OH | I.1 | 1.229 | 551.8 | B |
| I-1-87 | Cl | H | Cl | H | CH₃ | OH | I.1 | 1.362 | 530 | B |
| I-1-88 | H | F | Cl | H | Cl | OH | I.1 | 1.162 | 534 | B |
| I-1-89 | Cl | F | Cl | H | CH₃ | c-C₃H₅ | I.1a | 1.299 | 572 | A |
| I-1-90 | Cl | F | Cl | H | CH₃ | OH | I.1 | 1.247 | 547.8 | B |
| I-1-91 | Cl | H | Cl | H | CH₃ | c-C3H₅ | I.1a | 1.289 | 554 | A |
| I-1-92 | Cl | H | Cl | H | Cl | c-C₃H₅ | I.1a | 1.297 | 575.8 | A |
| I-1-93 | H | F | Cl | H | Cl | c-C₃H₅ | I.1a | 1.230 | 558 | A |
| I-1-94 | Cl | H | Cl | H | CH₃ | OCH₂CH₃ | I.1a | 1.367 | 558 | B |
| I-1-95 | Cl | F | Cl | H | CH₃ | CH₂CH₂OH | I.1a | 1.241 | 576 | B |
| I-1-96 | Cl | H | Cl | H | CH₃ | CH₂CH₂OH | I.1a | 1.227 | 558 | B |
| I-1-97 | Cl | F | Cl | H | CH₃ | CH₂CH₂F | I.1a | 1.330 | 577.9 | B |
| I-1-98 | Cl | H | Cl | H | CH₃ | CH₂CH₂F | I.1a | 1.318 | 559.9 | B |
| I-1-99 | Cl | H | Cl | H | Cl | CH₂CH₂F | I.1a | 1.306 | 580 | B |
| I-1-100 | H | F | Cl | H | Cl | CH₂CH₂F | I.1a | 1.242 | 564 | B |
| I-1-101 | Cl | F | Cl | H | CH₃ | OCH₂CH₃ | I.1a | 1.386 | 576 | B |
| I-1-102 | Cl | H | Cl | H | Cl | OCH₂CH₃ | I.1a | 1.327 | 577.9 | B |
| 1-1-103 | H | F | CI | H | CI | OCH₂CH₃ | 1.1a | 1.312 | 562 | B |
| I-1-104 | Cl | H | Cl | H | Cl | CH₂CH₂OH | I.1a | 1.249 | 577.8 | B |
| I-1-105 | H | F | Cl | H | Cl | CH₂CH₂OCH₃ | I.1a | 1.282 | 578 | B |
| I-1-106 | Cl | H | Cl | H | Cl | CH₂CH₂OCH₃ | I.1a | 1.347 | 592 | B |
| I-1-107 | Cl | H | Cl | H | CH₃ | CH₂CH₂OCH₃ | I.1a | 1.348 | 572.1 | B |
| I-1-108 | Cl | F | Cl | H | CH₃ | CH₂CH₂OCH₃ | I.1a | 1.354 | 590 | B |
| I-1-109 | Cl | F | Cl | H | Cl | CH₂CH₂OCH₃ | I.1a | 1.364 | 609.9 | B |

| | | | | | | | | | | |
|---|---|---|---|---|---|---|---|---|---|---|
| ^{x)} The given isomer in at least 85% by weight | | | | | | | | | | |

**Table C.2 - Compounds of formula 1.2**

| | | | | | | | | | | |
|---|---|---|---|---|---|---|---|---|---|---|
| 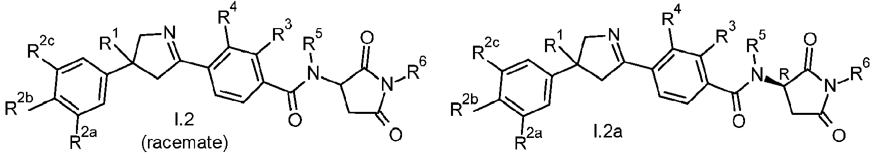 | | | | | | | | | | |
| For all compounds R¹ = CF₃, R⁵ = H | | | | | | | | | | |

| No. | R^{2a} | R^{2b} | R^{2c} | R⁴ | R³ | R⁶ | Isomer^{x)} | HPLC Rt [min] | M+H [m/z] | HPLC Method |
|---|---|---|---|---|---|---|---|---|---|---|
| I-2-1 | Cl | F | Cl | -CH₂CH₂CH₂- | | N(CH₃)₂ | 1.2 | 1.291 | 599 | B |
| I-2-2 | Cl | F | Cl | -CH₂CH₂CH₂- | | CH₂CH₂CH₃ | I.2a | 1.392 | 597.9 | A |
| I-2-3 | Cl | F | Cl | -CH₂CH₂CH₂- | | CH₂CF₃ | 1.2 | 1.383 | 638 | B |
| I-2-4 | Cl | H | Cl | H | CH₃ | CH₂CHF₂ | I.2a | 1.224 | 576 | B |
| I-2-5 | Cl | F | Cl | -CH₂CH₂CH₂- | | CH₂CHF₂ | I.2a | 1.327 | 620 | B |
| I-2-6 | Cl | H | Cl | H | Cl | CH₃ | I.2a | 1.220 | 547.8 | B |
| I-2-7 | Cl | H | Cl | H | Cl | CH₂CHF₂ | I.2a | 1.274 | 597.9 | B |

| | | | | | | | | | | |
|---|---|---|---|---|---|---|---|---|---|---|
| ^{x)} The given isomer in at least 85% by weight | | | | | | | | | | |

**Table C.3 - Compounds of formula 1.3**

| | | | | | | | | | | |
|---|---|---|---|---|---|---|---|---|---|---|
| 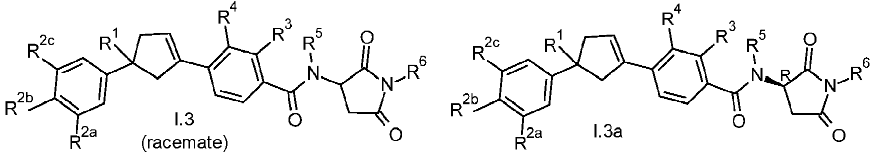 | | | | | | | | | | |
| For all compounds R¹ = CF₃, R⁵ = H | | | | | | | | | | |

| No. | R^{2a} | R^{2b} | R^{2c} | R⁴ | R³ | R⁶ | Isomer^{x)} | HPLC Rt [min] | M+H [m/z] | HPLC Method |
|---|---|---|---|---|---|---|---|---|---|---|
| I-3-1 | H | F | Cl | H | Cl | N(CH₃)₂ | I.3 | 1.270 | 558.1 | B |
| I-3-2 | H | F | Cl | H | Cl | CH₂CF₃ | I.3 | 1.345 | 597.1 | B |
| I-3-3 | H | F | Cl | H | Cl | CH₂CH₂CH₃ | I.3a | 1.354 | 556.9 | A |
| I-3-4 | H | F | Cl | H | Cl | CH₃ | I.3a | 1.261 | 529 | B |
| I-3-5 | H | F | Cl | H | Cl | CH₂CHF₂ | I.3a | 1.311 | 579.1 | B |

| | | | | | | | | | | |
|---|---|---|---|---|---|---|---|---|---|---|
| ^{x)} The given isomer in at least 85% by weight | | | | | | | | | | |

### II. Evaluation of pesticidal activity:

The activity of the compounds of formula I of the invention could be demonstrated and evaluated in biological tests described in the following.

If not otherwise specified the respective active compound is dissolved at the desired concentration in a mixture of 1:1 (vol:vol) distilled water: acetone. The test solution is prepared at the day of use.

### B.1 Diamond back moth (Plutella xylostella)

The active compound was dissolved at the desired concentration in a mixture of 1:1 (vol:vol) distilled water: aceteone. Surfactant (Kinetic HV) was added at a rate of 0.01% (vol/vol). The test solution was prepared at the day of use.

Leaves of cabbage were dipped in test solution and air-dried. Treated leaves were placed in petri dishes lined with moist filter paper and inoculated with ten 3^{rd} instar larvae. Mortality was recorded 72 hours after treatment. Feeding damages were also recorded using a scale of 0-100%.

In this test, compounds I-1-1, I-3-1, I-2-1, I-3-2, I-2-2, I-1-2, I-3-3, I-2-3, I-1-3, I-3-4, I-2-4, I-1-4, I-3-5, I-2-5, I-1-5, I-2-6, I-1-6, I-2-7, I-1-7, I-1-8, I-1-9, I-1-10, I-1-11, I-1-12, I-1-13, I-1-14, I-1-15, I-1-16, I-1-17, I-1-18, I-1-19, I-1-20, I-1-21, I-1-22, I-1-23, I-1-24, I-1-25, I-1-26, I-1-27, I-1-28, I-1-29, I-1-30, I-1-31, I-1-32, I-1-33, I-1-34, I-1-35, I-1-36, I-1-37, I-1-38, I-1-40, I-1-41, I-1-42, I-1-43, I-1-44, I-1-45, I-1-46, I-1-47, I-1-48, I-1-49, I-1-50, I-1-51, I-1-52, I-1-53, I-1-54, I-1-55, I-1-56, I-1-57, I-1-58, I-1-59, I-1-60, I-1-61, I-1-62, I-1-63, I-1-64, 1-1-65, 1-1-66, I-1-67, I-1-68, I-1-69, I-1-70, I-1-71, I-1-72, i-1-73, 1-1-74, 1-1-75, 1-1-76, 1-1-77, 1-1-78, 1-1-79, 1-1-80, I-1-I-1-81, 1-1-82, 1-1-83, 1-1-84, and 1-1-85, resp., at 300 ppm showed over 75% mortality in comparison with untreated controls.

### B.2 Green Peach Aphid (Myzus persicae)

For evaluating control of green peach aphid (*Myzus persicae*) through systemic means the test unit consisted of 96-well-microtiter plates containing liquid artificial diet under an artificial mem brane.

The compounds were formulated using a solution containing 75% v/v water and 25% v/v DMSO. Different concentrations of formulated compounds were pipetted into the aphid diet, using a custom built pipetter, at two replications.

After application, 5 - 8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed.

In this test, compounds I-1-1, I-3-2, I-2-2, I-1-2, I-3-3, I-2-3, I-1-3, I-3-4, I-2-4, I-1-4, I-3-5, I-2-5, 1-1-5, I-2-6, 1-1-6, I-2-7, 1-1-7, 1-1-8, 1-1-9, I-1-10, I-1-11, 1-1-12, 1-1-13, 1-1-14, 1-1-15, 1-1-16, I-1-18, 1-1-19, 1-1-20, 1-1-21, 1-1-22, 1-1-23, 1-1-24, 1-1-25, 1-1-26, 1-1-27, 1-1-28, 1-1-29, 1-1-30, 1-1-31, 1-1-32, 1-1-33, 1-1-36, 1-1-37, 1-1-38, 1-1-41, 1-1-42, 1-1-43, 1-1-44, 1-1-45, 1-1-46, 1-1-47, 1-1-48, 1-1-49, 1-1-50, 1-1-51, 1-1-52, 1-1-53, 1-1-54, 1-1-55, 1-1-56, 1-1-57, 1-1-58, 1-1-59, 1-1-60, 1-1-61, I-1-62, I-1-63, I-1-64, I-1-65, I-1-68, and I-1-69, resp., at 2500 ppm showed over 75% mortality in comparison with untreated controls.

### B.3 Vetch aphid (Megoura viciae)

For evaluating control of vetch aphid (*Megoura viciae*) through contact or systemic means the test unit consisted of 24-well-microtiter plates containing broad bean leaf disks.

The compounds were formulated using a solution containing 75% v/v water and 25% v/v DMSO. Different concentrations of formulated compounds were sprayed onto the leaf disks at 2.5 µl, using a custom built micro atomizer, at two replications.

After application, the leaf disks were air-dried and 5 - 8 adult aphids placed on the leaf disks inside the microtiter plate wells. The aphids were then allowed to suck on the treated leaf disks and incubated at about 23 + 1°C and about 50 + 5 % relative humidity for 5 days. Aphid mortality and fecundity was then visually assessed.

In this test, compounds I-1-1, I-3-2, I-2-2, I-1-2, I-3-3, I-2-3, I-1-3, I-3-4, I-2-4, I-1-4, I-3-5, I-2-5, I-1-5, I-2-6, I-1-6, I-2-7, I-1-7, I-1-8, I-1-9, I-1-10, I-1-11, I-1-12, I-1-13, I-1-14, I -1-15, I-1-16, I-1-18, I-1-19, I-1-20, I-1-21, I-1-22, I-1-23, I-1-24, I-1-25, I-1-26, I-1-27, I -1-28, I-1-29, I-1-30, I-1-31, I-1-32, I-1-36, I-1-37, I-1-38, I-1-41, I-1-42, I-1-43, I-1-44, I -1-45, I-1-46, I-1-47, I-1-48, I-1-49, I-1-50, I-1-51, I-1-52, I-1-53, I-1-54, I-1-55, I-1-56, I -1-57, I-1-58, I-1-59, I-1-60, I-1-61, I-1-62, I-1-63, I-1-64, I-1-65, I-1-68, and I-1-69, resp., at 2500 ppm showed over 75% mortality in comparison with untreated controls.

### B.4 Tobacco budworm (Heliothis virescens)

For evaluating control of tobacco budworm (*Heliothis virescens*) the test unit consisted of 96-well-microtiter plates containing an insect diet and 15-25 *H. virescens* eggs.

The compounds were formulated using a solution containing 75% v/v water and 25% v/v DMSO. Different concentrations of formulated compounds were sprayed onto the insect diet at 10 µl, using a custom built micro atomizer, at two replications.

After application, microtiter plates were incubated at about 28 + 1°C and about 80 + 5 % relative humidity for 5 days. Egg and larval mortality was then visually assessed.

In this test, compounds I-1-1, I-3-2, I-2-2, I-1-2, I-3-3, I-2-3, I-1-3, I-3-4, I-2-4, I-1-4, I-3-5, I-2-5, I-1-5, I-2-6, I-1-6, I-2-7, I-1-7, I-1-8, I-1-9, I-1-10, I-1-11, I-1-12, I-1-13, I-1-14, I-1-15, I-1-16, I-1-17, I-1-18, I-1-19, I-1-20, I-1-21, I-1-22, I-1-23, I-1-24, I-1-25, I-1-26, I-1-27, I-1-28, I-1-29, I-1-30, I-1-31, I-1-32, I-1-33, I-1-36, I-1-37, I-1-38, I-1-41, I-1-42, I-1-43, I-1-44, I-1-45, I-1-46, I-1-47, I-1-48, I-1-49, I-1-50, I-1-51, I-1-52, I-1-53, I-1-54, I-1-55, I-1-56, I-1-57, I-1-58, I-1-59, I-1-60, I-1-61, I-1-62, I-1-63, I-1-64, I-1-65, I-1-68, and I-1-69, resp., at 2500 ppm showed over 75% mortality in comparison with untreated controls.

### B.5 Boll weevil (Anthonomus grandis)

For evaluating control of boll weevil (*Anthonomus grandis*) the test unit consisted of 96-well-microtiter plates containing an insect diet and 5-10 *A. grandis* eggs.

The compounds were formulated using a solution containing 75% v/v water and 25% v/v DMSO. Different concentrations of formulated compounds were sprayed onto the insect diet at 5 µl, using a custom built micro atomizer, at two replications.

After application, microtiter plates were incubated at about 25 + 1°C and about 75 + 5 % relative humidity for 5 days. Egg and larval mortality was then visually assessed.

In this test, compounds I-1-1, I-3-2, I-2-2, I-1-2, I-3-3, I-2-3, I-1-3, I-3-4, I-2-4, I-1-4, I-3-5, I-2-5, I-1-5, I-2-6, I-1-6, I-2-7, I-1-7, I-1-8, I-1-9, I-1-10, I-1-11, I-1-12, I-1-13, I-1-14, I -1-15, I-1-16, I-1-17, I-1-18, I-1-19, I-1-20, I-1-21, I-1-22, I-1-23, I-1-24, I-1-25, I-1-26, I -1-27, I-1-28, I-1-29, I-1-30, I-1-31, I-1-32, I-1-33, I-1-36, I-1-37, I-1-38, I-1-41, I-1-42, I -1-43, I-1-44, I-1-45, I-1-46, I-1-47, I-1-48, I-1-49, I-1-50, I-1-51, I-1-52, I-1-53, I-1-54, I -1-55, I-1-56, I-1-57, I-1-58, I-1-59, I-1-60, I-1-61, I-1-62, I-1-63, I-1-64, I-1-65, I-1-68, and I-1-69, resp., at 2500 ppm showed over 75% mortality in comparison with untreated controls.

### B.7 Orchid thrips (dichromothrips corbetti)

*Dichromothrips corbettiadults* used for bioassay were obtained from a colony maintained continuously under laboratory conditions. For testing purposes, the test compound is diluted in a 1:1 mixture of acetone:water (vol:vol), plus Kinetic HV at a rate of 0.01% v/v.

Thrips potency of each compound was evaluated by using a floral-immersion technique. All petals of individual, intact orchid flowers were dipped into treatment solution and allowed to dry in Petri dishes. Treated petals were placed into individual re-sealable plastic along with about 20 adult thrips. All test arenas were held under continuous light and a temperature of about 28°C for duration of the assay. After 3 days, the numbers of live thrips were counted on each petal. The percent mortality was recorded 72 hours after treatment.

In this test, compounds I-1-1, I-3-1, I-2-1, I-3-2, I-2-2, I-1-2, I-3-3, I-2-3, I-1-3, I-3-4, I-2-4, I-1-4, I-3-5, I-2-5, I-1-5, I-2-6, I-1-6, I-2-7, I-1-7, I-1-8, I-1-9, I-1-10, I-1-11, I-1-12, I-1-13, I-1-14, I-1-15, I-1-16, I-1-17, I-1-18, I-1-19, I-1-20, I-1-21, I-1-22, I-1-23, I-1-24, I-1-25, I-1-26, I-1-27, I-1-28, I-1-29, I-1-30, I-1-31, I-1-32, I-1-33, I-1-34, I-1-35, I-1-36, I-1-37, I-1-38, I-1-40, I-1-41, I-1-42, I-1-43, I-1-44, I-1-45, I*-*1-46, I-1-47, I-1-48, I-1-49, I-1-50, I-1-51, I-1-52, I-1-53, I-1-54, I-1-55, I-1-56, I-1-57, I-1-58, I-1-59, I-1-60, I-1-61, I-1-62, I-1-63, I-1-64, I-1-65, I-1-66, I-1-67, I-1-68, I-1-69, I-1-70, I-1-71 , I-1-72, I-1-73, I-1-74, I-1-75, I-1-76, I-1-77, I-1-78, I-1-79, I-1-80, I-1-I-1-81, I-1-82, I-1-83, I-1-84, and I-1-85, resp., at 300 ppm showed over 75% mortality in comparison with untreated controls.

### B.8 Rice green leafhopper (Nephotettix virescens)

Rice seedlings were cleaned and washed 24 hours before spraying. The active compounds were formulated in 1:1 acetone:water (vol:vol), and 0.01% vol/vol surfactant (Kinetic HV) was added. Potted rice seedlings were sprayed with 5-6 ml test solution, air dried, covered with Mylar cages cages and inoculated with 10 adults. Treated rice plants were kept at about 28-29°C and relative humidity of about 50-60%. Percent mortality was recorded after 72 hours.

In this test, compounds I-3-1, I-2-1, I-3-2, I-2-2, I-1-2, I-3-3, I-2-3, I-1-3, I-3-4, I-2-4, I-1-4, I-3-5, I-2-5, I-1-5, I-2-6, I-1-6, I-2-7, I-1-7, I-1-8, I-1-9, I-1-10, I-1-11, I-1-12, I-1-13, I-1-14, I-1-15, I-1-16, I-1-18, I-1-19, I-1-20, I-1-21, I-1-22, I-1-23, I-1-24, I-1-25, I-1-27, I-1-28, I-1-29, I-1-30, I-1-31, I-1-32, I-1-34, I-1-35, I-1-36, I-1-37, I-1-38, I-1-40, I-1-41, I-1-42, I-1-46, I-1-47, I-1-48, I-1-49, I-1-50, I-1-51, I-1-52, I-1-53, I-1-54, I-1-55, I-1-57, I-1-58, I-1-59, I-1-65, I-1-66, I-1-67, I-1-68, I-1-69, I-1-70, I-1-71 , I-1-77, I-1-78, I-1-79, I-1-80, I-1-I-1-81, I-1-82, I-1-84, and I-1-85, resp., at 300 ppm showed over 75% mortality in comparison with untreated controls.

### B.9 Red spider Mite (Tetranychus kanzawai)

The active compound was dissolved at the desired concentration in a mixture of 1:1 (vol:vol) distilled water: acetone. Add surfactant (Kinetic HV) was added at a rate of 0.01% (vol/vol).The test solution was prepared at the day of use.

Potted cowpea beans of 4-5 days of age were cleaned with tap water and sprayed with 1-2 ml of the test solution using air driven hand atomizer. The treated plants were allowed to air dry and afterwards inoculated with 30 or more mites by clipping a cassava leaf section from rearing population. Treated plants were placed inside a holding room at about 25-27°C and about 50-60% relative humidity. Percent mortality was assessed 72 hours after treatment.

In this test, compounds I-2-1, I-2-2, I-1-2, I-3-3, I-2-3, I-1-3, I-3-4, I-2-4, I-1-4, I-3-5, I-2-5, I-1-5, I-2-6, I-1-6, I-2-7, I-1-7, I-1-8, I-1-9, I-1-10, I-1-11, I-1-12, I-1-13, I-1-14, I-1-15, I-1-16, I-1-18, I-1-19, I-1-20, I-1-21, I-1-22, I-1-23, I-1-24, I-1-25, I-1-27, I-1-28, I-1-29, I-1-30, I-1-31, I-1-32, I-1-34, I-1-35, I-1-36, I-1-37, I-1-38, I-1-40, I-1-41, I-1-44, I-1-45, I-1-46, I-1-47, I-1-48, I-1-49, I-1-50, I-1-51, I-1-52, I-1-53, I-1-54, I-1-55, I-1-57, I-1-58, I-1-59, I-1-60, I-1-61, I-1-65, I-1-66, I-1-67, I-1-68, I-1-69, I-1-70, I-1-71, I-1-77, I-1-78, I-1-79, I-1-80, I-1-1-1-81, I-1-82, I-1-83, and I-1-84, resp., at 300 ppm showed over 75% mortality in comparison with untreated controls.

### B.10 Southern armyworm (Spodoptera eridania)

The active compounds were formulated in cyclohexanone as a 10,000 ppm solution supplied in tubes. The tubes were inserted into an automated electrostatic sprayer equipped with an atomizing nozzle and they served as stock solutions for which lower dilutions were made in 50% acetone:50% water (v/v). A nonionic surfactant (Kinetic®) was included in the solution at a volume of 0.01% (v/v).

Lima bean plants (variety Sieva) were grown 2 plants to a pot and selected for treatment at the 1^{st} true leaf stage. Test solutions were sprayed onto the foliage by an automated electrostatic plant sprayer equipped with an atomizing spray nozzle. The plants were dried in the sprayer fume hood and then removed from the sprayer. Each pot was placed into perforated plastic bags with a zip closure. About 10 to 11 armyworm larvae were placed into the bag and the bags zipped closed. Test plants were maintained in a growth room at about 25°C and about 20-40% relative humidity for 4 days, avoiding direct exposure to fluorescent light (24 hour photoperiod) to prevent trapping of heat inside the bags. Mortality and reduced feeding were assessed 4 days after treatment, compared to untreated control plants.

In this test, compounds I-2-2, I-1-13, I-1-14, I-1-15, I-1-16, I-1-17, I-1-18, I-1-19, I-1-36, I -1-37, I-1-38, I-1-41 at 300 ppm, and I-1-2, I-1-3, I-1-4, I-1-6, I-1-7, I-1-8, I-1-9, I-1-10, I-1-13, I-1-14, I-1-16, I-1-18, and I-1-19, resp., at 10 ppm showed over 75% mortality in comparison with untreated controls.

### B.11 Green Soldier Stink Bug (Nezara viridula)

The active compound was dissolved at the desired concentration in a mixture of 1:1 (vol:vol) distilled water: acetone. Surfactant (Kinetic HV) was added at a rate of 0.01% (vol/vol). The test solution was prepared at the day of use. Soybean pods were placed in glass Petri dishes lined with moist filter paper and inoculated with ten late 3rd instar *N. viridula.* Using a hand atomizer, approximately 2 ml solution is sprayed into each Petri dish. Assay arenas were kept at about 25°C. Percent mortality was recorded after 5 days.

In this test, compounds I-3-1, I-2-1, I-3-2, I-2-2, I-1-2, I-3-3, I-1-3, I-3-4, I-2-4, I-1-4, I-3-5, I-2-5, I-2-6, I-1-6, I-2-7, I-1-7, I-1-8, I-1-9, I-1-10, I-1-11, I-1-12, I-1-13, I-1-14, I-1-15, I-1-16, I-1-18, I-1-19, I-1-20, I-1-21, I-1-22, I-1-23, I-1-24, I-1-25, I-1-27, I-1-28, I-1-29, I-1-30, I-1-31, I-1-32, I-1-34, I-1-35, I-1-36, I-1-37, I-1-38, I-1-40, I-1-41, I-1-44, I-1-45, I-1-46, I-1-47, I-1-48, I-1-49, I-1-50, I-1-51, I-1-52, I-1-53, I-1-54, I-1-55, I-1-58, I-1-59, I-1-60, I-1-61, I-1-62, I-1-63, I-1-64, I-1-65, I-1-66, I-1-67, I-1-68, I-1-69, I-1-70, I-1-71, I-1-77, I-1-78, I-1-79, I-1-80, I-1-I-1-81, I-1-82, and I-1-84, resp., at 300 ppm showed over 75% mortality in comparison with untreated controls.

### B.12 Neotropical Brown Stink Bug (Euschistusheros)

For evaluating control of *Euschistus heros* through contact the test unit consisted of 100 ml disposable plastic beakers with transparent lids.

The compounds were formulated using a solution containing 50% water and 50% acetone. 50ml of different concentrations of formulated compounds or mixtures were prepared in a glass petri dish and used for dipping three 6 cm sections of fresh pole bean. Each treatment was replicated eight times.

After application, the pole bean sections were air-dried and 5 adult E. heros placed in each beaker. The *E. heros* were then allowed to suck on the treated pole bean sections and incubated at 23 + 1°C, 50 + 5 % RH for 5 days. E. heros mortality was then visually assessed.

In this test, compounds I-1-2, I-1-6, I-1-7, I-1-8, I-1-9, I-1-10, I-1-11, I-1-12, I-1-13, I-1-14, I-1-15, I-1-16, I-1-18, I-1-19, I-1-20, I-1-22, I-1-23, I-1-24, I-1-25, I-1-28, I-1-29, I-1-30, I-1-31, I-1-32, and I-1-40, resp., at 500 ppm showed over 75% mortality in comparison with untreated controls.

### B.13 Brown Marmorated Stink Bug (Halyomorpha halys)

The active compound was dissolved at the desired concentration in a mixture of 1:1 (vol:vol) distilled water: acetone. Surfactant (Kinetic HV) was added at a rate of 0.01% (vol/vol). The test solution was prepared at the day of use.

Row peanuts and soybean seeds were placed into microwavable plastic cups and inoculated with five adult stage *H. halys.* Using a hand atomizer, approximately 1 ml solution is sprayed into each cup, insects and food present. A water source was provided (cotton wick with water). Each treatment is replicated 4-fold. Assay arenas are kept at about 25°C. Percent mortality was recorded after 5 days.

In this test, compounds I-1-2, I-1-3, I-1-11, I-1-12, I-1-13, I-1-14, I-1-15, and I-1-16, resp., at 100 ppm showed over 75% mortality in comparison with untreated controls.

### Comparative Examples

The beneficial activity of the compounds according to the invention over structurally close compounds known from prior art was demonstrated by the following comparative experiments:
The tables show % mortality in comparison to untreated controls.

### CE.1 Cowpea aphid (aphis craccivora)

Potted cowpea plants colonized with approximately 100 - 150 aphids of various stages were sprayed after the pest population has been recorded. Population reduction was assessed after 24, 72, and 120 hours.

| Compound | | 10 ppm |
|---|---|---|
| 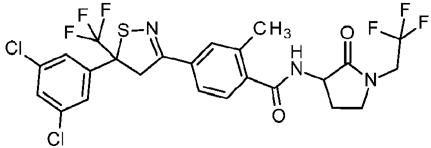 | No. 2-195 of WO 2013/092943 | 0% at 300 ppm |
| 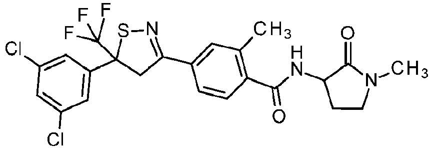 | No. 2-201 of WO 2013/092943 | 0% at 300 ppm |
| 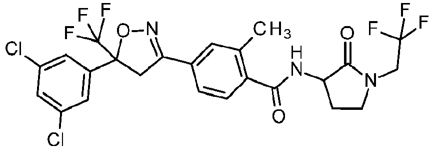 | No. 5-405 of US 20070066617 | 25% |
| 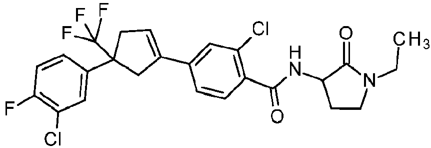 | No. I-9 of WO 2017/016883 | 25% |
| 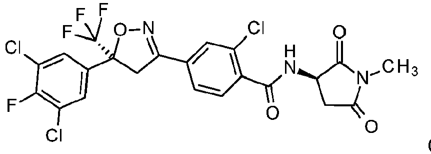 | Compound No. I-1-13 | 90% |
| 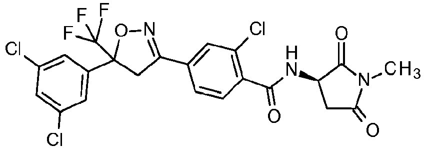 | Compound No. I-1-19 | 56% |
| 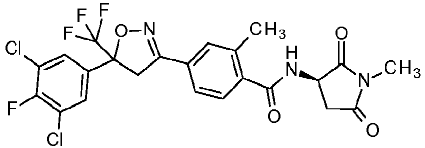 | Compound No. I-1-27 | 93% |
| 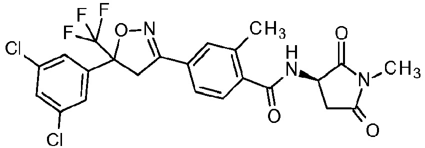 | Compound No. I-1-28 | 50% |
| 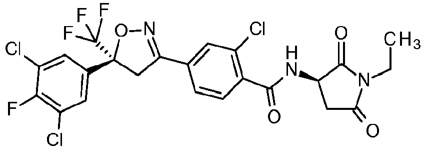 | Compound No. I-1-52 | 86% |
| 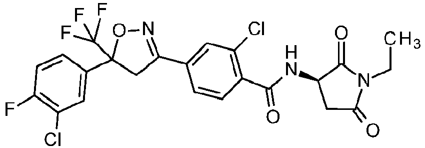 | Compound No. I-1-53 | 100% |

### CE.2 Rice brown plant hopper (Nilaparvata lugens)

Rice seedlings were cleaned and washed 24 hours before spraying. The active compounds were formulated in 50:50 acetone:water (vol:vol) and 0.1% vol/vol surfactant (EL 620) was added. Potted rice seedlings were sprayed with 5 ml test solution, air dried, placed in cages and inoculated with 10 adults. Treated rice plants were kept at about 28-29°C and relative humidity of about 50-60%. Percent mortality was recorded after 72 hours.

| Compound | | 10 ppm |
|---|---|---|
| 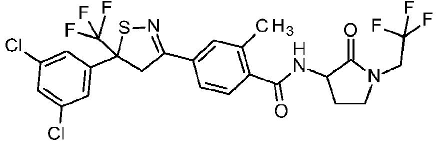 | No. 2-195 of WO 2013/092943 | 25% |
| 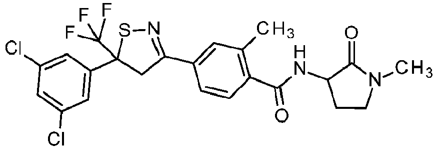 | No. 2-201 of WO 2013/092943 | 0% |
| 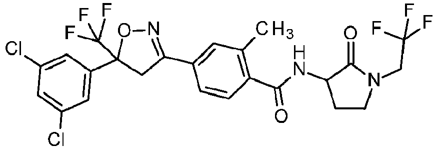 | No. 5-405 of US 20070066617 | 0% |
| 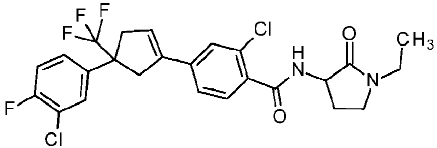 | No. I-9 of WO 2017/016883 | 25% |
| 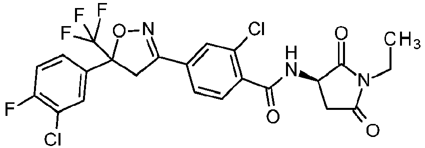 | Compound No. I-1-53 | 75% |

### CE.3 Orchid thrips (dichromothrips corbetti)

*Dichromothrips corbetti* adults used for bioassay were obtained from a colony maintained continuously under laboratory conditions. For testing purposes, the test compound was diluted to a concentration of 300 ppm (wt compound: vol diluent) in a 1:1 mixture of acetone:water (vol:vol), plus 0.01% vol/vol Kinetic® surfactant.

Thrips potency of each compound was evaluated by using a floral-immersion technique. Plastic petri dishes were used as test arenas. All petals of individual, intact orchid flowers were dipped into treatment solution and allowed to dry. Treated flowers were placed into individual petri dishes along with 10- 15 adult thrips. The petri dishes were then covered with lids. All test arenas were held under continuous light and a temperature of about 28°C for duration of the assay. After 4 days, the numbers of live thrips were counted on each flower, and along inner walls of each petri dish. The level of thrips mortality was extrapolated from pre-treatment thrips numbers.

| Compound | | 10 ppm |
|---|---|---|
| 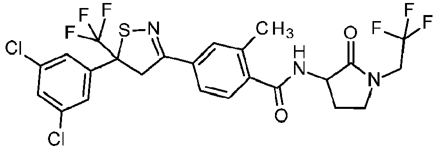 | No. 2-195 of WO 2013/092943 | 0% |
| 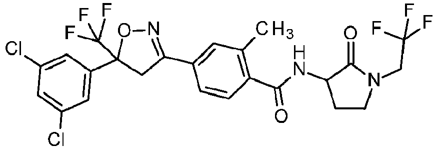 | No. 5-405 of US 20070066617 | 0% |
| 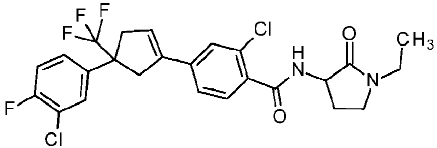 | No. I-9 of WO 2017/016883 | 0% |
| 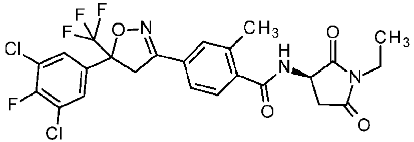 | Compound No. I-1-50 | 100% |
| 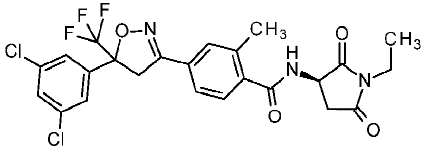 | Compound No. I-1-51 | 100% |
| 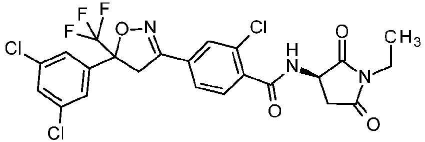 | Compound No. I-1-52 | 100% |
| 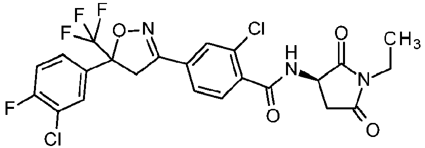 | Compound No. I-1-53 | 100% |
| 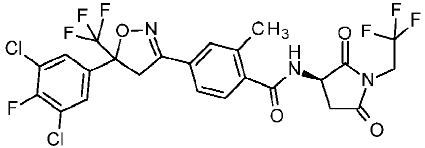 | Compound No. I-1-57 | 100% |
| 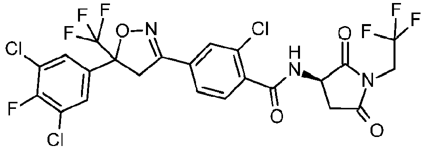 | Compound No. I-1-59 | 100% |

### CE.4 Silverleaf whitefly (bemisia argentifolii)

The active compounds were formulated in 50:50 acetone:water (vol:vol) and 100 ppm Kinetica™ surfactant.

Selected cotton plants were grown to the cotyledon state (one plant per pot). The cotyledons were dipped into the test solution to provide complete coverage of the foliage and placed in a well-vented area to dry. Each pot with treated seedling was placed in a plastic cup and 10 to 12 whitefly adults (approximately 3-5 day old) were introduced. The insects were colleted using an aspirator and a Tygon® tubing connected to a barrier pipette tip. The tip, containing the collected insects, was then gently inserted into the soil containing the treated plant, allowing insects to crawl out of the tip to reach the foliage for feeding. The cups were covered with a reusable screened lid. Test plants were maintained in the holding room at about 25°C and about 20-40% relative humidity for 3 days avoiding direct exposure to the fluorescent light (24 hour photoperiod) to prevent trapping of heat inside the cup. Mortality was assessed 3 days after treatment of the plants.

| Compound | | 10 ppm |
|---|---|---|
| 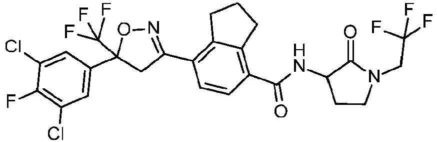 | Tab.128, A-9 of WO 2015128358 | 50% |
| 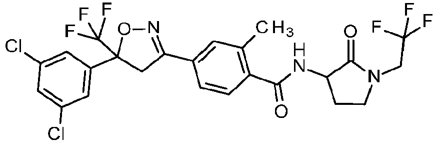 | No. 5-405 of US 20070066617 | 25% |
| 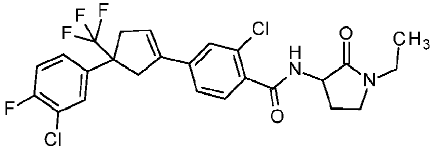 | No. I-9 of WO 2017/016883 | 25% |
| 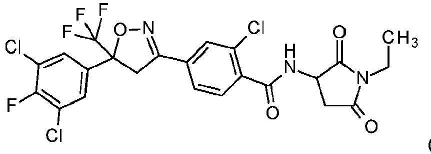 | Compound No. I-1-3 | 75% |
| 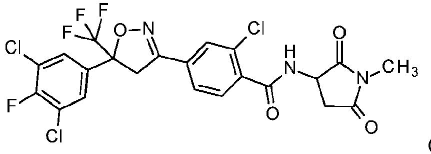 | Compound No. I-1-2 | 88% |

### CE.5 Rice green leafhopper (Nephotettix virescens)

Rice seedlings were cleaned and washed 24 hours before spraying. The active compounds were formulated in 50:50 acetone:water (vol:vol), and 0.1 % vol/vol surfactant (EL 620) was added. Potted rice seedlings were sprayed with 5 ml test solution, air dried, placed in cages and inoculated with 10 adults. Treated rice plants were kept at about 28-29°C and relative humidity of about 50-60%. Percent mortality was recorded after 72 hours.

| Compound | | 3 ppm |
|---|---|---|
| 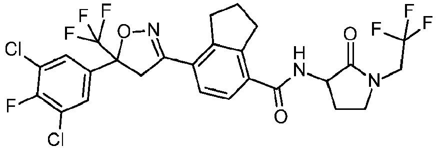 | Tab.128, A-9 of WO 2015128358 | 0% |
| 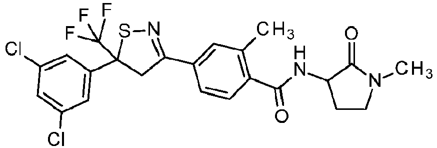 | No. 2-201 of WO 2013/092943 | 0% |
| 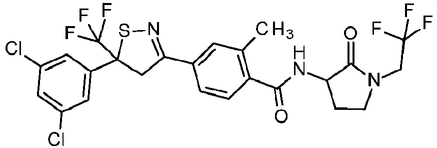 | No. 2-195 of WO 2013/092943 | 0% |
| 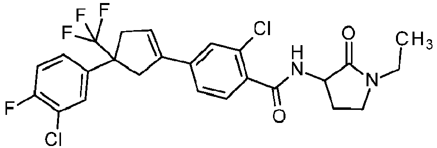 | No. I-9 of WO 2017/016883 | 0% |
| 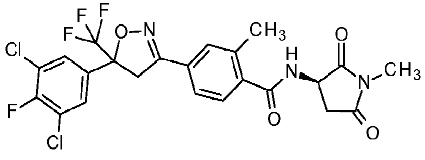 | Compound No. I-1-27 | 83% |
| 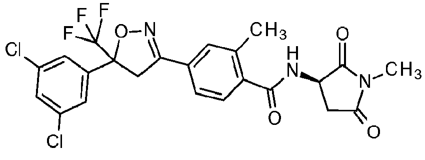 | Compound No. I-1-28 | 79% |
| 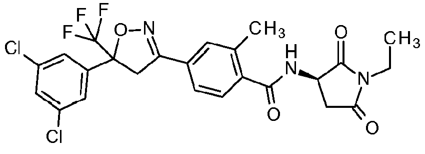 | Compound No. I-1-51 | 90% |

### CE.6 Diamond back moth (plutella xylostella)

### Contact Activity

Ten third- instar larvae are sprayed with 1 ml test solution and introduce to untreated leaves held petri dished lined with moist filter paper. Mortality is recorded 24, 72, and 120 hours after treatment.

| Compound | | 0.1 ppm |
|---|---|---|
| 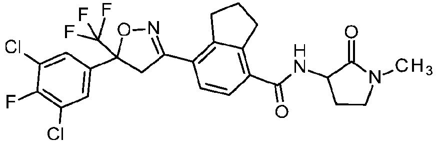 | Tab.126, A-9 of WO 2015128358 | 0% |
| 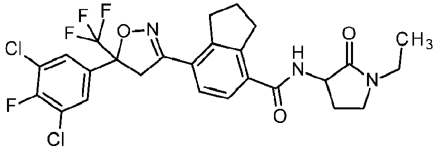 | Tab.127, A-9 of WO 2015128358 | 0% |
| 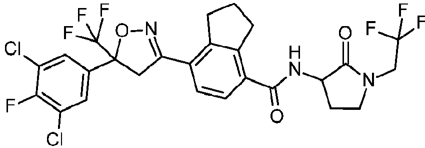 | Tab.128, A-9 of WO 2015128358 | 0% |
| 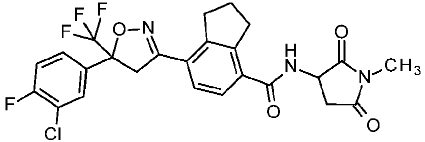 | Compound No. I-1-11 | 75% |
| 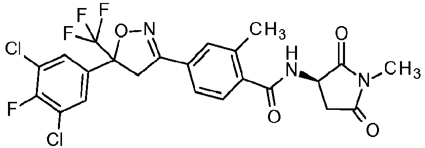 | Compound No. I-1-27 | 100% |
| 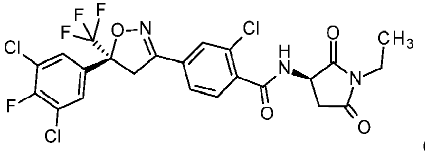 | Compound No. I-1-21 | 97% |

### CE.7 Southern armyworm (Spodoptera eridania), 2nd instar larvae

The active compounds were formulated in 50:50 acetone:water (vol) and 100 ppm Kinetica™ surfactant.

A pair of first true leaves of Sieva lima bean was dipped into the test solution and allowed to dry. The leaves were then placed in a plastic perforated zip enclosure bag and ten 2nd instar larvae were added. At 4 days, observations were made of mortality and reduced feeding.

| Compound | | 3 ppm |
|---|---|---|
| 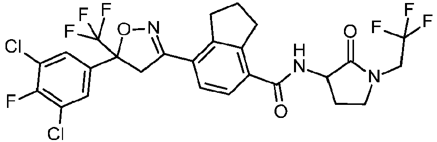 | Tab.128, A-9 of WO 2015128358 | 0% |
| 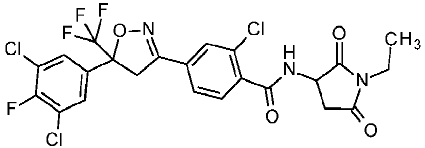 | Compound No. I-1-3 | 100% |
| 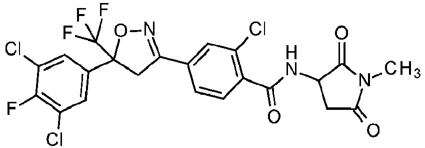 | Compound No. I-1-2 | 100% |
| 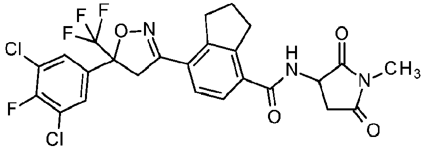 | Compound No. I-1-10 | 100% |

## Claims

1. Succinimide compounds compounds of formula I 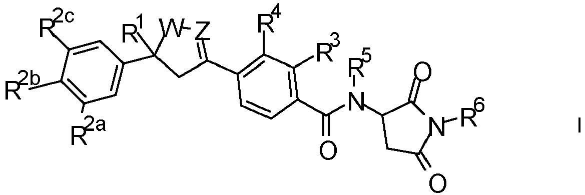 wherein
W-Z is -O-N=, -CH₂-N=, or -CH₂-CH=;
R¹ halomethyl;
R^{2a} halogen, halomethyl, or halomethoxy;
R^{2b}, R^{2c} are independently H, or as defined for R^{2a};
R³ is halogen, CN, NO₂, C₁-C₂-alkyl, halomethyl, C₁-C₂-alkoxy, S(O)ₘ-C₁-C₂-alkyl, C₁-C₂-haloalkoxy, or S(O)ₘ-C₁-C₂-haloalkyl;
R⁴ is H, or as defined for R³; or
R³ and R⁴ form together with the C-atoms they are bound to a 5-, or 6-membered saturated, partially, or fully unsaturated carbocyclic ring;
R⁵,R⁶ are independently H, CN, C₁-C₁₀-alkyl, C₃-C₈-cycloalkyl, C₂-C₁₀-alkenyl, C₃-C₈-cycloalkenyl, C₂-C₁₀-alkynyl, OR¹⁰, S(O)ₘR¹⁰, S(O)ₘN(R¹⁰)₂, N(R¹⁰)₂, which aliphatic groups are unsubstituted, partially or fully halogenated and/or substituted with one or more R^{a}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
R¹⁰ is independently H, C₁-C₆-alkyl, C₁-C₆-haloalkyl, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆-haloalkynyl, which groups are unsubstituted or substituted with one or more R^{a},
R^{a} is CN, N₃, NO₂, SCN, SF₅, Si(C₁-C₄-alkyl)₃, OR^{a1}, OSO₂R^{a1}, S(O)ₙR^{a1}, N(R^{a2})R^{a3}, C(=O)N(R^{a2})R^{a3}, C(=S)N(R^{a2})R^{a3}, C(=O)R^{a1}, C(=O)OR^{a1}, CH=NOR^{a1}, C₃-C₈-cycloalkyl, C₃-C₈-halocycloalkyl, which cyclic moieties may be substituted with R^{a4}; phenyl which is unsubstituted or substituted with one or more R^{A}; and 3- to 7-membered saturated, partially or fully unsaturated heterocycle comprising 1, 2 or 3 heteroatoms O, N(O)ₙ or S(O)ₘ as ring members, which heterocycle is unsubstituted or substituted with one or more R^{A},
m is 0, 1, or 2;
n is 0, or 1;
R^{a1} H, C₁-C₆-alkyl, C₁-C₆-haloalkyl, C₂-C₄-alkenyl, C₂-C₄-alkynyl, CH₂-CN, C₃-C₆-cycloalkyl, C₃-C₆-halocycloalkyl, C₃-C₆-cycloalkylmethyl, C₃-C₆-halocycloalkylmethyl, phenyl and hetaryl which aromatic rings are unsubstituted or partially or fully substituted with R^{A};
R^{a2} is H, or C₁-C₆-alkyl,
R^{a3} is H, C₁-C₆-alkyl, C₁-C₆-haloalkyl, C₂-C₆-alkenyl, C₂-C₆-haloalkenyl, C₂-C₆-alkynyl, C₂-C₆-haloalkynyl, or C₃-C₆-cycloalkyl, C₃-C₆-halocycloalkyl, C₃-C₆-cycloalkylmethyl, or C₃-C₆-halocycloalkylmethyl which rings are unsubstituted or substituted with a cyano;
R^{a4} is independently OH, CN, C₁-C₆-alkoxy, C₁-C₆-haloalkoxy, S(O)ₘ-C₁-C₆-alkyl, S(O)ₘ-C₁-C₆-haloalkyl, C(=O)N(R^{a2})R^{a3}, C₃-C₆-cycloalkyl, or C₃-C₆-halocycloalkyl which cycles are unsubstituted or substituted with one or more R^{a11}; or R^{a12}; or phenyl, partially or fully unsaturated heterocycle which rings are unsubstituted or substituted with one or more R^{A};
R^{a11} is independently OH, cyano, C₁-C₂-alkyl, or C₁-C₂-haloalkyl;
R^{A} is independently selected from halogen, CN, NO₂, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, C₁-C₄-alkoxy, C₁-C₄-haloalkoxy, S(O)ₘ-C₁-C₄-alkyl, S(O)ₘ-C₁-C₄-haloalkyl, C₁-C₄-alkylcarbonyl, C₁-C₄-haloalkylcarbonyl, C(=O)N(R^{a2})R^{a3}; or
two R^{A} present on the same carbon atom of a saturated or partially saturated ring may form together =O or =S; or
two R^{A} present on the same S or SO ring member of a heterocyclic ring may together form a group =N(C₁-C₆-alkyl), =NO(C₁-C₆-alkyl), =NN(H)(C₁-C₆-alkyl) or =NN(C₁-C₆-alkyl)₂;
and the N-oxides, stereoisomers and agriculturally or veterinarily acceptable salts thereof.

2. Compounds of formula I according to claim 1, which correspond to formula I.a

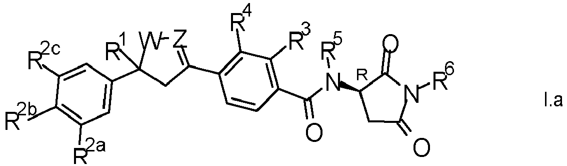

3. Compounds of formula I according to claim 1 or 2, which correspond to formula I.Aa

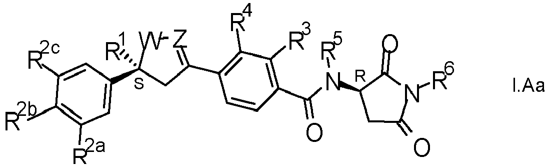

4. Compounds of formula I according to any of claims 1 to 3, which correspond to formula I.1

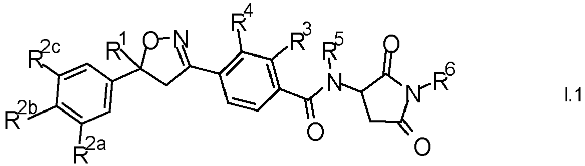

5. Compounds of formula I according to any of claims 1 to 3, which correspond to formula I.2

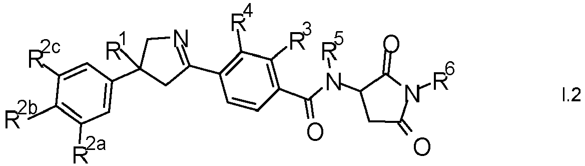

6. Compounds of formula I according to any of claims 1 to 3, which correspond to formula I.3

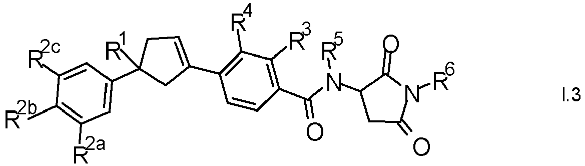

7. Compounds of formula I according to any of claims 1 to 6, wherein R³ is halogen, NO₂, CN, CH₃, fluoromethyl, CF₃, SCH₃, or OCH₃, and R⁴ is H.

8. Compounds of formula I according to any of claims 1 to 7, wherein R⁵ is H, C₁-C₆-alkyl, C₃-C₆-cycloalkyl, C₂-C₆-alkenyl, or C₂-C₆-alkynyl.

9. Compounds of formula I according to any of claims 1 to 8, wherein R⁶ is C₁-C₆-alkyl which is unsubstituted or substituted with phenyl or C(=O)-C₁-C₆-alkoxy; or R⁶ is C₁-C₄-alkyl, C₁-C₃-haloalkyl, C₃-C₄-cycloalkyl, C₁-C₃-alkoxy, C₁-C₃-haloalkoxy, C₁-C₄-alkylamino, and di-C₁-C₄-alkylamino.

10. An agricultural or veterinary composition comprising at least one compound according to any one of claims 1 to 9 and/or at least one agriculturally or veterinarily acceptable salt thereof, and at least one inert liquid and/or solid agriculturally or veterinarily acceptable carrier.

11. An agricultural composition for use in combating animal pests comprising at least one compound as defined in any of claims 1 to 9 and at least one inert liquid and/or solid acceptable carrier and, if desired, at least one surfactant.

12. The composition according to claim 10 or for use according to claim 11, comprising additionally a further active substance.

13. A method for protecting growing plants from attack or infestation by invertebrate pests, which method comprises contacting a plant, or soil or water in which the plant is growing, with a pesticidally effective amount of at least one compound as defined in any of claims 1 to 9.

14. Seed comprising a compound as defined in any of claims 1 to 9, or the enantiomers, diastereomers or salts thereof, in an amount of from 0.1 g to 10 kg per 100 kg of seed.

15. A compound of the formula I as defined in any of claims 1 to 9, a stereoisomer thereof and/or a veterinarily acceptable salt thereof, for use in a method for treating or protecting an animal from infestation or infection by invertebrate pests.

## Patentansprüche

1. Succinimidverbindungen der Formel I 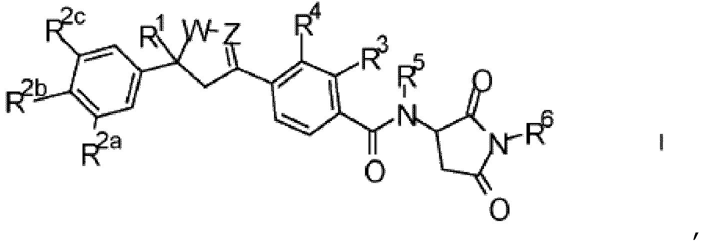 wobei
W-Z für -O-N=, -CH₂-N= oder -CH₂-CH= steht,
R¹ für Halogenmethyl steht,
R^{2a} für Halogen, Halogenmethyl oder Halogenmethoxy steht,
R^{2b}, R^{2c} unabhängig voneinander für H stehen oder wie für R^{2a} definiert sind,
R³ für Halogen, CN, NO₂, C₁-C₂-Alkyl, Halogenmethyl, C₁-C₂-Alkoxy, S(O)ₘ-C₁-C₂-Alkyl, C₁-C₂-Halogenalkoxy oder S(O)ₘ-C₁-C₂-Halogenalkyl steht,
R⁴ für H steht oder wie für R³ definiert ist oder
R³ und R⁴ zusammen mit den C-Atomen, an die sie gebunden sind, einen 5- oder 6-gliedrigen gesättigten, teilweise oder vollständig ungesättigten carbocyclischen Ring bilden,
R⁵, R⁶ unabhängig voneinander für H, CN, C₁-C₁₀-Alkyl, C₃-C₈-Cycloalkyl, C₂-C₁₀-Alkenyl, C₃-C₈-Cycloalkenyl, C₂-C₁₀-Alkinyl, OR¹⁰, S(O)ₘ)R¹⁰, S(O)ₘN(R¹⁰)₂, N(R¹⁰)₂, wobei die aliphatischen Gruppen unsubstituiert, teilweise oder vollständig halogeniert und/oder durch ein oder mehrere R^{a} substituiert sind, Phenyl, das unsubstituiert oder durch ein oder mehrere R^{A} substituiert ist, oder einen 3- bis 7-gliedrigen gesättigten, teilweise oder vollständig ungesättigten Heterocyclus mit 1, 2 oder 3 Heteroatomen O, N(O)ₙ oder S(O)ₘ als Ringgliedern, wobei der Heterocyclus unsubstituiert oder durch ein oder mehrere R^{A} substituiert ist, stehen,
R¹⁰ unabhängig für H, C₁-C₆-Alkyl, C₁-C₆-Halogenalkyl, C₃-C₈-Cycloalkyl, C₃-C₈-Cycloalkyl-C₁-C₄-alkyl, C₃-C₈-Halogencycloalkyl, C₂-C₆-Alkenyl, C₂-C₆-Halogenalkenyl, C₂-C₆-Alkinyl, C₂-C₆-Halogenalkinyl, wobei diese Gruppen unsubstituiert oder durch einen oder mehrere R^{a} substituiert sind, steht,
R^{a} für CN, N₃, NO₂, SCN, SF₅, Si(C₁-C₄-Alkyl)₃, OR^{a1}, OSO₂R^{a1}, S(O)ₙR^{a1}, N(R^{a2})R^{a3}, C(=O)N(R^{a2})R^{a3}, C(=S)N(R^{a2})R^{a3}, C(=O)R^{a1}, C(=O)OR^{a1}, CH=NOR^{a1}, C₃-C₈-Cycloalkyl, C₃-C₈-Halogencycloalkyl, wobei die cyclischen Gruppierungen durch R^{a4} substituiert sein können, Phenyl, das unsubstituiert oder durch ein oder mehrere R^{A} substituiert ist, oder einen 3-bis 7-gliedrigen gesättigten, teilweise oder vollständig ungesättigten Heterocyclus mit 1, 2 oder 3 Heteroatomen O, N(O)ₙ oder S(O)ₘ als Ringgliedern, wobei der Heterocyclus unsubstituiert oder durch ein oder mehrere R^{A} substituiert ist, steht,
m für 0, 1 oder 2 steht,
n für 0 oder 1 steht,
R^{a1} für H, C₁-C₆-Alkyl, C₁-C₆-Halogenalkyl, C₂-C₄-Alkenyl, C₂-C₄-Alkinyl, CH₂-CN, C₃-C₆-Cycloalkyl, C₃-C₆-Halogencycloalkyl, C₃-C₆-Cycloalkylmethyl, C₃-C₆-Halogencycloalkylmethyl, Phenyl oder Hetaryl, wobei die aromatischen Ringe unsubstituiert oder teilweise oder vollständig durch R^{A} substituiert sind, steht,
R^{a2} für H oder C₁-C₆-Alkyl steht,
R^{a3} für H, C₁-C₆-Alkyl, C₁-C₆-Halogenalkyl, C₂-C₆-Alkenyl, C₂-C₆-Halogenalkenyl, C₂-C₆-Alkinyl, C₂-C₆-Halogenalkinyl oder C₃-C₆-Cycloalkyl, C₃-C₆-Halogencycloalkyl, C₃-C₆-Cycloalkylmethyl oder C₃-C₆-Halogencycloalkylmethyl, wobei die Ringe unsubstituiert oder durch ein Cyano substituiert sind, steht,
R^{a4} unabhängig für OH, CN, C₁-C₆-Alkoxy, C₁-C₆-Halogenalkoxy, S(O)ₘ-C₁-C₆-Alkyl, S(O)ₘ-C₁-C₆-Halogenalkyl, C(=O)N(R^{a2})R^{a3}, C₃-C₆-Cycloalkyl oder C₃-C₆-Halogencycloalkyl, wobei die Cyclen unsubstituiert oder durch ein oder durch mehrere R^{a11} substituiert sind, oder Phenyl, teilweise oder vollständig ungesättigtes Heterocyclyl, wobei die Ringe unsubstituiert oder durch ein oder mehrere R^{a11} substituiert sind, steht,
R^{a11} unabhängig für OH, Cyano, C₁-C₂-Alkyl oder C₁-C₂-Halogenalkyl steht,
R^{A} unabhängig aus Halogen, CN, NO₂, C₁-C₄-Alkyl, C₁-C₄-Halogenalkyl, C₂-C₄-Alkenyl, C₂-C₄-Halogenalkenyl, C₂-C₄-Alkinyl, C₂-C₄-Halogenalkinyl, C₃-C₆-Cycloalkyl, C₃-C₆-Halogencycloalkyl, C₁-C₄-Alkoxy, C₁-C₄-Halogenalkoxy, S(O)ₘ-C₁-C₄-Alkyl, S(O)ₘ)-C₁-C₄-Halogenalkyl, C₁-C₄-Alkylcarbonyl, C₁-C₄-Halogenalkylcarbonyl und C(=O)N(R^{a2})R^{a3} ausgewählt ist oder
zwei an dem gleichen Kohlenstoffatom eines gesättigten oder teilweise ungesättigten Rings vorhandene R^{A} zusammen =O oder =S bilden können oder
zwei an dem gleichen S- oder SO-Ringglied eines heterocyclischen Rings vorhandene R^{A} zusammen eine Gruppe =N(C₁-C₆-Alkyl), =NO(C₁-C₆-Alkyl), =NN(H)(C₁-C₆-Alkyl) oder =NN(C₁-C₆-Alkyl)₂ bilden können,
und die N-Oxide, Stereoisomere und landwirtschaftlich oder veterinärmedizinisch unbedenklichen Salze davon.

2. Verbindungen der Formel I nach Anspruch 1, die Formel I.a entsprechen

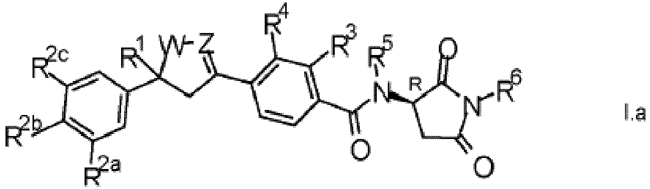

3. Verbindungen der Formel I nach Anspruch 1, die Formel I.Aa entsprechen

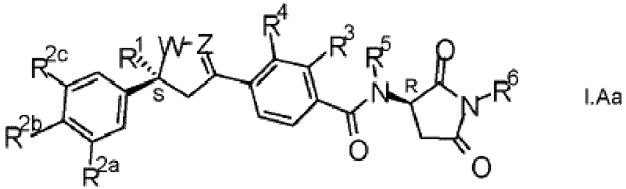

4. Verbindungen der Formel I nach einem der Ansprüche 1 bis 3, die Formel I.1 entsprechen

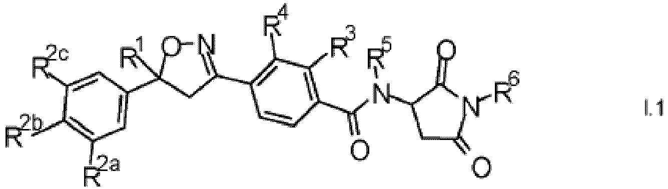

5. Verbindungen der Formel I nach einem der Ansprüche 1 bis 3, die Formel I.2 entsprechen

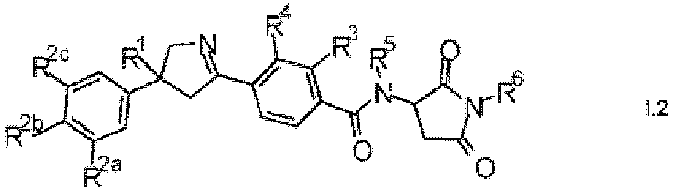

6. Verbindungen der Formel I nach einem der Ansprüche 1 bis 3, die Formel I.3 entsprechen

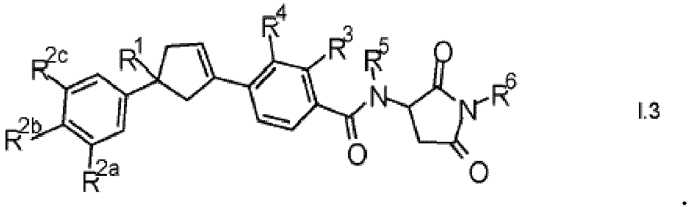

7. Verbindungen der Formel I nach einem der Ansprüche 1 bis 6, wobei R³ für Halogen, NO₂, CN, CH₃, Fluormethyl, CF₃, SCH₃ oder OCH₃ steht und R⁴ für H steht.

8. Verbindungen der Formel I nach einem der Ansprüche 1 bis 7, wobei R⁵ für H, C₁-C₆-Alkyl, C₃-C₆-Cycloalkyl, C₂-C₆-Alkenyl oder C₂-C₆-Alkinyl steht.

9. Verbindungen der Formel I nach einem der Ansprüche 1 bis 8, wobei R⁶ für C₁-C₆-Alkyl steht, das unsubstituiert oder durch Phenyl oder C(=O)-C₁-C₆-Alkoxy substituiert ist, oder R⁶ für C₁-C₄-Alkyl, C₁-C₃-Halogenalkyl, C₃-C₄-Cycloalkyl, C₁-C₃-Alkoxy, C₁-C₃-Halogenalkoxy, C₁-C₄-Alkylamino oder Di-C₁-C₄-Alkylamino steht.

10. Landwirtschaftliche oder veterinärmedizinische Zusammensetzung, umfassend mindestens eine Verbindung nach einem der Ansprüche 1 bis 9 und/oder mindestens ein landwirtschaftlich oder veterinärmedizinisch unbedenkliches Salz davon und mindestens einen inerten flüssigen und/oder festen landwirtschaftlich oder veterinärmedizinisch unbedenklichen Träger.

11. Landwirtschaftliche Zusammensetzung zur Verwendung bei der Bekämpfung von tierischen Schädlingen, umfassend mindestens eine wie in einem der Ansprüche 1 bis 9 definierte Verbindung und mindestens einen flüssigen und/oder festen unbedenklichen Träger und gegebenenfalls mindestens ein Tensid.

12. Zusammensetzung nach Anspruch 10 oder zur Verwendung nach Anspruch 11, zusätzlich umfassend einen weiteren Wirkstoff.

13. Verfahren zum Schützen von wachsenden Pflanzen gegen Angriff oder Befall durch wirbellose Schädlinge, wobei man bei dem Verfahren eine Pflanze oder Boden oder Wasser, in dem die Pflanze wächst, mit einer pestizid wirksamen Menge mindestens einer wie in einem der Ansprüche 1 bis 9 definierten Verbindung in Kontakt bringen.

14. Saatgut, umfassend eine wie in einem der Ansprüche 1 bis 9 definierte Verbindung oder die Enantiomere, Diastereomere oder Salze davon in einer Menge von 0,1 g bis 10 kg pro 100 kg Saatgut.

15. Verbindung der Formel I, definiert wie in einem der Ansprüche 1 bis 9, ein Stereoisomer davon und/oder ein veterinärmedizinisch unbedenkliches Salz davon, zur Verwendung in einem Verfahren zur Behandlung oder zum Schützen eines Tiers vor Befall oder Infektion durch wirbellose Schädlinge.

## Revendications

1. Composés de type succinimide de formule I 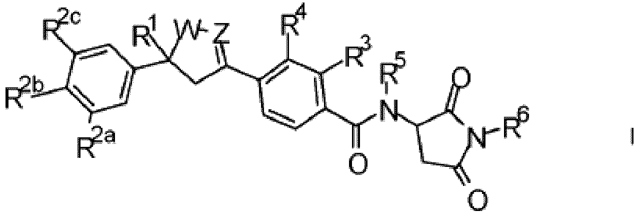
W-Z étant -O-N=, -CH₂-N=, ou -CH₂-CH= ;
R¹ halogénométhyle ;
R^{2a} halogène, halogénométhyle, ou halogénométhoxy ;
R^{2b}, R^{2c} étant indépendamment H, ou tels que définis pour R^{2a} ;
R³ étant halogène, CN, NO₂, C₁-C₂-alkyle, halogénométhyle, C₁-C₂-alcoxy, S (O) ₘ-C₁-C₂-alkyle, C₁-C₂-halogénoalcoxy, ou S (O) ₘ-C₁-C₂-halogénoalkyle ;
R⁴ étant H, ou tel que défini pour R³ ; ou
R³ et R⁴ formant ensemble avec les atomes de C auxquels ils sont liés un cycle carbocyclique à 5 ou 6 chaînons, saturé, partiellement insaturé, ou totalement insaturé ;
R⁵, R⁶ étant indépendamment H, CN, C₁-C₁₀-alkyle, C₃-C₈-cycloalkyle, C₂-C₁₀-alcényle, C₃-C₈-cycloalcényle, C₂-C₁₀-alcynyle, OR¹⁰, S(O)ₘR¹⁰, S(O)ₘN(R¹⁰)₂, N(R¹⁰) ₂, lesquels groupes aliphatiques étant non substitués, partiellement ou totalement halogénés et/ou substitués par un ou plusieurs R^{a} ; phényle qui est non substitué ou substitué par un ou plusieurs R^{A}; et un hétérocycle à 3 à 7 chaînons, saturé, partiellement insaturé ou totalement insaturé comprenant 1, 2 ou 3 hétéroatomes O, N(O)ₙ ou S(O)ₘ en tant qu'éléments de cycle, lequel hétérocycle étant non substitué ou substitué par un ou plusieurs R^{A},
R¹⁰ étant indépendamment H, C₁-C₆-alkyle, C₁-C₆-halogénoalkyle, C₃-C₈-cycloalkyle, C₃-C₈-cycloalkyl-C₁-C₄-alkyle, C₃-C₈-halogénocycloalkyle, C₂-C₆-alcényle, C₂-C₆-halogénoalcényle, C₂-C₆-alcynyle, C₂-C₆-halogénoalcynyle, lesquels groupes étant non substitués ou substitués par un ou plusieurs R^{a},
R^{a} étant CN, N₃, NO₂, SCN, SF₅, Si (C₁-C₄-alkyle) ₃, OR^{a1}, OSO₂R^{a1}, S(O)ₙR^{a1}, N(R^{a2})R^{a3}, C(=O)N(R^{a2})R^{a3}, C(=S)N(R^{a2})R^{a3}, C(=O)R^{a1}, C(=O) OR^{a1}, CH=NOR^{a1}, C₃-C₈-cycloalkyle, C₃-C₈-halogénocycloalkyle, lesquels fragments cycliques pouvant être substitués par R^{a4} ; phényle qui est non substitué ou substitué par un ou plusieurs R^{A} ; et un hétérocycle à 3 à 7 chaînons, saturé, partiellement insaturé ou totalement insaturé comprenant 1, 2 ou 3 hétéroatomes O, N(O)ₙ ou S(O)ₘ en tant qu'éléments de cycle, lequel hétérocycle étant non substitué ou substitué par un ou plusieurs R^{A},
m étant 0, 1, ou 2 ;
n étant 0, ou 1 ;
R^{a1} H, C₁-C₆-alkyle, C₁-C₆-halogénoalkyle, C₂-C₄-alcényle, C₂-C₄-alcynyle, CH₂-CN, C₃-C₆-cycloalkyle, C₃-C₆-halogénocycloalkyle, C₃-C₆-cycloalkylméthyle, C₃-C₆-halogénocycloalkylméthyle, phényle et hétaryle, lesquels cycles aromatiques étant non substitués ou partiellement substitués ou totalement substitués par R^{A} ;
R^{a2} étant H, ou C₁-C₆-alkyle,
R^{a3} étant H, C₁-C₆-alkyle, C₁-C₆-halogénoalkyle, C₂-C₆-alcényle, C₂-C₆-halogénoalcényle, C₂-C₆-alcynyle, C₂-C₆-halogénoalcynyle, ou C₃-C₆-cycloalkyle, C₃-C₆-halogénocycloalkyle, C₃-C₆-cycloalkylméthyle, ou C₃-C₆-halogénocycloalkylméthyle lesquels cycles étant non substitués ou substitués par un cyano ;
R^{a4} étant indépendamment OH, CN, C₁-C₆-alcoxy, C₁-C₆-halogénoalcoxy, S(O)ₘ-C₁-C₆-alkyle, S(O)ₘ-C₁-C₆-halogénoalkyle, C(=O) N(R^{a2})R^{a3}, C₃-C₆-cycloalkyle, ou C₃-C₆-halogénocycloalkyle, lesquels cycles étant non substitués ou substitués par un ou plusieurs R^{a11} ou phényle, hétérocycle partiellement ou totalement insaturé, lesquels cycles étant non substitués ou substitués par un ou plusieurs R^{A} ;
R^{a11} étant indépendamment OH, cyano, C₁-C₂-alkyle, ou C₁-C₂-halogénoalkyle ;
R^{A} étant indépendamment choisi parmi halogène, CN, NO₂, C₁-C₄-alkyle, C₁-C₄-halogénoalkyle, C₂-C₄-alcényle, C₂-C₄-halogénoalcényle, C₂-C₄-alcynyle, C₂-C₄-halogénoalcynyle, C₃-C₆-cycloalkyle, C₃-C₆-halogénocycloalkyle, C₁-C₄-alcoxy, C₁-C₄-halogénoalcoxy, S(O)ₘ-C₁-C₄-alkyle, S (O)ₘ-C₁-C₄-halogénoalkyle, C₁-C₄-alkylcarbonyle, C₁-C₄-halogénoalkylcarbonyle, C (=O) N(R^{a2})R^{a3} ; ou
deux R^{A} présents sur le même atome de carbone d'un cycle saturé ou partiellement saturé pouvant former ensemble =O ou =S ; ou
deux R^{A} présents sur le même élément de cycle S ou SO d'un cycle hétérocyclique pouvant former ensemble un groupe =N (C₁-C₆-alkyle) , =NO(C₁-C₆-alkyle), =NN(H) (C₁-C₆-alkyle) ou =NN(C₁-C₆-alkyle)₂ ;
et N-oxydes, stéréoisomères, et sels acceptables sur le plan agricole ou sur le plan vétérinaire correspondants.

2. Composés de formule I selon la revendication 1, qui correspondent à la formule I.a

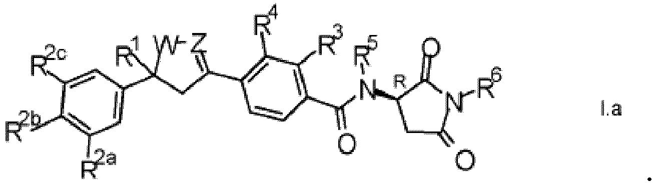

3. Composés de formule I selon la revendication 1 ou 2, qui correspondent à la formule I.Aa

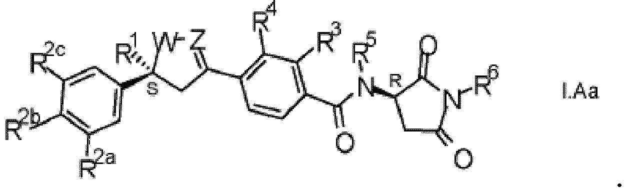

4. Composés de formule I selon l'une quelconque des revendications 1 à 3, qui correspondent à la formule I.1

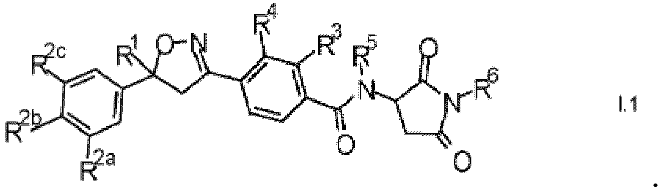

5. Composés de formule I selon l'une quelconque des revendications 1 à 3, qui correspondent à la formule I.2

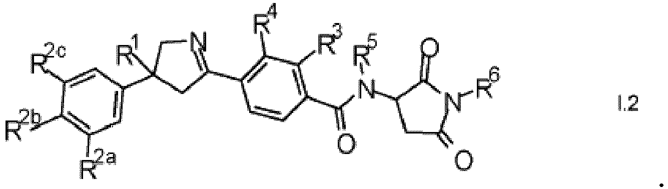

6. Composés de formule I selon l'une quelconque des revendications 1 à 3, qui correspondent à la formule I.3

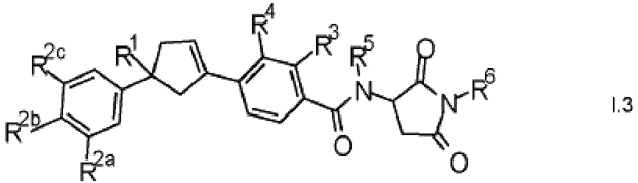

7. Composés de formule I selon l'une quelconque des revendications 1 à 6, R³ étant halogène, NO₂, CN, CH₃, fluorométhyle, CF₃, SCH₃, ou OCH₃, et R⁴ étant H.

8. Composés de formule I selon l'une quelconque des revendications 1 à 7, R⁵ étant H, C₁-C₆-alkyle, C₃-C₆-cycloalkyle, C₂-C₆-alcényle, ou C₂-C₆-alcynyle.

9. Composés de formule I selon l'une quelconque des revendications 1 à 8, R⁶ étant C₁-C₆-alkyle qui est non substitué ou substitué par phényle ou C(=O)-C₁-C₆-alcoxy ; ou R⁶ étant C₁-C₄-alkyle, C₁-C₃-halogénoalkyle, C₃-C₄-cycloalkyle, C₁-C₃-alcoxy, C₁-C₃-halogénoalcoxy, C₁-C₄-alkylamino, et di-C₁-C₄-alkylamino.

10. Composition agricole ou vétérinaire comprenant au moins un composé selon l'une quelconque des revendications 1 à 9 et/ou au moins un sel acceptable sur le plan agricole ou sur le plan vétérinaire correspondant, et au moins un support inerte liquide et/ou solide acceptable sur le plan agricole ou sur le plan vétérinaire.

11. Composition agricole pour une utilisation dans la lutte contre des animaux nuisibles comprenant au moins un composé tel que défini dans l'une quelconque des revendications 1 à 9 et au moins un support inerte acceptable, liquide et/ou solide et, si cela est souhaité, au moins un tensioactif.

12. Composition selon la revendication 10 ou pour une utilisation selon la revendication 11, comprenant de plus une substance active supplémentaire.

13. Procédé pour la protection de végétaux croissants contre l'attaque ou l'infestation par des nuisibles invertébrés, lequel procédé comprenant la mise en contact d'un végétal, ou du sol ou de l'eau dans lequel/laquelle le végétal croît, avec une quantité efficace sur le plan pesticide d'au moins un composé tel que défini dans l'une quelconque des revendications 1 à 9.

14. Semence comprenant un composé tel que défini dans l'une quelconque des revendications 1 à 9, ou les énantiomères, les diastéréoisomères ou les sels correspondants, en une quantité allant de 0,1 g à 10 kg par 100 kg de semences.

15. Composé de la formule I tel que défini dans l'une quelconque des revendications 1 à 9, stéréoisomère correspondant et/ou sel acceptable sur le plan vétérinaire correspondant, pour une utilisation dans un procédé pour le traitement ou la protection d'un animal contre l'infestation ou l'infection par des nuisibles invertébrés.
